# EUROPEAN PATENT SPECIFICATION

(11) **EP 2 983 710 B1**
(45) Date of publication and mention of the grant of the patent: **31.07.2019**
(21) Application number: 14782979.0
(22) Date of filing: 09.04.2014
(51) Int. Cl.: A61K 39/395, C07K 16/18, C07K 16/40, A61K 39/00

(54) **METHODS OF TREATMENT FOR NEUROMYELITIS OPTICA**
BEHANDLUNGSVERFAHREN FÜR NEUROMYELITIS OPTICA
MÉTHODES DE TRAITEMENT D'UNE NEUROMYÉLITE OPTIQUE

(30) Priority: 09.04.2013 US 201361810222 P; 15.05.2013 US 201361823865 P
(43) Date of publication of application: 17.02.2016
(73) Proprietor: Annexon, Inc., South San Francisco, CA 94083-2931 (US); The Regents of the University of California, Oakland, CA 94607 (US)
(72) Inventor: ROSENTHAL, Arnon, Woodside, CA 94062 (US); LEVITEN, Michael, Emerald Hills, CA 94062 (US); VERKMAN, Alan, S., San Francisco, CA 94143-0521 (US)
(74) Representative: Vossius & Partner Patentanwälte Rechtsanwälte mbB
(86) International application number: PCT/US2014/033560
(87) International publication number: WO 2014/169076

(56) References cited:
- US-A1- 2010 092 478
- US-A1- 2011 104 156
- US-A1- 2013 344 073
- DANIEL RICKLIN ET AL: "Complement: a key system for immune surveillance and homeostasis", NATURE IMMUNOLOGY, vol. 11, no. 9, 19 August 2010 (2010-08-19), pages 785-797, XP055306904, GB ISSN: 1529-2908, DOI: 10.1038/ni.1923
- TRADTRANTIP ET AL.: 'Enzymatic deglycosylation converts pathogenic neuromyelitis optica anti- aquaporin-4 immunoglobulin G into therapeutic antibody.' ANN NEUROL. vol. 73, no. 1, January 2013, pages 77 - 85, XP055285253
- PHUAN ET AL.: 'C1q-targeted monoclonal antibody prevents complement-dependent cytotoxicity and neuropathology in in vitro and mouse models of neuromyelitis optica.' ACTA NEUROPATHOL. vol. 125, no. 6, June 2013, pages 829 - 40, XP002734552

## Description

### BACKGROUND

### 1. Field

The present disclosure relates generally to antibodies as charactized in the appended claims for use in preventing, reducing risk of developing, or treating neuromyelitis optica (NMO) and, more specifically, to antibodies as charactized in the appended claims for use in the inhibition of the classical pathway of complement activation.

### 2. Description of Related Art

Neuromyelitis optica (NMO), also known as Devic's disease or Devic's syndrome, is an autoimmune 'aquaporinopathy' of the central nervous system that causes inflammatory demyelinating lesions primarily in spinal cord and optic nerve, leading to paralysis, blindness, and even death. Neuromyelitis optica (NMO) patients frequently raise autoantibodies (NMO-IgG) that are directed at astrocyte water channel aquaporin-4 (AQP4) and are known to activate the complement system, resulting in widespread tissue damage. There is a need for improved therapies for NMO because considerable morbidity and mortality persists even in the face of currently available treatment options, which commonly involve aggressive immunosuppression and plasma exchange.

A broad range of non-immunosuppressive therapies are under evaluation today, ranging from IL-6 antibody therapies and neutrophil elastase inhibition to treatments addressing complement activation, *e.g*., involving the complement factor C5 neutralizing antibody Eculizumab. Eculizumab is a first-in-class terminal complement inhibitor currently approved for the treatment of paroxysmal nocturnal hemoglobinuria (PNH) and atypical hemolytic uremic syndrome (aHUS) (*see, e.g.* FDA drug label for Eculizumab at http://www.accessdata.fda.gov/drugsatfda_docs/label/2007/125166lbl.pdf). Eculizumab was shown to provide clinical benefits in a small, open-label NMO trial (NCT00904826), suggesting complement inhibition as a possible treatment strategy for NMO. However, inhibition of the terminal complement pathway can cause serious side-effects due to the associated weakening of a patient's immune defenses against bacterial infections. Eculizumab therapy, for example, is associated with elevated risks of meningococcal meningitis and other bacterial infections (*see, e.g.* FDA drug label for Eculizumab - Box Warning at http://www.accessdata.fda.gov/drugsatfda_docs/label/2007/125166lbl.pdf). Moreover, Eculizumab is not expected to prevent complement-dependent cell-mediated cytotoxicity (CDCC) to the extent that CDCC is driven by complement factor C3a, an anaphalotoxin produced upstream of the Eculizumab target C5-convertase (*see, e.g.,* Klos A., Tenner A.J., Johswich K.O., Ager R.R., Reis E.S. & Köhl J., The role of the anaphylatoxins in health and disease. Mol Immunol. 2009, 46(14), 2753-66.). CDCC is commonly believed to play an important role in NMO pathogenesis because inhibition of neutrophil and/or eosinophil activity was found to alleviate NMO pathology in a rodent model (*see, e.g.,* Saadoun S., Waters P., MacDonald C., Bell B.A., Vincent A., Verkman A.S. & Papadopoulos M.C., Neutrophil protease inhibition reduces neuromyelitis optica-immunoglobulin G-induced damage in mouse brain. Ann Neurol. 2012, 71(3), 323-333.). Thus, despite Eculizumab's clinical promise, a need remains for alternative treatment options that avoid the complications associated with inhibiting the terminal complement pathway.

US 2010/092478 points to a general association between overall complement activity and NMO. Ricklin et al. 2010 (Nature Immunology, 11(9):785-797) provides a review about the function, structure and dynamics of the complement network and its multiple roles in homeostasis and disease. Tradtrantip et al. 2013 (Ann Neurol., 73(1):77-85) describes that deglycosylation with bacteria-derived endoglycosidase S (EndoS) converts pathogenic NMO-IgG autoantibodies into therapeutic blocking antibodies. US 2011/104156 describes approaches for the treatment of the autoimmune neuromuscular disease Myasthenia gravis (MG).

There is a need to develop new treatment options for NMO patients. Specifically, there is a need for new tools and methods of inhibiting early-stage complement activation in order to validate selective inhibition of early-stage complement pathways as a viable therapeutic strategy for the treatment of NMO.

### BRIEF SUMMARY

The present disclosure is generally directed to antibodies as charactized in the appended claims for use in preventing, reducing risk of developing, or treating NMO that includes inhibiting the classical pathway of complement activation by neutralizing the complement factors C1q, C1r, or C1s, *e.g.,* through the administration of said antibodies, such as monoclonal, chimeric, humanized antibodies, antibody fragments, *etc*., which bind to one or more of these complement factors.

In certain aspects, the present disclosure provides an antibody for use in preventing, reducing risk of developing, or treating an individual having neuromyelitis optica (NMO), where the antibody is: i) an anti-C1q antibody, where the anti-C1q antibody inhibits the interaction between C1q and an autoantibody or between C1q and C1r, or between C1q and C1s; ii) an anti-C1r antibody, where the anti-C1r antibody inhibits the interaction between C1r and C1q or between C1r and C1s, or where the anti-C1r antibody inhibits the catalytic activity of C1r or inhibits the processing of pro-C1r to an active protease; or iii) an anti-C1s antibody, where the anti-C1s antibody inhibits the interaction between C1s and C1q or between C1s and C1r or between C1s and C2 or C4, or where the anti-C1s antibody inhibits the catalytic activity of C1s or inhibits the processing of pro-C1s to an active protease.

In certain embodiments that may be combined with any of the preceding embodiments, the antibody is a monoclonal antibody. In certain embodiments that may be combined with any of the preceding embodiments, the antibody binds mammalian C1q, C1r, or C1s. In certain embodiments that may be combined with any of the preceding embodiments, the antibody binds mammalian C1 complex. In certain embodiments that may be combined with any of the preceding embodiments, the antibody binds human C1q, C1r, or C1s. In certain embodiments that may be combined with any of the preceding embodiments, the antibody binds human C1 complex. In certain embodiments that may be combined with any of the preceding embodiments, the antibody binds to an epitope comprising amino acid residues on human C1q, C1r, or C1s. In certain embodiments that may be combined with any of the preceding embodiments, the antibody is a mouse antibody, a human antibody, a humanized antibody, or a chimeric antibody. In certain embodiments that may be combined with any of the preceding embodiments, the antibody is an antibody fragment selected from Fab, Fab'-SH, Fv, scFv, and (Fab')₂ fragments. In certain embodiments that may be combined with any of the preceding embodiments, the antibody is a bispecific antibody recognizing a first antigen and a second antigen. In certain embodiments that may be combined with any of the preceding embodiments, the first antigen is selected from C1q, C1r, and C1s and the second antigen is an antigen facilitating transport across the blood-brain-barrier. In certain embodiments that may be combined with any of the preceding embodiments, the second antigen is selected from transferrin receptor (TR), insulin receptor (HIR), insulin-like growth factor receptor (IGFR), low-density lipoprotein receptor related proteins 1 and 2 (LPR-1 and 2), diphtheria toxin receptor, CRM197, a llama single domain antibody, TMEM 30(A), a protein transduction domain, TAT, Syn-B, penetratin, a poly-arginine peptide, an angiopep peptide, and ANG1005. In certain embodiments that may be combined with any of the preceding embodiments, the antibody inhibits the classical complement activation pathway. In certain embodiments that may be combined with any of the preceding embodiments, the antibody inhibits the classical complement activation pathway by an amount that ranges from at least 30% to at least 99.9%. In certain embodiments that may be combined with any of the preceding embodiments, the antibody inhibits the alternative complement activation pathway. In certain embodiments that may be combined with any of the preceding embodiments, the antibody inhibits the alternative complement activation pathway by an amount that ranges from at least 30% to at least 99.9%. In certain embodiments that may be combined with any of the preceding embodiments, the antibody inhibits complement-dependent cell-mediated cytotoxicity (CDCC) activation pathway. In certain embodiments that may be combined with any of the preceding embodiments, the antibody inhibits complement-dependent cell-mediated cytotoxicity (CDCC) activation pathway by an amount that ranges from at least 30% to at least 99.9%. In certain embodiments that may be combined with any of the preceding embodiments, the antibody does not inhibit the lectin complement activation pathway. In certain embodiments that may be combined with any of the preceding embodiments, the antibody is an anti-C1q antibody that comprises a dissociation constant (K_{D}) that ranges from 100nM to 0.005nM or less than 0.005nM. In certain embodiments that may be combined with any of the preceding embodiments, the antibody is an anti-C1s antibody that comprises a dissociation constant (K_{D}) that ranges from 100nM to 0.005nM or less than 0.005nM. In certain embodiments that may be combined with any of the preceding embodiments, the antibody is an anti-C1r antibody that comprises a dissociation constant (K_{D}) that ranges from 100nM to 0.005nM or less than 0.005nM. In certain embodiments that may be combined with any of the preceding embodiments, the antibody inhibits autoantibody-dependent and complement-dependent cytotoxicity (CDC). In certain embodiments that may be combined with any of the preceding embodiments, the antibody inhibits complement-dependent cell-mediated cytotoxicity (CDCC). In certain embodiments that may be combined with any of the preceding embodiments, the antibody prevents amplification of the alternative complement activation pathway initiated by C1q binding. In certain embodiments that may be combined with any of the preceding embodiments, the antibody comprises an *in vitro* EC₅₀ that ranges from 3 µg/ml to 0.05 µg/ml or less than 0.05 µg/ml. In certain embodiments that may be combined with any of the preceding embodiments, the antibody does not inhibit autoantibody-dependent cellular cytotoxicity (ADCC). In certain embodiments that may be combined with any of the preceding embodiments, a therapeutically effective amount of two antibodies is to be administered, and the two antibodies are selected from the anti-C1q antibody, the anti-C1r antibody, and the anti-C1s antibody. In certain embodiments that may be combined with any of the preceding embodiments, the use further includes that a therapeutically effective amount of an inhibitor of antibody-dependent cellular cytotoxicity (ADCC) is to be administered. In certain embodiments that may be combined with any of the preceding embodiments, the use further includes that a therapeutically effective amount of an inhibitor of the alternative complement activation pathway is to be administered. In certain embodiments that may be combined with any of the preceding embodiments, the use further includes that a therapeutically effective amount of an inhibitor of the interaction between the autoantibody and its corresponding autoantigen is to be administered. In certain embodiments that may be combined with any of the preceding embodiments, the autoantibody is an NMO-IgG. In certain embodiments that may be combined with any of the preceding embodiments, the autoantibody binds Aquaporin 4 (AQP4). In certain embodiments that may be combined with any of the preceding embodiments, the antibody is an anti-C1q antibody that binds C1q with a binding stoichiometry that ranges from 20:1 to 1.0:1 or less than 1.0:1. In certain embodiments that may be combined with any of the preceding embodiments, the antibody is an anti-C1q antibody that binds C1q with a binding stoichiometry that ranges from 6:1 to 1.0:1 or less than 1.0:1. In certain embodiments that may be combined with any of the preceding embodiments, the antibody is an anti-C1q antibody that binds C1q with a binding stoichiometry that ranges from 2.5:1 to 1.0:1 or less than 1.0:1. In certain embodiments that may be combined with any of the preceding embodiments, the antibody is an anti-C1r antibody that binds C1r with a binding stoichiometry that ranges from 20:1 to 1.0:1 or less than 1.0:1. In certain embodiments that may be combined with any of the preceding embodiments, the antibody is an anti-C1r antibody that binds C1r with a binding stoichiometry that ranges from 6:1 to 1.0:1 or less than 1.0:1. In certain embodiments that may be combined with any of the preceding embodiments, the antibody is an anti-C1r antibody that binds C1r with a binding stoichiometry that ranges from 2.5:1 to 1.0:1 or less than 1.0:1. In certain embodiments that may be combined with any of the preceding embodiments, the antibody is an anti-C1s antibody that binds C1s with a binding stoichiometry that ranges from 20:1 to 1.0:1 or less than 1.0:1. In certain embodiments that may be combined with any of the preceding embodiments, the antibody is an anti-C1s antibody that binds C1s with a binding stoichiometry that ranges from 6:1 to 1.0:1 or less than 1.0:1. In certain embodiments that may be combined with any of the preceding embodiments, the antibody is an anti-C1s antibody that binds C1s with a binding stoichiometry that ranges from 2.5:1 to 1.0:1 or less than 1.0:1.

It is to be understood that one, some, or all of the properties of the various embodiments described herein may be combined to form other embodiments of the present invention. These and other aspects of the invention will become apparent to one of skill in the art. These and other embodiments of the invention are further described by the detailed description that follows.

### DESCRIPTION OF THE FIGURES

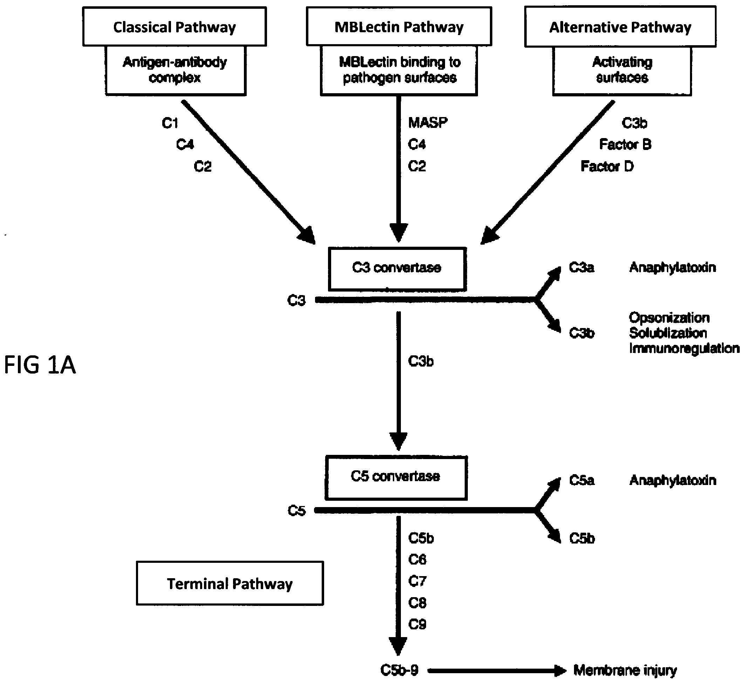

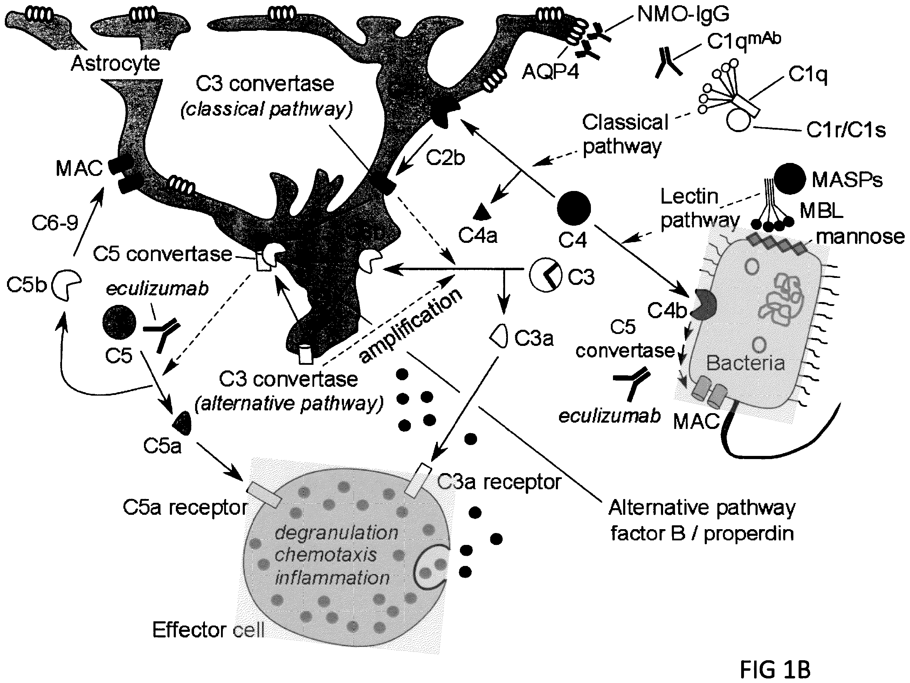

**FIG.** 1 shows schematic representations of the complement system. **FIG. 1A** shows a general schematic representation of the complement cascade, including the three complement activation pathways and the terminal pathway. **FIG. 1B** shows a model of events related to complement activation in neuromyelitis optica (NMO) pathogenesis. According to this model, autoantibody (NMO-IgG) binding to astrocyte AQP4 ultimately results in complement-dependent cytotoxicity (CDC) through the classical pathway. Astrocyte damage is amplified by: *i*) generation of C3a and C5a, which are involved in complement-dependent cell-mediated cytotoxicity (CDCC) by causing effector cell chemotaxis, binding and degranulation, and *ii*) activation of the alternative pathway. Inhibition of C1q binding to NMO-IgG prevents NMO-IgG dependent activation of the classical and alternative pathways, and CDCC. C5 convertase inhibition prevents downstream complement activation though all complement pathways, including the lectin pathway but does not block upstream complement activation events such as anaphylatoxin activity of C3a and opsinization, solubilization and immunoregulation by c3b. MBL: mannose-binding lectin, MASP: mannose-binding lectin-associated serine proteases.

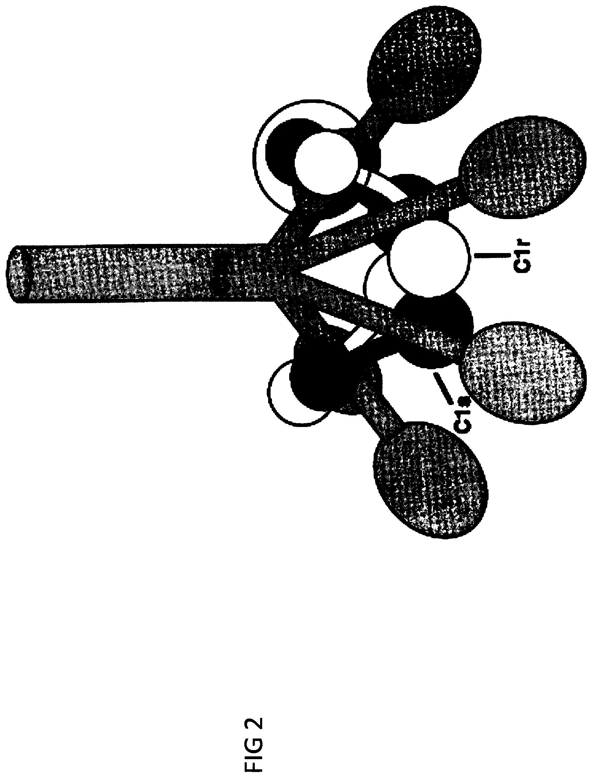

**FIG. 2** shows a schematic of the C1 complex.

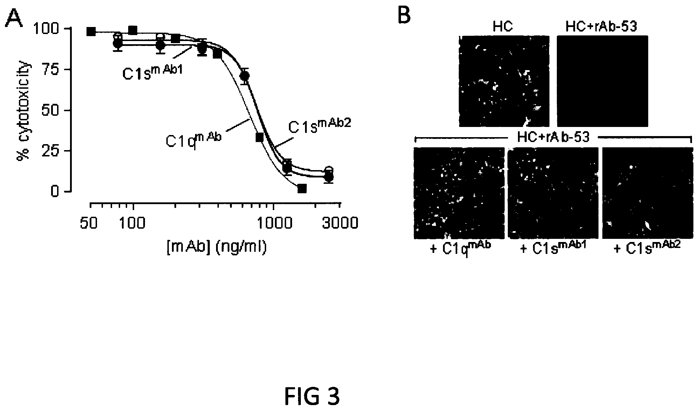

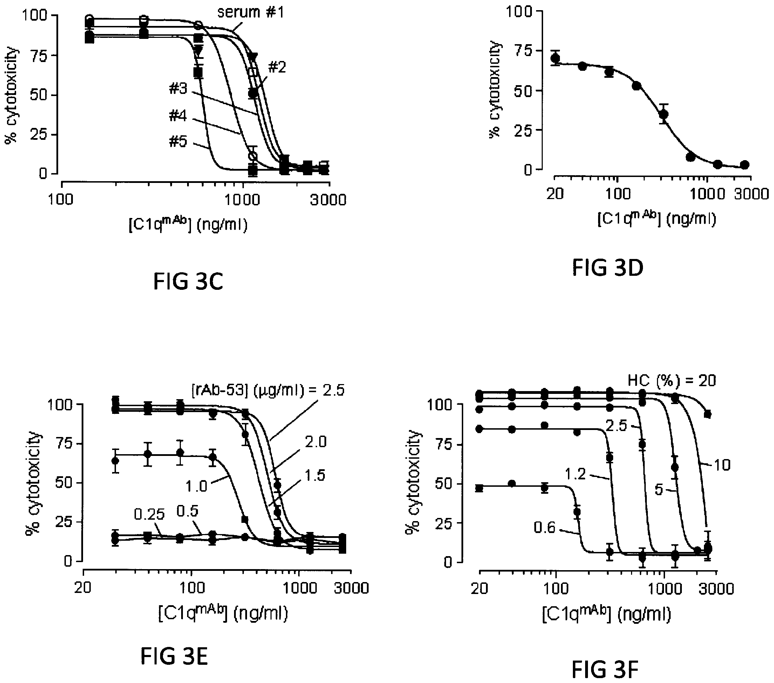

**FIG.** 3 illustrates how C1-targeted monoclonal antibodies can be used to prevent NMO-IgG-dependent, complement-dependent cytotoxicity (CDC). **FIG. 3A** shows CDC in M23-AQP4-expressing CHO cells incubated for 60 min with 1.5 µg/ml rAb-53, 2% human complement (HC), and C1q^{mAb}, C1s^{mAb1} or C1s^{mAb2} (S.E., n = 4). **FIG. 3B** shows live/dead (green/red) staining of cells incubated with 1.5 µg/ml of the monoclonal antibody rAb-53, 2% HC and 2 µg/ml C1q^{mAb}, C1s^{mAb1} or C1s^{mAb2}. **FIG. 3C** shows a graph depicting CDC in cells incubated with serum from 5 different NMO patients (2.5%, heat-inactivated), 2% HC and C1q^{mAb} (S.E., n = 4). **FIG. 3D** is a graph depicting CDC in primary cultures of murine astrocytes incubated with 10 µg/ml rAb-53 (with CDC-enhancing mutation), 5% HC and C1q^{mAb} (S.E., n = 4). **FIG. 3E** is a graph depicting CDC in M23-AQP4-expressing CHO cells incubated with 2% HC and indicated concentrations of rAb-53 and C1q^{mAb} (left, n = 3). **FIG. 3F** is a graph depicting CDC with 1.5 µg/ml rAb-53 and indicated concentrations of HC and C1q^{mAb} (S.E., n = 3).

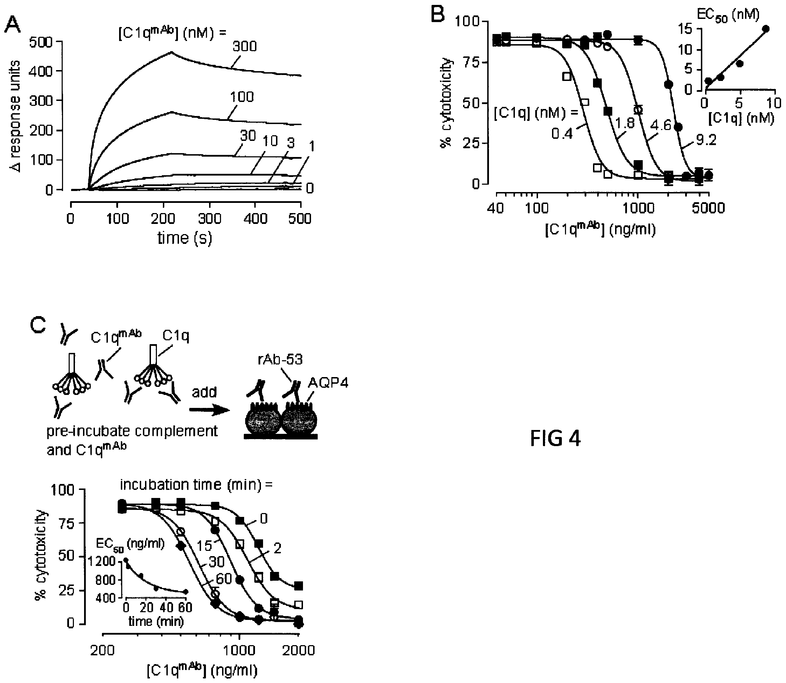

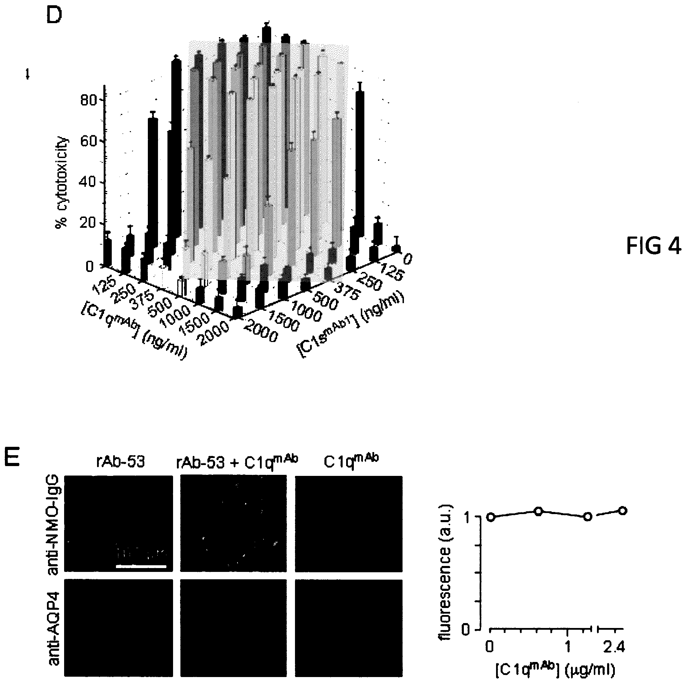

**FIG. 4** illustrates the properties of one anti-C1q antibody (C1q^{mAb}). **FIG. 4A** shows an SPR sensorgram showing concentration-dependent binding of C1q^{mAb} to C1q. Purified C1q protein was ligated onto a CM5 sensor chip. C1q^{mAb} was injected for 180 s over the C1q followed by 300 s washout at a flow rate of 20 µl/min. **FIG. 4B** depicts CDC in M23-AQP4-expressing CHO cells incubated with 1.5 µg/ml of the monoclonal antibody rAb-53, C1q^{mAb} and 2% C1q-depleted serum containing indicated concentrations of purified human C1q protein. Inset shows EC₅₀ vs. C1q concentration. **FIG. 4C** shows CDC in M23-AQP4-expressing CHO cells incubated with 1.5 µg/ml of the monoclonal antibody rAb-53, onto which was added a preincubated (for indicated times) mixture of C1q^{mAb} and 2% HC. Inset shows apparent EC₅₀ *vs.* time. **FIG. 4D** CDC assayed with 1.5 µg/ml rAb-53, 2% HC and different concentrations of C1q^{mAb} and C1s^{mAb1} (S.E., n=3). **FIG. 4E** (left panels) show M23-AQP4-expressing CHO cells were incubated with 1.5 µg/ml rAb-53 (immunostained green), shown with AQP4 immunofluorescence (red), with and without 2.5 µg/ml C1q^{mAb}. **FIG. 4E** (right panel) is a graph showing rAb-53 binding to AQP4 in the presence of C1q^{mAb} measured using a Amplex Red HRP fluorescence assay.

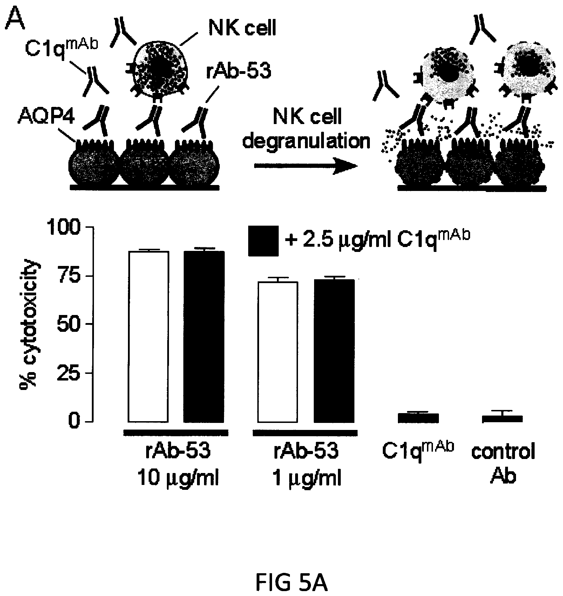

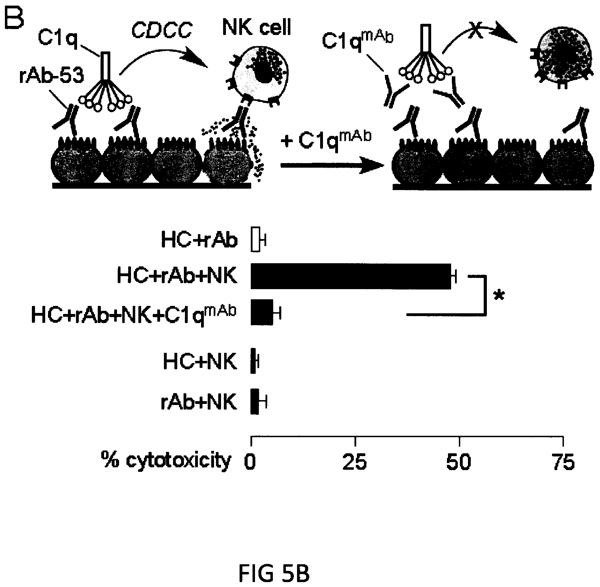

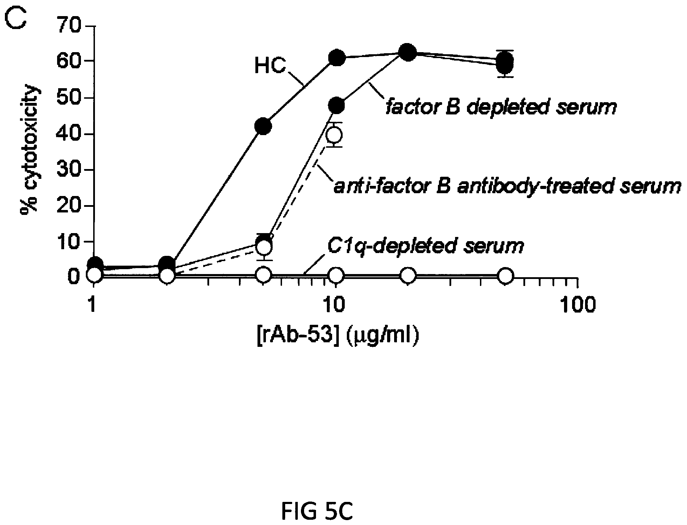

**FIG. 5** illustrates how an anti-C1q antibody (C1q^{mAb}) prevents complement-dependent cell-mediated cytotoxicity (CDCC) but not antibody-dependent cellular cytotoxicity (ADCC). **FIG. 5A** shows ADCC in M23-AQP4-expressing CHO cells incubated with 100,000 NK cells, 1 or 10 µg/ml of the monoclonal antibody rAb-53, with or without 2.5 µg/ml C1q^{mAb}. Controls included NK cells with C1q^{mAb} and the monoclonal antibody rAb-2B4 (control antibody) alone (S.E., n = 4). **FIG. 5B** shows CDCC in M23-AQP4-expressing CHO cells incubated with 100,000 NK cells, 0.25 µg/ml of the monoclonal antibody rAb-53, 2.5 µg/ml C1q^{mAb} and 1% HC. Controls included NK cells with 1% HC and 0.25 µg/ml of the monoclonal antibody rAb-53 alone (S.E., n = 4, *p < 0.01). **FIG. 5C** shows amplification of CDC by the alternative complement pathway. M23-AQP4-expressing CHO cells incubated with the monoclonal antibody rAb-53 together with 2% HC, 2% factor-B depleted human serum, or anti-factor B antibody-treated (100 µg/ml) HC (2%). C1q-depleted serum with rAb-53 shown as control (S.E., n = 4).

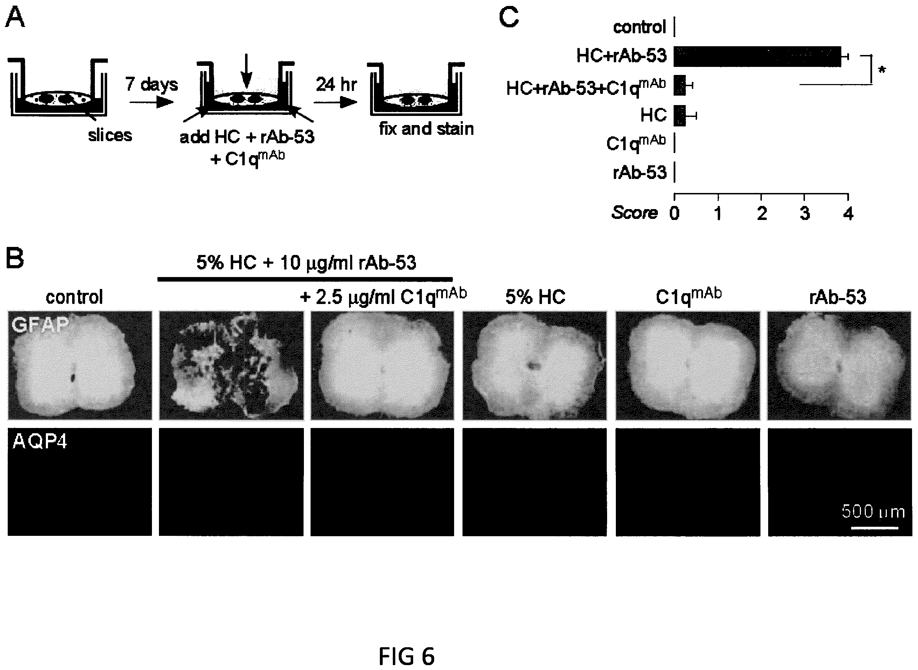

**FIG. 6** illustrates how an anti-C1q antibody (C1q^{mAb}) prevents lesions in an *ex vivo* spinal cord slice mouse model of NMO. **FIG. 6A** shows a schematic of the spinal cord slice model of NMO. Mouse spinal cord slices were cultured for 7 days, followed by 24 h in the presence of 10 µg/ml of the monoclonal antibody rAb-53 and/or 5% HC, with or without 2.5 µg/ml C1q^{mAb}. **FIG. 6B** shows immunofluorescence of AQP4 (red) and GFAP (green). **FIG. 6C** shows NMO lesion scores (S.E., n = 4-6, *p < 0.01).

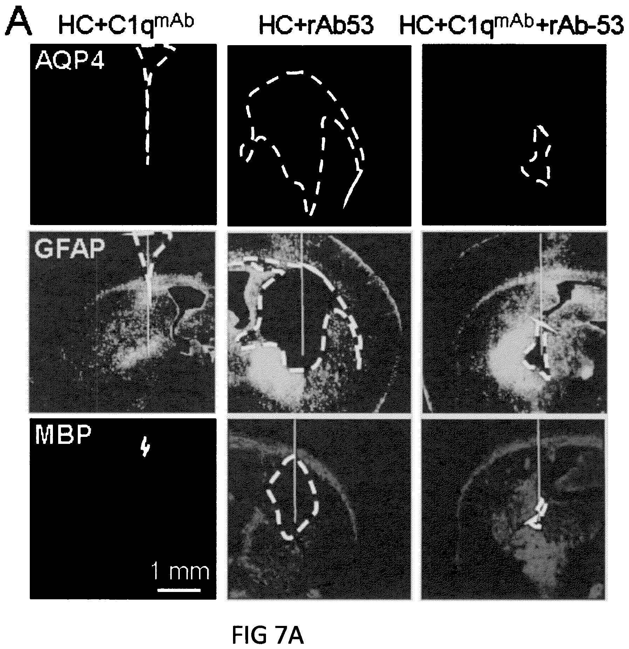

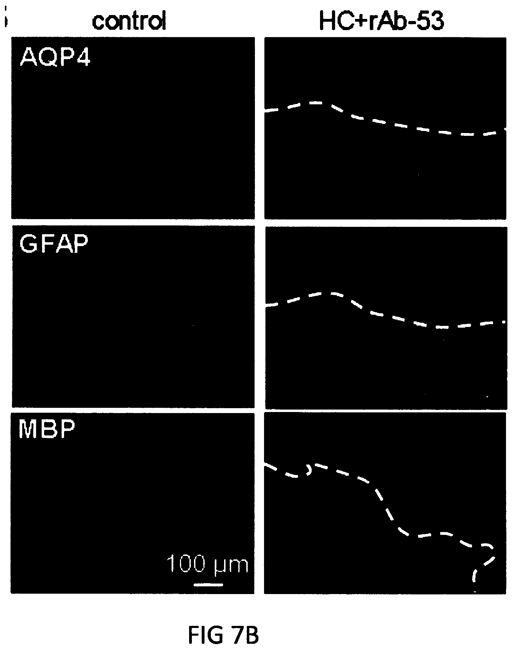

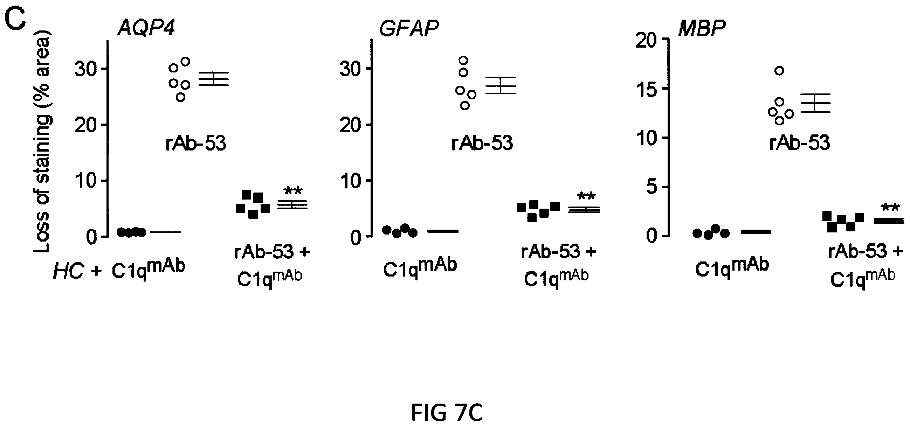

**FIG. 7** illustrates how an anti-C1q antibody (C1q^{mAb}) prevents lesions in an *in vivo* mouse model of NMO involving intracerebral injection of NMO antibody and complement. **FIG. 7A** shows brains injected with 3 µl HC and 1.3 µg C1q^{mAb} (left), 0.9 µg rAb-53 (middle), and 0.9 µg rAb-53 + 1.3 µg C1q^{mAb} (right). Representative GFAP, AQP4 and MBP immunofluorescence at 3 days after injection. Yellow lines represent the needle tract, and white lines delimit the lesion with loss of AQP4, GFAP, and myelin. **FIG. 7B** shows higher magnification of brains injected with rAb-53 and HC. White dashed lines delimit the lesion. Contralateral (noninjected control) hemispheres are shown. **FIG. 7C** is a summary of lesion size from experiments as in A (S.E., 4-5 mice per group, *p < 0.01).

### DETAILED DESCRIPTION OF THE INVENTION

### General techniques

The techniques and procedures described or referenced herein are generally well understood and commonly employed using conventional methodology by those skilled in the art, such as, for example, the widely utilized methodologies described in Sambrook et al., Molecular Cloning: A Laboratory Manual 3d edition (2001) Cold Spring Harbor Laboratory Press, Cold Spring Harbor, N.Y.; Current Protocols in Molecular Biology (F.M. Ausubel, et al. eds., (2003)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, A Laboratory Manual, and Animal Cell Culture (R.I. Freshney, ed. (1987)); Oligonucleotide Synthesis (M.J. Gait, ed., 1984); Methods in Molecular Biology, Humana Press; Cell Biology: A Laboratory Notebook (J.E. Cellis, ed., 1998) Academic Press; Animal Cell Culture (R.I. Freshney), ed., 1987); Introduction to Cell and Tissue Culture (J.P. Mather and P.E. Roberts, 1998) Plenum Press; Cell and Tissue Culture: Laboratory Procedures (A. Doyle, J.B. Griffiths, and D.G. Newell, eds., 1993-8) J. Wiley and Sons; Handbook of Experimental Immunology (D.M. Weir and C.C. Blackwell, eds.); Gene Transfer Vectors for Mammalian Cells (J.M. Miller and M.P. Calos, eds., 1987); PCR: The Polymerase Chain Reaction, (Mullis et al., eds., 1994); Current Protocols in Immunology (J.E. Coligan et al., eds., 1991); Short Protocols in Molecular Biology (Wiley and Sons, 1999); Immunobiology (C.A. Janeway and P. Travers, 1997); Antibodies (P. Finch, 1997); Antibodies: A Practical Approach (D. Catty., ed., IRL Press, 1988-1989); Monoclonal Antibodies: A Practical Approach (P. Shepherd and C. Dean, eds., Oxford University Press, 2000); Using Antibodies: A Laboratory Manual (E. Harlow and D. Lane (Cold Spring Harbor Laboratory Press, 1999); The Antibodies (M. Zanetti and J. D. Capra, eds., Harwood Academic Publishers, 1995); and Cancer: Principles and Practice of Oncology (V.T. DeVita et al., eds., J.B. Lippincott Company, 1993).

### Definitions

As used herein, the term *"preventing"* includes providing prophylaxis with respect to occurrence or recurrence of a particular disease, disorder, or condition in an individual. An individual may be predisposed to, susceptible to a particular disease, disorder, or condition, or at risk of developing such a disease, disorder, or condition, but has not yet been diagnosed with the disease, disorder, or condition.

As used herein, an individual "*at risk*" of developing a particular disease, disorder, or condition may or may not have detectable disease or symptoms of disease, and may or may not have displayed detectable disease or symptoms of disease prior to the treatment methods described herein. "At risk" denotes that an individual has one or more risk factors, which are measurable parameters that correlate with development of a particular disease, disorder, or condition, as known in the art. An individual having one or more of these risk factors has a higher probability of developing a particular disease, disorder, or condition than an individual without one or more of these risk factors.

As used herein, the term *"treatment"* refers to clinical intervention designed to alter the natural course of the individual being treated during the course of clinical pathology. Desirable effects of treatment include decreasing the rate of progression, ameliorating or palliating the pathological state, and remission or improved prognosis of a particular disease, disorder, or condition. An individual is successfully "treated", for example, if one or more symptoms associated with a particular disease, disorder, or condition are mitigated or eliminated.

An *"effective amount"* refers to at least an amount effective, at dosages and for periods of time necessary, to achieve the desired therapeutic or prophylactic result. An effective amount may be provided in one or more administrations.

A "*therapeutically effective amount*" is at least the minimum concentration required to effect a measurable improvement of a particular disease, disorder, or condition. A therapeutically effective amount herein may vary according to factors such as the disease state, age, sex, and weight of the patient, and the ability of the anti-C1q, anti-C1r, or anti-C1s antibody to elicit a desired response in the individual. A therapeutically effective amount may also be one in which any toxic or detrimental effects of the anti-C1q, anti-C1r, or anti-C1s antibody are outweighed by the therapeutically beneficial effects.

*"Chronic"* administration refers to administration of the medicament(s) in a continuous as opposed to acute mode, so as to maintain the initial therapeutic effect (activity) for an extended period of time. *"Intermittent"* administration refers to treatment that is not consecutively done without interruption, but rather is cyclic in nature.

As used herein, administration *"in conjunction"* with another compound or composition includes simultaneous administration and/or administration at different times. Administration in conjunction also encompasses administration as a co-formulation or administration as separate compositions, including at different dosing frequencies or intervals, and using the same route of administration or different routes of administration.

An "*individual*" for purposes of treatment, prevention, or reduction of risk refers to any animal classified as a mammal, including humans, domestic and farm animals, and zoo, sport, or pet animals, such as dogs, horses, rabbits, cattle, pigs, hamsters, gerbils, mice, ferrets, rats, cats, and the like. Preferably, the individual is human.

As used herein, *"autoantibody"* means any antibody that recognizes a host antigen, such as AQP4, in an individual having NMO and activates the classical pathway of complement activation. In the first step of this activation process complement factor C1q binds to the autoantibody-autoantigen-immune complex. Autoantibodies may include naturally occurring antibodies, such as serum antibodies from NMO patients (commonly referred to as NMO-IgG) or monoclonal antibodies, such as rAb-53 *(see, e.g.,* Examples 3 and 5-7).

The term "*immunoglobulin*" (Ig) is used interchangeably with *"antibody"* herein. The term "antibody" herein is used in the broadest sense and specifically covers monoclonal antibodies, polyclonal antibodies, multispecific antibodies (e.g. bispecific antibodies) formed from at least two intact antibodies, and antibody fragments so long as they exhibit the desired biological activity.

The basic 4-chain antibody unit is a heterotetrameric glycoprotein composed of two identical light (L) chains and two identical heavy (H) chains. The pairing of a V_{H} and V_{L} together forms a single antigen-binding site. For the structure and properties of the different classes of antibodies, *see, e.g*., Basic and Clinical Immunology, 8th Ed., Daniel P. Stites, Abba I. Terr and Tristram G. Parslow (eds.), Appleton & Lange, Norwalk, CT, 1994, page 71 and Chapter 6.

The L chain from any vertebrate species can be assigned to one of two clearly distinct types, called kappa ("κ") and lambda ("λ"), based on the amino acid sequences of their constant domains. Depending on the amino acid sequence of the constant domain of their heavy chains (CH), immunoglobulins can be assigned to different classes or isotypes. There are five classes of immunoglobulins: IgA, IgD, IgE, IgG, and IgM, having heavy chains designated alpha ("α"), delta ("δ"), epsilon ("ε"), gamma ("γ") and mu ("µ"), respectively. The y and α classes are further divided into subclasses (isotypes) on the basis of relatively minor differences in the CH sequence and function, *e.g*., humans express the following subclasses: IgG1, IgG2, IgG3, IgG4, IgA1, and IgA2. The subunit structures and three dimensional configurations of different classes of immunoglobulins are well known and described generally in, for example, Abbas et al., Cellular and Molecular Immunology, 4th ed. (W.B. Saunders Co., 2000).

*"Native antibodies"* are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light chain variable domain is aligned with the variable domain of the heavy chain. Particular amino acid residues are believed to form an interface between the light chain and heavy chain variable domains.

An *"isolated"* antibody, such as an anti-C1q, C1r, or C1s antibody of the present disclosure, is one that has been identified, separated and/or recovered from a component of its production environment (*e.g*., naturally or recombinantly). Preferably, the isolated polypeptide is free of association with all other contaminant components from its production environment. Contaminant components from its production environment, such as those resulting from recombinant transfected cells, are materials that would typically interfere with research, diagnostic or therapeutic uses for the antibody, and may include enzymes, hormones, and other proteinaceous or non-proteinaceous solutes. In preferred embodiments, the polypeptide will be purified: (1) to greater than 95% by weight of antibody as determined by, for example, the Lowry method, and in some embodiments, to greater than 99% by weight; (2) to a degree sufficient to obtain at least 15 residues of N-terminal or internal amino acid sequence by use of a spinning cup sequenator, or (3) to homogeneity by SDS-PAGE under non-reducing or reducing conditions using Coomassie blue or, preferably, silver stain. Isolated antibody includes the antibody *in situ* within recombinant T-cells since at least one component of the antibody's natural environment will not be present. Ordinarily, however, an isolated polypeptide or antibody will be prepared by at least one purification step.

The *"variable region"* or *"variable domain"* of an antibody, such as an anti-C1q, C1r, or C1s antibody of the present disclosure, refers to the amino-terminal domains of the heavy or light chain of the antibody. The variable domains of the heavy chain and light chain may be referred to as "V_{H}" and "V_{L}", respectively. These domains are generally the most variable parts of the antibody (relative to other antibodies of the same class) and contain the antigen binding sites.

The term *"variable"* refers to the fact that certain segments of the variable domains differ extensively in sequence among antibodies, such as anti-C1q, C1r, or C1s antibodies of the present disclosure,. The V domain mediates antigen binding and defines the specificity of a particular antibody for its particular antigen. However, the variability is not evenly distributed across the entire span of the variable domains. Instead, it is concentrated in three segments called hypervariable regions (HVRs) both in the light-chain and the heavy chain variable domains. The more highly conserved portions of variable domains are called the framework regions (FR). The variable domains of native heavy and light chains each comprise four FR regions, largely adopting a beta-sheet configuration, connected by three HVRs, which form loops connecting, and in some cases forming part of, the beta-sheet structure. The HVRs in each chain are held together in close proximity by the FR regions and, with the HVRs from the other chain, contribute to the formation of the antigen binding site of antibodies (*see* Kabat et al., Sequences of Immunological Interest, Fifth Edition, National Institute of Health, Bethesda, MD (1991)). The constant domains are not involved directly in the binding of antibody to an antigen, but exhibit various effector functions, such as participation of the antibody in antibody-dependent-cellular toxicity.

The term *"monoclonal antibody*" as used herein refers to an antibody, such as an anti-C1q, C1r, or C1s antibody of the present disclosure, obtained from a population of substantially homogeneous antibodies, *i.e.,* the individual antibodies comprising the population are identical except for possible naturally occurring mutations and/or post-translation modifications (*e.g*., isomerizations, amidations) that may be present in minor amounts. Monoclonal antibodies are highly specific, being directed against a single antigenic site. In contrast to polyclonal antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including, for example, the hybridoma method *(e.g.,* Kohler and Milstein., Nature, 256:495-97 (1975); Hongo et al., Hybridoma, 14 (3):253-260 (1995), Harlow et al., Antibodies: A Laboratory Manual, (Cold Spring Harbor Laboratory Press, 2d ed. 1988); Hammerling et al., in: Monoclonal Antibodies and T-Cell Hybridomas 563-681 (Elsevier, N.Y., 1981)), recombinant DNA methods (see, *e.g.,* U.S. Patent No. 4,816,567), phage-display technologies (*see, e.g.,* Clackson et al., Nature, 352:624-628 (1991); Marks et al., J. Mol. Biol. 222:581-597 (1992); Sidhu et al., J. Mol. Biol. 338(2): 299-310 (2004); Lee et al., J. Mol. Biol. 340(5):1073-1093 (2004); Fellouse, Proc. Nat'l Acad. Sci. USA 101(34):12467-472 (2004); and Lee et al., J. Immunol. Methods 284(1-2):119-132 (2004), and technologies for producing human or human-like antibodies in animals that have parts or all of the human immunoglobulin loci or genes encoding human immunoglobulin sequences (see, *e.g.,* WO 1998/24893; WO 1996/34096; WO 1996/33735; WO 1991/10741; Jakobovits et al., Proc. Nat'l Acad. Sci. USA 90:2551 (1993); Jakobovits et al., Nature 362:255-258 (1993); Bruggemann et al., Year in Immunol. 7:33 (1993); U.S. Patent Nos. 5,545,807; 5,545,806; 5,569,825; 5,625,126; 5,633,425; and 5,661,016; Marks et al., Bio/Technology 10:779-783 (1992); Lonberg et al., Nature 368:856-859 (1994); Morrison, Nature 368:812-813 (1994); Fishwild et al., Nature Biotechnol. 14:845-851 (1996); Neuberger, Nature Biotechnol. 14:826 (1996); and Lonberg and Huszar, Intern. Rev. Immunol. 13:65-93 (1995).

The terms *"full-length antibody," "intact antibody"* or *"whole antibody"* are used interchangeably to refer to an antibody, such as and anti-C1q, C1r, or C1s antibody of the present disclosure, in its substantially intact form, as opposed to an antibody fragment. Specifically whole antibodies include those with heavy and light chains including an Fc region. The constant domains may be native sequence constant domains (*e.g*., human native sequence constant domains) or amino acid sequence variants thereof. In some cases, the intact antibody may have one or more effector functions.

An *"antibody fragment"* comprises a portion of an intact antibody, preferably the antigen binding and/or the variable region of the intact antibody. Examples of antibody fragments include Fab, Fab', F(ab')₂ and Fv fragments; diabodies; linear antibodies *(see* U.S. Patent 5,641,870, Example 2; Zapata et al., Protein Eng. 8(10):1057-1062 (1995)); single-chain antibody molecules and multispecific antibodies formed from antibody fragments.

Papain digestion of antibodies, such as anti-C1q, C1r, or C1s antibodies of the present disclosure, produces two identical antigen-binding fragments, called *"Fab"* fragments, and a residual "*Fc*" fragment, a designation reflecting the ability to crystallize readily. The Fab fragment consists of an entire L chain along with the variable region domain of the H chain (V_{H}), and the first constant domain of one heavy chain (C_{H}1). Each Fab fragment is monovalent with respect to antigen binding, *i.e.,* it has a single antigen-binding site. Pepsin treatment of an antibody yields a single large F(ab')₂ fragment which roughly corresponds to two disulfide linked Fab fragments having different antigen-binding activity and is still capable of cross-linking antigen. Fab' fragments differ from Fab fragments by having a few additional residues at the carboxy terminus of the C_{H}1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The Fc fragment comprises the carboxy-terminal portions of both H chains held together by disulfides. The effector functions of antibodies are determined by sequences in the Fc region, the region which is also recognized by Fc receptors (FcR) found on certain types of cells.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three HVRs specific for an antigen) has the ability to recognize and bind antigen, although at a lower affinity than the entire binding site.

*"Single-chain Fv"* also abbreviated as *"sFv"* or *"scFv*" are antibody fragments that comprise the VH and VL antibody domains connected into a single polypeptide chain. Preferably, the sFv polypeptide further comprises a polypeptide linker between the V_{H} and V_{L} domains which enables the sFv to form the desired structure for antigen binding. For a review of the sFv, *see* Plückthun in The Pharmacology of Monoclonal Antibodies, vol. 113, Rosenburg and Moore eds., Springer-Verlag, New York, pp. 269-315 (1994).

*"Functional fragments"* of antibodies, such as anti-C1q, C1r, or C1s antibodies of the present disclosure, comprise a portion of an intact antibody, generally including the antigen binding or variable region of the intact antibody or the F region of an antibody which retains or has modified FcR binding capability. Examples of antibody fragments include linear antibody, single-chain antibody molecules and multispecific antibodies formed from antibody fragments.

The term *"diabodies"* refers to small antibody fragments prepared by constructing sFv fragments (see preceding paragraph) with short linkers (about 5-10) residues) between the V_{H} and V_{L} domains such that inter-chain but not intra-chain pairing of the V domains is achieved, thereby resulting in a bivalent fragment, *i.e.,* a fragment having two antigen-binding sites. Bispecific diabodies are heterodimers of two "crossover" sFv fragments in which the V_{H} and V_{L} domains of the two antibodies are present on different polypeptide chains. Diabodies are described in greater detail in, for example, EP 404,097; WO 93/11161; Hollinger et al., Proc. Nat'l Acad. Sci. USA 90:6444-48 (1993).

As used herein, a *"chimeric antibody"* refers to an antibody (immunoglobulin), such as an anti-C1q, C1r, or C1s antibody of the present disclosure, in which a portion of the heavy and/or light chain is identical with or homologous to corresponding sequences in antibodies derived from a particular species or belonging to a particular antibody class or subclass, while the remainder of the chain(s) is(are) identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Nat'l Acad. Sci. USA, 81:6851-55 (1984)). Chimeric antibodies of interest herein include PRIMATIZED® antibodies wherein the antigen-binding region of the antibody is derived from an antibody produced by, *e.g*., immunizing macaque monkeys with an antigen of interest. As used herein, *"humanized antibody"* is a subset of "chimeric antibodies."

*"Humanized"* forms of non-human (*e.g.,* murine) antibodies, such as anti-C1q, C1r, or C1s antibodies of the present disclosure, are chimeric antibodies that contain minimal sequence derived from non-human immunoglobulin. In one embodiment, a humanized antibody is a human immunoglobulin (recipient antibody) in which residues from an HVR of the recipient are replaced by residues from an HVR of a non-human species (donor antibody) such as mouse, rat, rabbit or non-human primate having the desired specificity, affinity, and/or capacity. In some instances, FR residues of the human immunoglobulin are replaced by corresponding non-human residues. Furthermore, humanized antibodies may comprise residues that are not found in the recipient antibody or in the donor antibody. These modifications may be made to further refine antibody performance, such as binding affinity. In general, a humanized antibody will comprise substantially all of at least one, and typically two, variable domains, in which all or substantially all of the hypervariable loops correspond to those of a non-human immunoglobulin sequence, and all or substantially all of the FR regions are those of a human immunoglobulin sequence, although the FR regions may include one or more individual FR residue substitutions that improve antibody performance, such as binding affinity, isomerization, immunogenicity, and the like. The number of these amino acid substitutions in the FR is typically no more than 6 in the H chain, and in the L chain, no more than 3. The humanized antibody optionally will also comprise at least a portion of an immunoglobulin constant region (Fc), typically that of a human immunoglobulin. For further details, *see, e.g.,* Jones et al., Nature 321:522-525 (1986); Riechmann et al., Nature 332:323-329 (1988); and Presta, Curr. Op. Struct. Biol. 2:593-596 (1992). *See also,* for example, Vaswani and Hamilton, Ann. Allergy, Asthma & Immunol. 1:105-115 (1998); Harris, Biochem. Soc. Transactions 23:1035-1038 (1995); Hurle and Gross, Curr. Op. Biotech. 5:428-433 (1994); and U.S. Patent Nos. 6,982,321 and 7,087,409.

A *"human antibody"* is one that possesses an amino-acid sequence corresponding to that of an antibody, such as an anti-C1q, C1r, or C1s antibody of the present disclosure, produced by a human and/or has been made using any of the techniques for making human antibodies as disclosed herein. This definition of a human antibody specifically excludes a humanized antibody comprising non-human antigen-binding residues. Human antibodies can be produced using various techniques known in the art, including phage-display libraries. Hoogenboom and Winter, J. Mol. Biol., 227:381 (1991); Marks et al., J. Mol. Biol., 222:581 (1991). Also available for the preparation of human monoclonal antibodies are methods described in Cole et al., Monoclonal Antibodies and Cancer Therapy, Alan R. Liss, p. 77 (1985); Boerner et al., J. Immunol., 147(1):86-95 (1991). *See also* van Dijk and van de Winkel, Curr. Opin. Pharmacol. 5:368-74 (2001). Human antibodies can be prepared by administering the antigen to a transgenic animal that has been modified to produce such antibodies in response to antigenic challenge, but whose endogenous loci have been disabled, *e.g*., immunized xenomice *(see, e.g.,* U.S. Patent Nos. 6,075,181 and 6,150,584 regarding XENOMOUSE™ technology). *See also,* for example, Li et al., Proc. Nat'l Acad. Sci. USA, 103:3557-3562 (2006) regarding human antibodies generated via a human B-cell hybridoma technology.

The term *"hypervariable region," "HVR,"* or "*HV*," when used herein refers to the regions of an antibody-variable domain, such as that of an anti-C1q, C1r, or C1s antibody of the present disclosure, that are hypervariable in sequence and/or form structurally defined loops. Generally, antibodies comprise six HVRs; three in the VH (H1, H2, H3), and three in the VL (L1, L2, L3). In native antibodies, H3 and L3 display the most diversity of the six HVRs, and H3 in particular is believed to play a unique role in conferring fine specificity to antibodies. *See, e.g.,* Xu et al., Immunity 13:37-45 (2000); Johnson and Wu in Methods in Molecular Biology 248:1-25 (Lo, ed., Human Press, Totowa, NJ, 2003)). Indeed, naturally occurring camelid antibodies consisting of a heavy chain only are functional and stable in the absence of light chain. See, *e.g.,* Hamers-Casterman et al., Nature 363:446-448 (1993) and Sheriff et al., Nature Struct. Biol. 3:733-736 (1996).

A number of HVR delineations are in use and are encompassed herein. The HVRs that are Kabat complementarity-determining regions (CDRs) are based on sequence variability and are the most commonly used (Kabat et al., *supra*)*.* Chothia refers instead to the location of the structural loops (Chothia and Lesk J. Mol. Biol. 196:901-917 (1987)). The AbM HVRs represent a compromise between the Kabat CDRs and Chothia structural loops, and are used by Oxford Molecular's AbM antibody-modeling software. The "contact" HVRs are based on an analysis of the available complex crystal structures. The residues from each of these HVRs are noted below.

| Loop | Kabat | AbM | Chothia | Contact |
|---|---|---|---|---|
| L1 | L24-L34 | L24-L34 | L26-L32 | L30-L36 |
| L2 | L50-L56 | L50-L56 | L50-L52 | L46-L55 |
| L3 | L89-L97 | L89-L97 | L91-L96 | L89-L96 |
| H1 | H31-H35B | H26-H35B | H26-H32 | H30-H35B (Kabat numbering) |
| H1 | H31-H35 | H26-H35 | H26-H32 | H30-H35 (Chothia numbering) |
| H2 | H50-H65 | H50-H58 | H53-H55 | H47-H58 |
| H3 | H95-H102 | H95-H102 | H96-H101 | H93-H101 |

HVRs may comprise "extended HVRs" as follows: 24-36 or 24-34 (L1), 46-56 or 50-56 (L2), and 89-97 or 89-96 (L3) in the VL, and 26-35 (H1), 50-65 or 49-65 (a preferred embodiment) (H2), and 93-102, 94-102, or 95-102 (H3) in the VH. The variable-domain residues are numbered according to Kabat et al., *supra*, for each of these extended-HVR definitions.

"*Framework*" or "*FR*" residues are those variable-domain residues other than the HVR residues as herein defined.

The phrase *"variable-domain residue-numbering as in Kabat"* or *"amino-acid-position numbering as in Kabat,"* and variations thereof, refers to the numbering system used for heavy-chain variable domains or light-chain variable domains of the compilation of antibodies in Kabat et al., *supra.* Using this numbering system, the actual linear amino acid sequence may contain fewer or additional amino acids corresponding to a shortening of, or insertion into, a FR or HVR of the variable domain. For example, a heavy-chain variable domain may include a single amino acid insert (residue 52a according to Kabat) after residue 52 of H2 and inserted residues (*e.g.,* residues 82a, 82b, and 82c, *etc.* according to Kabat) after heavy-chain FR residue 82. The Kabat numbering of residues may be determined for a given antibody by alignment at regions of homology of the sequence of the antibody with a "standard" Kabat numbered sequence.

The Kabat numbering system is generally used when referring to a residue in the variable domain (approximately residues 1-107 of the light chain and residues 1-113 of the heavy chain) (*e.g*., Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (*e.g.,* the EU index reported in Kabat *et al*., *supra*)*.* The "EU index as in Kabat" refers to the residue numbering of the human IgG1 EU antibody. Unless stated otherwise herein, references to residue numbers in the variable domain of antibodies means residue numbering by the Kabat numbering system. Unless stated otherwise herein, references to residue numbers in the constant domain of antibodies means residue numbering by the EU numbering system (*e.g*., *see* United States Patent Publication No. 2010-280227).

An *"acceptor human framework"* as used herein is a framework comprising the amino acid sequence of a VL or VH framework derived from a human immunoglobulin framework or a human consensus framework. An acceptor human framework "derived from" a human immunoglobulin framework or a human consensus framework may comprise the same amino acid sequence thereof, or it may contain pre-existing amino acid sequence changes. In some embodiments, the number of pre-existing amino acid changes are 10 or less, 9 or less, 8 or less, 7 or less, 6 or less, 5 or less, 4 or less, 3 or less, or 2 or less. Where pre-existing amino acid changes are present in a VH, preferable those changes occur at only three, two, or one of positions 71H, 73H and 78H; for instance, the amino acid residues at those positions may by 71A, 73T and/or 78A. In one embodiment, the VL acceptor human framework is identical in sequence to the VL human immunoglobulin framework sequence or human consensus framework sequence.

A "*human consensus framework*" is a framework that represents the most commonly occurring amino acid residues in a selection of human immunoglobulin VL or VH framework sequences. Generally, the selection of human immunoglobulin VL or VH sequences is from a subgroup of variable domain sequences. Generally, the subgroup of sequences is a subgroup as in Kabat et al., Sequences of Proteins of Immunological Interest, 5th Ed. Public Health Service, National Institutes of Health, Bethesda, MD (1991). Examples include for the VL, the subgroup may be subgroup kappa I, kappa II, kappa III or kappa IV as in Kabat *et al*., *supra.* Additionally, for the VH, the subgroup may be subgroup I, subgroup II, or subgroup III as in Kabat *et al*., *supra.*

An *"amino-acid modification*" at a specified position, *e.g.,* of an anti-C1q, C1r, or C1s antibody of the present disclosure, refers to the substitution or deletion of the specified residue, or the insertion of at least one amino acid residue adjacent the specified residue. Insertion "adjacent" to a specified residue means insertion within one to two residues thereof. The insertion may be N-terminal or C-terminal to the specified residue. The preferred amino acid modification herein is a substitution.

An "*affinity-matured*" antibody, such as an anti-C1q, C1r, or C1s antibody of the present disclosure, is one with one or more alterations in one or more HVRs thereof that result in an improvement in the affinity of the antibody for antigen, compared to a parent antibody that does not possess those alteration(s). In one embodiment, an affinity-matured antibody has nanomolar or even picomolar affinities for the target antigen. Affinity-matured antibodies are produced by procedures known in the art. For example, Marks et al., Bio/Technology 10:779-783 (1992) describes affinity maturation by VH- and VL-domain shuffling. Random mutagenesis of HVR and/or framework residues is described by, for example: Barbas et al. Proc Nat. Acad. Sci. USA 91:3809-3813 (1994); Schier et al. Gene 169:147-155 (1995); Yelton et al. J. Immunol. 155:1994-2004 (1995); Jackson et al., J. Immunol. 154(7):3310-9 (1995); and Hawkins et al, J. Mol. Biol. 226:889-896 (1992).

As use herein, the term *"specifically recognizes"* or "*specifically binds*" refers to measurable and reproducible interactions such as attraction or binding between a target and an antibody, such as an anti-C1q, C1r, or C1s antibody of the present disclosure, that is determinative of the presence of the target in the presence of a heterogeneous population of molecules including biological molecules. For example, an antibody, such as an anti-C1q, C1r, or C1s antibody of the present disclosure, that specifically or preferentially binds to a target or an epitope is an antibody that binds this target or epitope with greater affinity, avidity, more readily, and/or with greater duration than it binds to other targets or other epitopes of the target. It is also understood by reading this definition that, for example, an antibody (or a moiety) that specifically or preferentially binds to a first target may or may not specifically or preferentially bind to a second target. As such, *"specific binding"* or *"preferential binding"* does not necessarily require (although it can include) exclusive binding. An antibody that specifically binds to a target may have an association constant of at least about 10³ M⁻¹ or 10⁴ M⁻¹, sometimes about 10⁵ M⁻¹ or 10⁶ M ⁻¹, in other instances about 10⁶ M⁻¹ or 10⁷ M⁻¹, about 10⁸ M⁻¹ to 10⁹ M⁻¹, or about 10¹⁰ M⁻¹ to 10¹¹ M⁻¹ or higher. A variety of immunoassay formats can be used to select antibodies specifically immunoreactive with a particular protein. For example, solid-phase ELISA immunoassays are routinely used to select monoclonal antibodies specifically immunoreactive with a protein. *See, e.g.,* Harlow and Lane (1988) Antibodies, A Laboratory Manual, Cold Spring Harbor Publications, New York, for a description of immunoassay formats and conditions that can be used to determine specific immunoreactivity.

As used herein, an *"interaction"* between a complement protein, such as complement factors C1q, C1r, and C1s, and a second protein encompasses, without limitation, protein-protein interaction, a physical interaction, a chemical interaction, binding, covalent binding, and ionic binding. As used herein, an antibody "inhibits interaction" between two proteins when the antibody disrupts, reduces, or completely eliminates an interaction between the two proteins. An antibody of the present disclosure, or fragment thereof, "inhibits interaction" between two proteins when the antibody or fragment thereof binds to one of the two proteins.

A *"blocking"* antibody, an "*antagonist*" antibody, an *"inhibitory"* antibody, or a *"neutralizing"* antibody is an antibody, such as an anti-C1q, C1r, or C1s antibody of the present disclosure that inhibits or reduces one or more biological activities of the antigen it binds, such as interactions with one or more proteins. In some embodiments, blocking antibodies, antagonist antibodies, inhibitory antibodies, or *"neutralizing"* antibodies substantially or completely inhibit one or more biological activities or interactions of the antigen.

Antibody *"effector functions"* refer to those biological activities attributable to the Fc region (a native sequence Fc region or amino acid sequence variant Fc region) of an antibody, and vary with the antibody isotype.

The term *"Fc region"* herein is used to define a C-terminal region of an immunoglobulin heavy chain, including native-sequence Fc regions and variant Fc regions. Although the boundaries of the Fc region of an immunoglobulin heavy chain might vary, the human IgG heavy-chain Fc region is usually defined to stretch from an amino acid residue at position Cys226, or from Pro230, to the carboxyl-terminus thereof. The C-terminal lysine (residue 447 according to the EU numbering system) of the Fc region may be removed, for example, during production or purification of the antibody, or by recombinantly engineering the nucleic acid encoding a heavy chain of the antibody. Accordingly, a composition of intact antibodies may comprise antibody populations with all K447 residues removed, antibody populations with no K447 residues removed, and antibody populations having a mixture of antibodies with and without the K447 residue. Suitable native-sequence Fc regions for use in the antibodies of the invention include human IgG1, IgG2, IgG3 and IgG4.

A "*native sequence Fc region"* comprises an amino acid sequence identical to the amino acid sequence of an Fc region found in nature. Native sequence human Fc regions include a native sequence human IgG1 Fc region (non-A and A allotypes); native sequence human IgG2 Fc region; native sequence human IgG3 Fc region; and native sequence human IgG4 Fc region as well as naturally occurring variants thereof.

A "*variant Fc region"* comprises an amino acid sequence which differs from that of a native sequence Fc region by virtue of at least one amino acid modification, preferably one or more amino acid substitution(s). Preferably, the variant Fc region has at least one amino acid substitution compared to a native sequence Fc region or to the Fc region of a parent polypeptide, *e.g.* from about one to about ten amino acid substitutions, and preferably from about one to about five amino acid substitutions in a native sequence Fc region or in the Fc region of the parent polypeptide. The variant Fc region herein will preferably possess at least about 80% homology with a native sequence Fc region and/or with an Fc region of a parent polypeptide, and most preferably at least about 90% homology therewith, more preferably at least about 95% homology therewith.

"*Fc receptor"* or *"FcR"* describes a receptor that binds to the Fc region of an antibody. The preferred FcR is a native sequence human FcR. Moreover, a preferred FcR is one which binds an IgG antibody (a gamma receptor) and includes receptors of the FcyRI, FcyRII, and FcγRIII subclasses, including allelic variants and alternatively spliced forms of these receptors, FcyRII receptors include FcyRIIA (an "activating receptor") and FcyRIIB (an "inhibiting receptor"), which have similar amino acid sequences that differ primarily in the cytoplasmic domains thereof. Activating receptor FcyRIIA contains an immunoreceptor tyrosine-based activation motif ("ITAM") in its cytoplasmic domain. Inhibiting receptor FcyRIIB contains an immunoreceptor tyrosine-based inhibition motif ("ITIM") in its cytoplasmic domain. (*see, e.g.,* M. Daëron, Annu. Rev. Immunol. 15:203-234 (1997)). FcRs are reviewed in Ravetch and Kinet, Annu. Rev. Immunol. 9:457-92 (1991); Capel et al., Immunomethods 4:25-34 (1994); and de Haas et al., J. Lab. Clin. Med. 126: 330-41 (1995). Other FcRs, including those to be identified in the future, are encompassed by the term "FcR" herein. FcRs can also increase the serum half-life of antibodies.

Binding to FcRn *in vivo* and serum half-life of human FcRn high-affinity binding polypeptides can be assayed, *e.g*., in transgenic mice or transfected human cell lines expressing human FcRn, or in primates to which the polypeptides having a variant Fc region are administered. WO 2004/42072 (Presta) describes antibody variants with improved or diminished binding to FcRs. *See also, e.g*., Shields et al., J. Biol. Chem. 9(2):6591-6604 (2001).

The term "kₒₙ", as used herein, is intended to refer to the rate constant for association of an antibody to an antigen.

The term "k_{off}", as used herein, is intended to refer to the rate constant for dissociation of an antibody from the antibody/antigen complex.

The term "K_{D}", as used herein, is intended to refer to the equilibrium dissociation constant of an antibody-antigen interaction.

As used herein, "*percent (%) amino acid sequence identity"* and "*homology*" with respect to a peptide, polypeptide or antibody sequence refers to the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or MEGALIGN™ (DNASTAR) software. Those skilled in the art can determine appropriate parameters for measuring alignment, including any algorithms known in the art needed to achieve maximal alignment over the full length of the sequences being compared.

An *"isolated"* molecule or cell is a molecule or a cell that is identified and separated from at least one contaminant molecule or cell with which it is ordinarily associated in the environment in which it was produced. Preferably, the isolated molecule or cell is free of association with all components associated with the production environment. The isolated molecule or cell is in a form other than in the form or setting in which it is found in nature. Isolated molecules therefore are distinguished from molecules existing naturally in cells; isolated cells are distinguished from cells existing naturally in tissues, organs, or individuals. In some embodiments, the isolated molecule is an anti-C1s antibody of the present disclosure. In other embodiments, the isolated cell is a host cell or hybridoma cell producing an anti-C1s antibody of the present disclosure.

An "*isolated*" nucleic acid molecule encoding an antibody, such as an anti-C1q, C1r, or C1s antibody of the present disclosure, is a nucleic acid molecule that is identified and separated from at least one contaminant nucleic acid molecule with which it is ordinarily associated in the environment in which it was produced. Preferably, the isolated nucleic acid is free of association with all components associated with the production environment. The isolated nucleic acid molecules encoding the polypeptides and antibodies herein is in a form other than in the form or setting in which it is found in nature. Isolated nucleic acid molecules therefore are distinguished from nucleic acid encoding the polypeptides and antibodies herein existing naturally in cells.

The term *"vector,"* as used herein, is intended to refer to a nucleic acid molecule capable of transporting another nucleic acid to which it has been linked. One type of vector is a *"plasmid,"* which refers to a circular double stranded DNA into which additional DNA segments may be ligated. Another type of vector is a phage vector. Another type of vector is a viral vector, wherein additional DNA segments may be ligated into the viral genome. Certain vectors are capable of autonomous replication in a host cell into which they are introduced (*e.g*., bacterial vectors having a bacterial origin of replication and episomal mammalian vectors). Other vectors (*e.g*., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as *"recombinant expression vectors,"* or simply, *"expression vectors."* In general, expression vectors of utility in recombinant DNA techniques are often in the form of plasmids. In the present specification, "*plasmid*" and *"vector"* may be used interchangeably as the plasmid is the most commonly used form of vector.

*"Polynucleotide,"* or *"nucleic acid,"* as used interchangeably herein, refer to polymers of nucleotides of any length, and include DNA and RNA. The nucleotides can be deoxyribonucleotides, ribonucleotides, modified nucleotides or bases, and/or their analogs, or any substrate that can be incorporated into a polymer by DNA or RNA polymerase or by a synthetic reaction. A polynucleotide may comprise modified nucleotides, such as methylated nucleotides and their analogs. If present, modification to the nucleotide structure may be imparted before or after assembly of the polymer. The sequence of nucleotides may be interrupted by non-nucleotide components. A polynucleotide may comprise modification(s) made after synthesis, such as conjugation to a label. Other types of modifications include, for example, *"caps,"* substitution of one or more of the naturally occurring nucleotides with an analog, internucleotide modifications such as, for example, those with uncharged linkages (*e.g*., methyl phosphonates, phosphotriesters, phosphoamidates, carbamates, *etc.)* and with charged linkages (*e.g.,* phosphorothioates, phosphorodithioates, *etc.*)*,* those containing pendant moieties, such as, for example, proteins (*e.g*., nucleases, toxins, antibodies, signal peptides, ply-L-lysine, *etc.*)*,* those with intercalators (*e.g.,* acridine, psoralen, *etc.*)*,* those containing chelators (*e.g*., metals, radioactive metals, boron, oxidative metals, *etc.*)*,* those containing alkylators, those with modified linkages (*e.g.,* alpha anomeric nucleic acids, *etc.*)*,* as well as unmodified forms of the polynucleotides(s). Further, any of the hydroxyl groups ordinarily present in the sugars may be replaced, for example, by phosphonate groups, phosphate groups, protected by standard protecting groups, or activated to prepare additional linkages to additional nucleotides, or may be conjugated to solid or semi-solid supports. The 5' and 3' terminal OH can be phosphorylated or substituted with amines or organic capping group moieties of from 1 to 20 carbon atoms. Other hydroxyls may also be derivatized to standard protecting groups. Polynucleotides can also contain analogous forms of ribose or deoxyribose sugars that are generally known in the art, including, for example, 2'-O-methyl-, 2'-O-allyl-, 2'-fluoro- or 2'-azido-ribose, carbocyclic sugar analogs, α-anomeric sugars, epimeric sugars such as arabinose, xyloses or lyxoses, pyranose sugars, furanose sugars, sedoheptuloses, acyclic analogs, and basic nucleoside analogs such as methyl riboside. One or more phosphodiester linkages may be replaced by alternative linking groups. These alternative linking groups include, but are not limited to, embodiments wherein phosphate is replaced by P(O)S ("thioate"), P(S)S ("dithioate"), (O)NR2 ("amidate"), P(O)R, P(O)OR', CO, or CH2 ("formacetal"), in which each R or R' is independently H or substituted or unsubstituted alkyl (1-20 C) optionally containing an ether (-O-) linkage, aryl, alkenyl, cycloalkyl, cycloalkenyl or araldyl. Not all linkages in a polynucleotide need be identical. The preceding description applies to all polynucleotides referred to herein, including RNA and DNA.

A "*host cell*" includes an individual cell or cell culture that can be or has been a recipient for vector(s) for incorporation of polynucleotide inserts. Host cells include progeny of a single host cell, and the progeny may not necessarily be completely identical (in morphology or in genomic DNA complement) to the original parent cell due to natural, accidental, or deliberate mutation. A host cell includes cells transfected *in vivo* with a polynucleotide(s) of this invention.

*"Carriers"* as used herein include pharmaceutically acceptable carriers, excipients, or stabilizers that are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

The term *"about"* as used herein refers to the usual error range for the respective value readily known to the skilled person in this technical field. Reference to *"about"* a value or parameter herein includes (and describes) embodiments that are directed to that value or parameter *per se.*

As used herein and in the appended claims, the singular forms "a," "an," and "the" include plural reference unless the context clearly indicates otherwise. For example, reference to an "antibody" is a reference to from one to many antibodies, such as molar amounts, and includes equivalents thereof known to those skilled in the art, and so forth.

It is understood that aspect and embodiments of the invention described herein include "comprising," "consisting," and "consisting essentially of' aspects and embodiments.

### Overview

Described herein are methods of preventing, reducing risk of developing, or treating individuals having neuromyelitis optica (NMO). Without wishing to be bound by theory, it is believed that inhibition of the classical pathway of complement activation is an effective therapeutic strategy for the treatment of NMO **(****FIG. 1A****)**. It is further believed that effective strategies for inhibiting the classical pathway include inhibiting the interaction between C1q and autoantibodies (*e.g*., NMO-IgG or anti-AQP4 autoantibodies), inhibiting the interaction between C1q and C1r or C1s, blocking the catalytic activity of C1r or C1s, and blocking the interactions between C1r or C1s and their respective substrates **(****FIG. 1A** **and** **1B****).** It is also believed that effective agents for the inhibition of the classical complement pathway include neutralizing antibodies for C1 complex, C1q, C1r, and/or C1s **(****FIG. 2****)**.

Accordingly, certain aspects of the present disclosure relates to anti-C1q antibodies, anti-C1r antibodies, and/or anti-C1s antibodies for use in preventing, reducing risk of developing, or treating neuromyelitis optica (NMO) in individuals in need thereof.

In one aspect, the present disclosure provides antibodies as charactized in the appended claims for use in methods of preventing, reducing risk of developing, or treating an individual having NMO by administering to the individual a therapeutically effective amount of at least one antibody, wherein the at least one antibody is an anti-C1q antibody. In some embodiments, the anti-C1q antibody is a C1q neutralizing antibody. In some embodiments, the anti-C1q antibody binds to C1 complex. In some embodiments, the anti-C1q antibody inhibits the interaction between C1q and an autoantibody, between C1q and C1r, and/or between C1q and C1s.

In another aspect, the present disclosure provides antibodies as charactized in the appended claims for use in methods of preventing, reducing risk of developing, or treating an individual having NMO by administering to the individual a therapeutically effective amount of at least one antibody, wherein the at least one antibody is an anti-C1r antibody. In some embodiments, the anti-C1r antibody is a C1r neutralizing antibody. In some embodiments, the anti-C1r antibody binds to C1 complex. In some embodiments, the anti-C1r antibody inhibits the interaction between C1r and C1q and/or between C1r and C1s. In some embodiments, the anti-C1r antibody inhibits the catalytic activity of C1r or the processing of pro-C1r to an active protease.

In another aspect, the present disclosure provides antibodies as charactized in the appended claims for use in methods of preventing, reducing risk of developing, or treating an individual having NMO by administering to the individual a therapeutically effective amount of at least one antibody, wherein the at least one antibody is an anti-C1s antibody. In some embodiments, the anti-C1s antibody is a C1s neutralizing antibody. In some embodiments, the anti-C1s antibody binds to C1 complex. In some embodiments, the anti-C1s antibody inhibits the interaction between C1s and C1q, between C1s and C1r, and/or between C1s and its substrates C2 and C4. In some embodiments, the anti-C1s antibody inhibits the catalytic activity of C1s or the processing of pro-C1s into an active protease.

### Complement Proteins

The methods of this disclosure involve administering or using antibodies that specifically recognize complement factors C1q, C1r, and/or C1s, or the C1 complex of the classical complement activation pathway. The recognized complement factors may be derived, without limitation, from any organism having a complement system, including any mammalian organism such as human, mouse, rat, rabbit, monkey, dog, cat, cow, horse, camel, sheep, goat, or pig.

As used herein "*C1 complex"* refers to a protein complex that may include, without limitation, one C1q protein, two C1r proteins, and two C1s proteins (*e.g*., C1qr²s²).

As used herein *"complement factors C1q, C1r, or C1s*" refers to both wild type sequences and naturally occurring variant sequences.

### C1q

A non-limiting example of a complement factor C1q recognized by antibodies of this invention is human C1q, including the three polypeptide chains A, B, and C:
*C1q, chain A (homo sapiens)*, Accession No. Protein Data Base: NP_057075.1; GenBank No.: NM_015991:
   >gi|7705753|ref|NP_057075.1| complement C1q subcomponent subunit A precursor [Homo sapiens]
*C1q, chain B (homo sapiens)*, Accession No. Protein Data Base: NP_000482.3; GenBank No.: NM_000491.3:
   >gi|87298828|ref|NP_000482.3| complement C1q subcomponent subunit B precursor [Homo sapiens] 
MMMKIPWGSIPVLMLLLLLGLIDISQAQLSCTGPPAIPGIPGIPGTPGPDGQPGTPGIKGEK
GLPGLAGDHGEFGEKGDPGIPGNPGKVGPKGPMGPKGGPGAPGAPGPKGESGDYKATQ
KIAFSATRTINVPLRRDQTIRFDHVITNMNNNYEPRSGKFTCKVPGLYYFTYHASSRGNL
CVNLMRGRERAQKVVTFCDYAYNTFQVTTGGMVLKLEQGENVFLQATDKNSLLGMEG
ANSIFSGFLLFPDMEA
*C1q, chain C (homo sapiens)*, Accession No. Protein Data Base: NP_001107573.1; GenBank No.: NM_001114101.1:
   >gi|166235903|ref|NP_001107573.1| complement C1q subcomponent subunit C precursor [Homo sapiens]

Accordingly, an anti-C1q antibody of the present disclosure may bind to polypeptide chain A, polypeptide chain B, and/or polypeptide chain C of a C1q protein. In some embodiments, an anti-C1q antibody of the present disclosure binds to polypeptide chain A, polypeptide chain B, and/or polypeptide chain C of human C1q or a homolog thereof, such as mouse, rat, rabbit, monkey, dog, cat, cow, horse, camel, sheep, goat, or pig C1q.

### C1r

A non-limiting example of a complement factor C1r recognized by antibodies of this invention is human C1r (Accession No. Protein Data Base: NP_001724.3; GenBank No.: NM_001733.4):
>gi|66347875|ref|NP_001724.3| complement C1r subcomponent precursor [Homo sapiens]

MWLLYLLVPALFCRAGGSIPIPQKLFGEVTSPLFPKPYPNNFETTTVITVPTGYRVKLVFQ
QDLEPSEGCFYDYVKISADKKSLGRFCGQLGSPLGNPPGKKEFMSQGNKMLLTFHTDFS
NEENGTIMFYKGFLAYYQAVDLDECASRSKLGEEDPQPQCQHLCHNYVGGYFCSCRPG
YELQEDRHSCQAECSSELYTEASGYISSLEYPRSYPPDLRCNYSIRVERGLTLHLKFLEPF
DIDDHQQVHCPYDQLQIYANGKNIGEFCGKQRPPDLDTSSNAVDLLFFTDESGDSRGWK
LRYTTEIIKCPQPKTLDEFTIIQNLQPQYQFRDYFIATCKQGYQLIEGNQVLHSFTAVCQD
DGTWHRAMPRCKIKDCGQPRNLPNGDFRYTTTMGVNTYKARIQYYCHEPYYKMQTRA
GSRESEQGVYTCTAQGIWKNEQKGEKIPRCLPVCGKPVNPVEQRQRIIGGQKAKMGNFP
WQVFTNIHGRGGGALLGDRWILTAAHTLYPKEHEAQSNASLDVFLGHTNVEELMKLGN
HPIRRVSVHPDYRQDESYNFEGDIALLELENSVTLGPNLLPICLPDNDTFYDLGLMGYVS
GFGVMEEKIAHDLRFVRLPVANPQACENWLRGKNRMDVFSQNMFCAGHPSLKQDACQ
GDSGGVFAVRDPNTDRWVATGIVSWGIGCSRGYGFYTKVLNYVDWIKKEMEEED

Accordingly, an anti-C1r antibody of the present disclosure may bind to human C1r or a homolog thereof, such as mouse, rat, rabbit, monkey, dog, cat, cow, horse, camel, sheep, goat, or pig C1r.

### C1s

A non-limiting example of a complement factor C1s recognized by antibodies of this invention is human C1s (Accession No. Protein Data Base: NP_958850.1; GenBank No.: NM_201442.2):
>gi|41393602|ref|NP_958850.1| complement C1s subcomponent precursor [Homo sapiens]

Accordingly, an anti-C1s antibody of the present disclosure may bind to human C1s or a homolog thereof, such as mouse, rat, rabbit, monkey, dog, cat, cow, horse, camel, sheep, goat, or pig C1s.

### Anti-C1q, anti-C1r, and anti-C1s Antibodies

The antibodies of the present disclosure recognize complement factors C1q, C1r, and/or C1s, and/or the C1 complex of the classical complement activation pathway.

In some embodiments, an antibody of the present disclosure is an anti-C1s antibody that comprises a light chain variable domain and a heavy chain variable domain, wherein the light chain variable domain comprises the HVR-L1, HVR-L2, and HVR-L3 and/or the heavy chain variable domain comprises the HVR-H1, HVR-H2, and HVR-H3 of monoclonal antibody 5A1 produced by a hybridoma cell line deposited with ATCC on 5/15/2013 or progeny thereof (ATCC Accession No. PTA-120351). In some embodiments, anti-C1s antibody comprises a light chain variable domain and a heavy chain variable domain, wherein the light chain variable domain comprises the HVR-L1, HVR-L2, and HVR-L3 and/or the heavy chain variable domain comprises the HVR-H1, HVR-H2, and HVR-H3 of a monoclonal antibody 5C12 produced by a hybridoma cell line deposited with ATCC on 5/15/2013 or progeny thereof (ATCC Accession No. PTA-120352). In some embodiments the anti-C1s antibody is humanized. In some embodiments, the anti-C1s antibody is a chimeric antibody.

In some embodiments, the antibodies of the present disclosure neutralize at least one activity of complement factors C1q, C1r, and/or C1s, and/or at least one activity of C1 complex. In some embodiments, the antibodies inhibit the interaction between complement factors C1q, C1r, and/or C1s and autoantibodies, other complement factors, and/or complement protease substrates such as C2 and C4. In some embodiments, the antibodies inhibit the catalytic activity of the serine proteases C1r and C1s and/or inhibit the processing of a serine protease pro-form to an active protease. In some embodiments the antibodies inhibit the classical pathway. In certain embodiments the antibodies further inhibit the alternative pathway. In some embodiments, the antibodies inhibit autoantibody-dependent and complement-dependent cytotoxicity (CDC). In some embodiments, the antibodies further inhibit complement-dependent cell-mediated cytotoxicity (CDCC).

The functional properties of the antibodies of this invention, such as dissociation constants for antigens, inhibition of protein-protein interactions (*e.g.,* C1q-autoantibody interactions), inhibition of autoantibody-dependent and complement-dependent cytotoxicity (CDC), inhibition of complement-dependent cell-mediated cytotoxicity (CDCC), and/or lesion formation, may, without limitation, be measured by *in vitro, ex vivo,* or *in vivo* experiments.

The dissociation constant (K_{D}) of the anti-C1q, anti-C1r, and/or anti-C1s antibodies for their respective antigens may be less than 100nM, less than 90 nM, less than 80 nM, less than 70 nM, less than 60 nM, less than 50 nM, less than 40 nM, less than 30 nM, less than 20 nM, less than 10 nM, less than 9 nM, less than 8 nM, less than 7 nM, less than 6 nM, less than 5 nM, less than 4 nM, less than 3 nM, less than 2 nM, less than 1 nM, less than 0.5 nM, less than 0.1 nM, less than 0.05 nM, less than 0.01 nM, or less than 0.005 nM. In some embodiments, the antibody has a dissociation constant (K_{D}) for its corresponding antigen that ranges from 100 nM to 0.005 nM or less than 0.005 nM. Preferably, dissociation constant is less than 20nM. Antibody dissociation constants for antigens other than C1q, C1r, C1s, and/or C1 complex may be at least 5-fold, at least 10-fold, at least 100-fold, at least 1,000-fold, at least 10,000-fold, or at least 100,000-fold higher than the dissociation constants for their respective antigens. For example, the dissociation constant of a C1q antibody of the present disclosure may be at least 1,000-fold higher for C1s than for C1q. Dissociation constants may be determined through any analytical technique, including any biochemical or biophysical technique such as surface plasmon resonance (SPR), isothermal titration calorimetry (ITC), differential scanning calorimetry (DSC), circular dichroism (CD), stopped-flow analysis, and colorimetric or fluorescent protein melting analyses (*see also* Example 1). Dissociation constants (K_{D}) of the anti-C1q, anti-C1r, and/or anti-C1s antibodies for their respective antigens may be determined, e.g., using full-length antibodies or antibody fragments, such as Fab fragments. The antibodies of the present disclosure may bind to C1q, C1r, C1s, or C1 complex antigens derived from any organism having a complement system, including any mammalian organism such as human, mouse, rat, rabbit, monkey, dog, cat, cow, horse, camel, sheep, goat, or pig. In preferred embodiments, the antibodies of this disclosure bind to epitopes comprising amino acid residues on human C1q, C1r, or C1s.

In some embodiments, an antibody of the present disclosure is an anti-C1q antibody. In some embodiments, the anti-C1q antibody inhibits the interaction between C1q and an autoantibody. In some embodiments, the anti-C1q antibody is antibody 4A4B11 (also referred to as C1q^{mAb} in Examples 1-7; *see also* U.S. Patent No. 4,595,654 and ATCC HB-8327TM) or a humanized antibody of 4A4B11. In certain embodiments, the autoantibody recognizes aquaporin-4 *(e.g.,* NMO-IgG or rAb-53; *see also* Examples 2 and 3). In some embodiments, the anti-C1q antibody inhibits the interaction between C1q and the autoantibody at a stoichiometry of less than 2.5:1; 2.0:1; 1.5:1; or 1.0:1 *(see also* Examples 4 and **FIG. 4C**). In other embodiments, the anti-C1q antibody binds to C1q with a stoichiometry of less than 20:1; less than 19.5:1; less than19:1; less than 18.5:1; less than 18:1; less than 17.5:1; less than 17:1; less than 16.5:1; less than 16:1; less than 15.5:1; less than 15:1; less than 14.5:1; less than 14:1; less than 13.5:1; less than 13:1; less than 12.5:1; less than 12:1; less than 11.5:1; less than 11:1; less than 10.5:1; less than 10:1; less than 9.5:1; less than 9:1; less than 8.5:1; less than 8:1; less than 7.5:1; less than 7:1; less than 6.5:1; less than 6:1; less than 5.5:1; less than 5:1; less than 4.5:1; less than 4:1; less than 3.5:1; less than 3:1; less than 2.5:1; less than 2.0:1; less than 1.5:1; or less than 1.0:1 *(see also* Examples 4 and **FIG. 4C****)**. In certain embodiments, the anti-C1q antibody binds C1q with a binding stoichiometry that ranges from 20:1 to 1.0:1 or less than 1.0:1. In certain embodiments, the anti-C1q antibody binds C1q with a binding stoichiometry that ranges from 6:1 to 1.0:1 or less than1.0:1. In certain embodiments, the anti-C1q antibody binds C1q with a binding stoichiometry that ranges from 2.5:1 to 1.0:1 or less than1.0:1. In some embodiments, the anti-C1q antibody inhibits the interaction between C1q and C1r, or between C1q and C1s, or between C1q and both C1r and C1s. In some embodiments, the anti-C1q antibody inhibits the interaction between C1q and C1r, between C1q and C1s, and/or between C1q and both C1r and C1s. In some embodiments, the anti-C1q antibody binds to the C1q A-chain. In other embodiments, the anti-C1q antibody binds to the C1q B-chain. In other embodiments, the anti-C1q antibody binds to the C1q C-chain. In some embodiments, the anti-C1q antibody binds to the C1q A-chain, the C1q B-chain and/or the C1q C-chain. In some embodiments, the anti-C1q antibody binds to the globular domain of the C1q A-chain, B-chain, and/or C-chain. In other embodiments, the anti-C1q antibody binds to the collagen-like domain of the C1q A-chain, the C1q B-chain, and/or the C1q C-chain.

In some embodiments, an antibody of the present disclosure is an anti-C1r antibody. In some embodiments that anti-C1r antibody inhibits the interaction between C1r and C1q. In some embodiments, the anti-C1r antibody inhibits the interaction between C1r and C1s. In some embodiments, the anti-C1r antibody inhibits the interaction between C1r and C1q and between C1r and C1s. In some embodiments, the anti-C1r antibody inhibits the catalytic activity of C1r and/or the processing of pro-C1r to an active protease. In some embodiments, the anti-C1r antibody inhibits the respective interactions at a stoichiometry of less than 2.5:1; 2.0:1; 1.5:1; or 1.0:1. In some embodiments, the anti-C1r antibody binds to C1r with a stoichiometry of less than 20:1; less than 19.5:1; less than19:1; less than 18.5:1; less than 18:1; less than 17.5:1; less than 17:1; less than 16.5:1; less than 16:1; less than 15.5:1; less than 15:1; less than 14.5:1; less than 14:1; less than 13.5:1; less than 13:1; less than 12.5:1; less than 12:1; less than 11.5:1; less than 11:1; less than 10.5:1; less than 10:1; less than 9.5:1; less than 9:1; less than 8.5:1; less than 8:1; less than 7.5:1; less than 7:1; less than 6.5:1; less than 6:1; less than 5.5:1; less than 5:1; less than 4.5:1; less than 4:1; less than 3.5:1; less than 3:1; less than 2.5:1; less than 2.0:1; less than 1.5:1; or less than 1.0:1. In certain embodiments, the anti-C1r antibody binds C1r with a binding stoichiometry that ranges from 20:1 to 1.0:1 or less than1.0:1. In certain embodiments, the anti-C1r antibody binds C1r with a binding stoichiometry that ranges from 6:1 to 1.0:1 or less than 1.0:1. In certain embodiments, the anti-C1r antibody binds C1r with a binding stoichiometry that ranges from 2.5:1 to 1.0:1 or less than 1.0:1. In some embodiments the anti-C1r antibody inhibits activation of C1r, C1s, and/or both C1r and C1s. Activation of the serine proteases C1r and C1s can be measured, without limitation, by standard colorimetric or fluorescent serine protease assays or by a standard complement-mediated cell lysis assays that are well known in the art.

In some embodiments, an antibody of the present disclosure is an anti-C1s antibody. In some embodiments, the anti-C1s antibody inhibits the interaction between C1s and C1q. In some embodiments, the anti-C1s antibody inhibits the interaction between C1s and C1r. In some embodiments the anti-C1s antibody inhibits the interaction between C1s and C1q and between C1s and C1r. In some embodiments, the anti-C1s antibody inhibits the catalytic activity of C1s or the processing of pro-C1s to an active protease. In some embodiments, the anti-C1s antibody inhibits the interaction between C1s and its substrates such as C2 and C4. In some embodiments, the anti-C1s antibody inhibits the respective interactions, at a stoichiometry of less than 2.5:1; 2.0:1; 1.5:1; or 1.0:1. In some embodiments, the anti-C1s antibody binds to C1s with a stoichiometry of less than 20:1; less than 19.5:1; less than19:1; less than 18.5:1; less than 18:1; less than 17.5:1; less than 17:1; less than 16.5:1; less than 16:1; less than 15.5:1; less than 15:1; less than 14.5:1; less than 14:1; less than 13.5:1; less than 13:1; less than 12.5:1; less than 12:1; less than 11.5:1; less than 11:1; less than 10.5:1; less than 10:1; less than 9.5:1; less than 9:1; less than 8.5:1; less than 8:1; less than 7.5:1; less than 7:1; less than 6.5:1; less than 6:1; less than 5.5:1; less than 5:1; less than 4.5:1; less than 4:1; less than 3.5:1; less than 3:1; less than 2.5:1; less than 2.0:1; less than 1.5:1; or less than 1.0:1 In certain embodiments, the anti-C1s antibody binds C1s with a binding stoichiometry that ranges from 20:1 to 1.0:1 or less than 1.0:1. In certain embodiments, the anti-C1s antibody binds C1s with a binding stoichiometry that ranges from 6:1 to 1.0:1 or less than1.0:1. In certain embodiments, the anti-C1s antibody binds C1s with a binding stoichiometry that ranges from 2.5:1 to 1.0:1 or less than1.0:1. In some embodiments the anti-C1s antibody inhibits activation of C1s, C1r, and/or both C1s and C1r.

Where antibodies of this disclosure inhibit the interaction between two or more complement factors, such as the interaction of C1q and C1s, or the interaction between factor C1q and an autoantibody, the interaction occurring in the presence of the antibody is reduced by at least 10%, at least 20%, at least 30%, at least 40%, at least 50%, at least 60%, at least 70%, at least 80%, at least 90%, at least 95%, at least 99%, or at least 99.9% compared to the interaction occurring in the absence of the antibody, but otherwise identical conditions. In certain embodiments, the interaction occurring in the presence of the antibody is reduced by an amount that ranges from at least 30% to at least 99.9%. Where antibodies of this disclosure inhibit activation of C1s and/or C1r, the serine protease activity of C1s and/or C1r in the presence of the antibody is reduced by at least 10%, at least 20%, at least 30%, at least 40%, at least 50%, at least 60%, at least 70%, at least 80%, at least 90%, at least 95%, at least 99%, or at least 99.9% compared to the serine protease activity in the absence of the antibody. In certain embodiments, the serine protease activity of C1s and/or C1r in the presence of the antibody in the presence of the antibody is reduced by an amount that ranges from at least 30% to at least 99.9%.

In some embodiments, the antibodies of this disclosure inhibit autoantibody-dependent and complement-dependent cytotoxicity (CDC) by at least 10%, at least 20%, at least 30%, at least 40%, at least 50%, at least 60%, at least 70%, at least 80%, at least 90%, at least 95%, at least 99%, or at least 99.9% relative to a control wherein the antibodies of the present disclosure were absent. In certain embodiments, antibodies of the present disclosure inhibit CDC by an amount that ranges from at least 30% to at least 99.9%. The *in vitro* EC₅₀ values for antibodies of this disclosure, *e.g*., with respect to inhibition of autoantibody-dependent and complement-dependent cytotoxicity (CDC), may be less than 3 µg/ml; less than 2.5 µg/ml; 2.0 µg/ml; less than 1.5 µg/ml; less than 1.0 µg/ml; less than 0.5 µg/ml; less than 0.25 µg/ml; less than 0.1 µg/ml; or less than 0.05 µg/ml. Preferably, *in vitro* EC₅₀ values are less than 1.0 µg/ml *(see also* Example 3). In certain embodiments, the *in vitro* EC₅₀ values for antibodies of this disclosure range from 3 µg/ml to 0.05 µg/ml or less than 0.05 µg/ml. The *in vitro* EC₅₀ values for antibodies of the present disclosure may be calculated by an *in vitro* assay known in the art for calculating *in vitro* EC₅₀ values. Non-limiting examples include the *in vitro* assays described in Examples 1, 3, and 4. In some embodiments, the *in vitro* EC₅₀ values for antibodies of the present disclosure may be calculated in the presence of human complement, for example present in human serum. In some embodiments, the amount of human complement, for example human serum, ranges from less than 1% to at least 20%. In certain embodiments, the amount of human complement, for example human serum, is less than 1%. In certain embodiments, the amount of human complement, for example human serum, is at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, or at least 20%.

In some embodiments, the antibodies of this disclosure inhibit complement-dependent cell-mediated cytotoxicity (CDCC) by at least 10%, at least 20%, at least 30%, at least 40%, at least 50%, at least 60%, at least 70%, at least 80%, at least 90%, at least 95%, at least 99%, or at least 99.9% relative to a control wherein the antibodies of the present disclosure were absent. In certain embodiments, antibodies of the present disclosure inhibit CDCC by an amount that ranges from at least 30% to at least 99.9%. Methods for measuring CDCC are well known in the art (*see also* Example 5 for possible methods). The *in vitro* EC₅₀ values for antibodies of this disclosure, for example, with respect CDCC inhibition may be less than 3 µg/ml; less than 2.5 µg/ml; 2.0 µg/ml; less than 1.5 µg/ml; less than 1.0 µg/ml; less than 0.5 µg/ml; less than 0.25 µg/ml; less than 0.1 µg/ml; or less than 0.05 µg/ml. Preferably, *in vitro* EC₅₀ values are less than 1.0µg/ml. In certain embodiments, the *in vitro* EC₅₀ values for antibodies of the present disclosure range from 3 µg/ml to 0.05 µg/ml or less than 0.05 µg/ml. In some embodiments, the *in vitro* EC₅₀ values for antibodies of the present disclosure may be calculated in the presence of human complement, for example present in human serum. In some embodiments, the amount of human complement, for example human serum, ranges from less than 1% to at least 20%. In certain embodiments, the amount of human complement, for example human serum, is less than 1%. In certain embodiments, the amount of human complement, for example human serum, is at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, or at least 20%. In preferred embodiments, the antibodies of the present disclosure inhibit CDCC but not antibody-dependent cellular cytotoxicity (ADCC; *see also* Example 5). In some embodiments, the antibodies of the present disclosure do not inhibit the lectin complement activation pathway.

As disclosed herein, the alternative pathway may amplify CDC initiated by C1 complex, *e.g*., by C1q binding to an autoantibody such as NMO-IgG. Accordingly, in some of the embodiments, the antibodies of the present disclosure may inhibit the alternative pathway by at least 10%, at least 20%, at least 30%, at least 40%, at least 50%, at least 60%, at least 70%, at least 80%, at least 90%, at least 95%, at least 99%, or at least 99.9% relative to a control wherein the antibodies of the present disclosure were absent (*see also* Example 5). In certain embodiments, antibodies of the present disclosure inhibit the alternative pathway by an amount that ranges from at least 30% to at least 99.9%.

Additional anti-C1q, anti-C1r, or anti-C1s antibodies, *e.g*., antibodies that specifically bind to a C1q, C1r, or C1s protein of the present disclosure, may be identified, screened, and/or characterized for their physical/chemical properties and/or biological activities by various assays known in the art.

In some embodiments, the present disclosure provides anti-C1q, anti-C1r, and/or anti-C1s antibodies. The antibodies of this disclosure may have one or more of the following characteristics. The antibodies of this disclosure may be polyclonal antibodies, monoclonal antibodies, human antibodies, humanized antibodies, chimeric antibodies, antibody fragments *(e.g.,* Fab, Fab'-SH, Fv, scFv, and (Fab')₂ fragments), bispecific and polyspecific antibodies, multivalent antibodies, and heteroconjugate antibodies. The antibodies of this disclosure may further contain engineered effector functions, amino acid sequence modifications or other antibody modifications known in the art; *e.g.,* the constant region of the anti-C1q, anti-C1r, and/or anti-C1s antibodies described herein may be modified to impair complement activation.

In certain embodiment, antibodies of the present disclosure are bispecific antibodies recognizing a first antigen and a second antigen. In some embodiments, the first antigen is a C1q antigen, a C1r antigen, and/or a C1s antigen. In some embodiments, the second antigen is an antigen facilitating transport across the blood-brain-barrier, including without limitation, transferrin receptor (TR), insulin receptor (HIR), insulin-like growth factor receptor (IGFR), low-density lipoprotein receptor related proteins 1 and 2 (LPR-1 and 2), diphtheria toxin receptor, CRM197, a llama single domain antibody, TMEM 30(A), a protein transduction domain, TAT, Syn-B, penetratin, a poly-arginine peptide, an angiopep peptide, and ANG1005.

In some embodiments, the antibodies of this disclosure prevent NMO, or one or more symptoms of NMO. In certain embodiments, prevention of NMO or one or more symptoms of NMO by the antibodies of the present disclosure is measure by prevention of lesion formation in an *ex vivo* spinal cord slice model of NMO, or by an *in vivo* mouse model of NMO. In some embodiments, the antibodies of this disclosure prevent lesion formation in an *ex vivo* spinal cord slice model of NMO or in an *in vivo* mouse model of NMO. Methods for measuring lesion formation *ex vivo* or *in vivo* are well known in the art *(see also* Examples 6 and 7 for exemplary methods). In certain embodiments, ex *vivo* lesion formation may be reduced at least by a relative score of 0.5, 1.0, 1.5, 2.0, 2.5, 3.0, 3.5, or 4.0 (*see also* **FIG. 6A-C** for an illustration of a relative scoring method). Preferably, *ex vivo* lesion formation is reduced by a relative score of at least 2.5. The *in vitro* EC₅₀ values for antibodies of the present disclosure, for example, with respect to the prevention *of ex vivo* lesion formation may be less than 3 µg/ml; less than 2.5 µg/ml; less than 2.0 µg/ml; less than 1.5 µg/ml; less than 1.0 µg/ml; less than 0.5 µg/ml; less than 0.25 µg/ml; less than 0.1 µg/ml; or less than 0.05 µg/ml. In certain embodiments, the *in vitro* EC₅₀ values for antibodies of the present disclosure range from 3 µg/ml to 0.05 µg/ml or less than 0.05 µg/ml. Preferably, *in vitro* EC₅₀ values are less than 1.0µM. In certain embodiments, the amount of human complement, for example human serum, is less than 1%. In certain embodiments, the amount of human complement, for example human serum, is at least 1%, at least 2%, at least 3%, at least 4% at least 5%, at least 6%, at least 7%, at least 8%, at least 9%, at least 10%, at least 11%, at least 12%, at least 13%, at least 14%, at least 15%, at least 16%, at least 17%, at least 18%, at least 19%, or at least 20%. *In vivo* lesion formation may be reduced by at least 5%, 10%, 15%, 20%, 35%, 40%, or 50% in terms of loss of staining (% of area). Staining may be assessed, without limitation, by AQP4 staining, GFAP staining, or MBP staining. *See also* **FIG. 7A-C** for an illustration of a quantification method. Preferably, *in vivo* lesion formation is reduced by at least 10%.

In certain embodiments, antibodies of the present disclosure inhibit CDC by an amount that ranges from at least 30% to at least 99.9%.

Additional anti-C1q, anti-C1r, and anti-C1s antibodies, *e.g*., antibodies that specifically bind to a C1 complex, a C1q protein, a C1r protein, or a C1s protein of the present disclosure, may be identified, screened, and/or characterized for their physical/chemical properties and/or biological activities by various assays known in the art.

### Antibody preparation

Anti-C1q, anti-C1r, and anti-C1s antibodies of the present disclosure can encompass polyclonal antibodies, monoclonal antibodies, human antibodies, humanized antibodies, chimeric antibodies, antibody fragments (*e.g*., Fab, Fab'-SH, Fv, scFv, and (Fab')₂ fragments), bispecific and polyspecific antibodies, multivalent antibodies, heteroconjugate antibodies, library derived antibodies, antibodies having modified effector functions, fusion proteins containing an antibody portion, and any other modified configuration of the immunoglobulin molecule that includes an antigen recognition site, such as an epitope having amino acid residues of a C1q, C1r, and/or C1s protein of the present disclosure, including glycosylation variants of antibodies, amino acid sequence variants of antibodies, and covalently modified antibodies. The anti-C1q, anti-C1r, and anti-C1s antibodies may be human, murine, rat, or of any other origin (including chimeric or humanized antibodies).

### (1) Polyclonal antibodies

Polyclonal antibodies, such as polyclonal anti-C1q, anti-C1r, and/or anti-C1s antibodies, are generally raised in animals by multiple subcutaneous (sc) or intraperitoneal (ip) injections of the relevant antigen and an adjuvant. It may be useful to conjugate the relevant antigen (*e.g*., a purified or recombinant C1q, C1r, or C1s protein of the present disclosure) to a protein that is immunogenic in the species to be immunized, *e.g*., keyhole limpet hemocyanin (KLH), serum albumin, bovine thyroglobulin, or soybean trypsin inhibitor, using a bifunctional or derivatizing agent, *e.g*., maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are independently lower alkyl groups. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

The animals are immunized against the desired antigen, immunogenic conjugates, or derivatives by combining, *e.g*., 100 µg (for rabbits) or 5 µg (for mice) of the protein or conjugate with 3 volumes of Freund's complete adjuvant and injecting the solution intradermally at multiple sites. One month later, the animals are boosted with 1/5 to 1/10 the original amount of peptide or conjugate in Freund's complete adjuvant by subcutaneous injection at multiple sites. Seven to fourteen days later, the animals are bled and the serum is assayed for antibody titer. Animals are boosted until the titer plateaus. Conjugates also can be made in recombinant-cell culture as protein fusions. Also, aggregating agents such as alum are suitable to enhance the immune response.

### (2) Monoclonal antibodies

Monoclonal antibodies, such as monoclonal anti-C1q, anti-C1r, and/or anti-C1s antibodies, are obtained from a population of substantially homogeneous antibodies, *i.e.,* the individual antibodies comprising the population are identical except for possible naturally occurring mutations and/or post-translational modifications (*e.g*., isomerizations, amidations) that may be present in minor amounts. Thus, the modifier "monoclonal" indicates the character of the antibody as not being a mixture of discrete antibodies.

For example, the monoclonal anti-C1q, anti-C1r, and/or anti-C1s antibodies may be made using the hybridoma method first described by Kohler et al., Nature, 256:495 (1975), or may be made by recombinant DNA methods (U.S. Patent No. 4,816,567).

In the hybridoma method, a mouse or other appropriate host animal, such as a hamster, is immunized as hereinabove described to elicit lymphocytes that produce or are capable of producing antibodies that will specifically bind to the protein used for immunization (*e.g.,* a purified or recombinant C1q, C1r, C1s protein of the present disclosure). Alternatively, lymphocytes may be immunized *in vitro.* Lymphocytes then are fused with myeloma cells using a suitable fusing agent, such as polyethylene glycol, to form a hybridoma cell (Goding, Monoclonal Antibodies: Principles and Practice, pp.59-103 (Academic Press, 1986)).

The immunizing agent will typically include the antigenic protein *(e.g.,* a purified or recombinant C1q, C1r, or C1s protein of the present disclosure) or a fusion variant thereof. Generally peripheral blood lymphocytes ("PBLs") are used if cells of human origin are desired, while spleen or lymph node cells are used if non-human mammalian sources are desired. The lymphoctyes are then fused with an immortalized cell line using a suitable fusing agent, such as polyethylene glycol, to form a hybridoma cell. Goding, Monoclonal Antibodies: Principles and Practice, Academic Press (1986), pp. 59-103.

Immortalized cell lines are usually transformed mammalian cells, particularly myeloma cells of rodent, bovine or human origin. Usually, rat or mouse myeloma cell lines are employed. The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which are substances that prevent the growth of HGPRT-deficient-cells.

Preferred immortalized myeloma cells are those that fuse efficiently, support stable high-level production of antibody by the selected antibody-producing cells, and are sensitive to a medium such as HAT medium. Among these, preferred are murine myeloma lines, such as those derived from MOPC-21 and MPC-11 mouse tumors (available from the Salk Institute Cell Distribution Center, San Diego, California USA), as well as SP-2 cells and derivatives thereof (*e.g*., X63-Ag8-653) (available from the American Type Culture Collection, Manassas, Virginia USA). Human myeloma and mouse-human heteromyeloma cell lines have also been described for the production of human monoclonal antibodies (Kozbor, J. Immunol., 133:3001 (1984); Brodeur et al., Monoclonal Antibody Production Techniques and Applications, pp. 51-63 (Marcel Dekker, Inc., New York, 1987)).

Culture medium in which hybridoma cells are growing is assayed for production of monoclonal antibodies directed against the antigen (*e.g*., a C1q, C1r, or C1s protein of the present disclosure). Preferably, the binding specificity of monoclonal antibodies produced by hybridoma cells is determined by immunoprecipitation or by an *in vitro* binding assay, such as radioimmunoassay (RIA) or enzyme-linked immunosorbent assay (ELISA).

The culture medium in which the hybridoma cells are cultured can be assayed for the presence of monoclonal antibodies directed against the desired antigen (*e.g*., a C1q, C1r, or C1s protein of the present disclosure). Preferably, the binding affinity and specificity of the monoclonal antibody can be determined by immunoprecipitation or by an *in vitro* binding assay, such as radioimmunoassay (RIA) or enzyme-linked assay (ELISA). Such techniques and assays are known in the in art. For example, binding affinity may be determined by the Scatchard analysis of Munson et al., Anal. Biochem., 107:220 (1980).

After hybridoma cells are identified that produce antibodies of the desired specificity, affinity, and/or activity, the clones may be subcloned by limiting dilution procedures and grown by standard methods (Goding, *supra*)*.* Suitable culture media for this purpose include, for example, D-MEM or RPMI-1640 medium. In addition, the hybridoma cells may be grown *in vivo* as tumors in a mammal.

The monoclonal antibodies secreted by the subclones are suitably separated from the culture medium, ascites fluid, or serum by conventional immunoglobulin purification procedures such as, for example, protein A-Sepharose chromatography, hydroxylapatite chromatography, gel electrophoresis, dialysis, affinity chromatography, and other methods as described above.

Anti-C1q, anti-C1r, and/or anti-C1s monoclonal antibodies may also be made by recombinant DNA methods, such as those disclosed in U.S. Patent No. 4,816,567, and as described above. DNA encoding the monoclonal antibodies is readily isolated and sequenced using conventional procedures (e.g., by using oligonucleotide probes that specifically bind to genes encoding the heavy and light chains of murine antibodies). The hybridoma cells serve as a preferred source of such DNA. Once isolated, the DNA may be placed into expression vectors, which are then transfected into host-cells such as *E. coli* cells, simian COS cells, Chinese hamster ovary (CHO) cells, or myeloma cells that do not otherwise produce immunoglobulin protein, in order to synthesize monoclonal antibodies in such recombinant host-cells. Review articles on recombinant expression in bacteria of DNA encoding the antibody include Skerra et al., Curr. Opin. Immunol., 5:256-262 (1993) and Plückthun, Immunol. Rev. 130:151-188 (1992).

In certain embodiments, anti-C1q, anti-C1r, and/or anti-C1s antibodies can be isolated from antibody phage libraries generated using the techniques described in McCafferty et al., Nature, 348:552-554 (1990). Clackson et al., Nature, 352:624-628 (1991) and Marks et al., J. Mol. Biol., 222:581-597 (1991) described the isolation of murine and human antibodies, respectively, from phage libraries. Subsequent publications describe the production of high affinity (nanomolar ("nM") range) human antibodies by chain shuffling (Marks et al., Bio/Technology, 10:779-783 (1992)), as well as combinatorial infection and *in vivo* recombination as a strategy for constructing very large phage libraries (Waterhouse et al., Nucl. Acids Res., 21:2265-2266 (1993)). Thus, these techniques are viable alternatives to traditional monoclonal antibody hybridoma techniques for isolation of monoclonal antibodies of desired specificity (e.g., those that bind a C1q, C1r, or C1s protein of the present disclosure).

The DNA encoding antibodies or fragments thereof may also be modified, for example, by substituting the coding sequence for human heavy- and light-chain constant domains in place of the homologous murine sequences (U.S. Patent No. 4,816,567; Morrison, et al., Proc. Natl Acad. Sci. USA, 81:6851 (1984)), or by covalently joining to the immunoglobulin coding sequence all or part of the coding sequence for a non-immunoglobulin polypeptide. Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

The monoclonal antibodies described herein (*e.g*., anti-C1q, anti-C1r, and/or anti-C1s antibodies of the present disclosure or fragments thereof) may by monovalent, the preparation of which is well known in the art. For example, one method involves recombinant expression of immunoglobulin light chain and a modified heavy chain. The heavy chain is truncated generally at any point in the Fc region so as to prevent heavy chain crosslinking. Alternatively, the relevant cysteine residues may be substituted with another amino acid residue or are deleted so as to prevent crosslinking. *In vitro* methods are also suitable for preparing monovalent antibodies. Digestion of antibodies to produce fragments thereof, particularly Fab fragments, can be accomplished using routine techniques known in the art.

Chimeric or hybrid anti-C1q, anti-C1r, and/or anti-C1s antibodies also may be prepared *in vitro* using known methods in synthetic protein chemistry, including those involving crosslinking agents. For example, immunotoxins may be constructed using a disulfide-exchange reaction or by forming a thioether bond. Examples of suitable reagents for this purpose include iminothiolate and methyl-4-mercaptobutyrimidate.

### (3) Humanized antibodies

Anti-C1q, anti-C1r, and/or anti-C1s antibodies of the present disclosure or antibody fragments thereof may further include humanized or human antibodies. Humanized forms of non-human (*e.g*., murine) antibodies are chimeric immunoglobulins, immunoglobulin chains or fragments thereof (such as Fab, Fab'-SH, Fv, scFv, F(ab')₂ or other antigen-binding subsequences of antibodies) which contain minimal sequence derived from non-human immunoglobulin. Humanized antibodies include human immunoglobulins (recipient antibody) in which residues from a complementarity determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally will also comprise at least a portion of an immunoglobulin constant region (Fc), typically that of a human immunoglobulin. Jones et al., Nature 321: 522-525 (1986); Riechmann et al., Nature 332: 323-329 (1988) and Presta, Curr. Opin. Struct. Biol. 2: 593-596 (1992).

Methods for humanizing non-human anti-C1q, anti-C1r, and/or anti-C1s antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and coworkers, Jones et al., Nature 321:522-525 (1986); Riechmann et al., Nature 332:323-327 (1988); Verhoeyen et al., Science 239:1534-1536 (1988), or through substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework (FR) for the humanized antibody. Sims et al., J. Immunol., 151:2296 (1993); Chothia et al., J. Mol. Biol., 196:901 (1987). Another method uses a particular framework derived from the consensus sequence of all human antibodies of a particular subgroup of light or heavy chains. The same framework may be used for several different humanized antibodies. Carter et al., Proc. Nat'l Acad. Sci. USA 89:4285 (1992); Presta et al., J. Immunol. 151:2623 (1993).

Furthermore, it is important that antibodies be humanized with retention of high affinity for the antigen and other favorable biological properties. To achieve this goal, according to a preferred method, humanized antibodies are prepared by a process of analyzing the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, *i.e.,* the analysis of residues that influence the ability of the candidate immunoglobulin to bind its antigen. In this way, FR residues can be selected and combined from the recipient and import sequences so that the desired antibody characteristic, such as increased affinity for the target antigen or antigens (*e.g.,* C1q, C1r, C1s proteins of the present disclosure), is achieved. In general, the CDR residues are directly and most substantially involved in influencing antigen binding.

Various forms of the humanized anti-C1q, anti-C1r, and/or anti-C1s antibody are contemplated. For example, the humanized anti-C1q, anti-C1r, and/or anti-C1s antibody may be an antibody fragment, such as an Fab, which is optionally conjugated with one or more cytotoxic agent(s) in order to generate an immunoconjugate. Alternatively, the humanized anti-C1q, anti-C1r, or anti-C1s antibody may be an intact antibody, such as an intact IgG1 antibody.

### (4) Human antibodies

Alternatively, human anti-C1q, anti-C1r, and/or anti-C1s antibodies can be generated. For example, it is now possible to produce transgenic animals (*e.g*., mice) that are capable, upon immunization, of producing a full repertoire of human antibodies in the absence of endogenous immunoglobulin production. The homozygous deletion of the antibody heavy-chain joining region (J_{H}) gene in chimeric and germ-line mutant mice results in complete inhibition of endogenous antibody production. Transfer of the human germ-line immunoglobulin gene array in such germ-line mutant mice will result in the production of human antibodies upon antigen challenge. *See, e.g.,* Jakobovits et al., Proc. Nat'l Acad. Sci. USA, 90:2551 (1993); Jakobovits et al., Nature, 362:255-258 (1993); Bruggermann et al., Year in Immunol., 7:33 (1993); U.S. Patent Nos. 5,591,669 and WO 97/17852.

Alternatively, phage display technology can be used to produce human anti-C1q, anti-C1r, and/or anti-C1s antibodies and antibody fragments *in vitro,* from immunoglobulin variable (V) domain gene repertoires from unimmunized donors. McCafferty et al., Nature 348:552-553 (1990); Hoogenboom and Winter, J. Mol. Biol. 227: 381 (1991). According to this technique, antibody V domain genes are cloned in-frame into either a major or minor coat protein gene of a filamentous bacteriophage, such as M13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B-cell. Phage display can be performed in a variety of formats, reviewed in, *e.g.,* Johnson, Kevin S. and Chiswell, David J., Curr. Opin Struct. Biol. 3:564-571 (1993). Several sources of V-gene segments can be used for phage display. Clackson et al., Nature 352:624-628 (1991) isolated a diverse array of anti-oxazolone antibodies from a small random combinatorial library of V genes derived from the spleens of immunized mice. A repertoire of V genes from unimmunized human donors can be constructed and antibodies to a diverse array of antigens (including self-antigens) can be isolated essentially following the techniques described by Marks et al., J. Mol. Biol. 222:581-597 (1991), or Griffith et al., EMBO J. 12:725-734 (1993). *See also* U.S. Patent. Nos. 5,565,332 and 5,573,905. Additionally, yeast display technology can be used to produce human anti-C1q, anti-C1r, or anti-C1s antibodies and antibody fragments *in vitro (e.g.,* WO 2009/036379; WO 2010/105256; WO 2012/009568; US 2009/0181855; US 2010/0056386; and Feldhaus and Siegel (2004) J. Immunological Methods 290:69-80). In other embodiments, ribosome display technology can be used to produce human anti-C1q, anti-C1r, or anti-C1s antibodies and antibody fragments *in vitro* (*e.g.,* Roberts and Szostak (1997) Proc Natl Acad Sci 94:12297-12302; Schaffitzel et al. (1999) J. Immunolical Methods 231:119-135; Lipovsek and Plückthun (2004) J. Immunological Methods 290:51-67).

The techniques of Cole et al., and Boerner et al., are also available for the preparation of human anti-C1q, anti-C1r, and/or anti-C1s monoclonal antibodies (Cole et al., Monoclonal Antibodies and Cancer Therapy, Alan R. Liss, p. 77 (1985) and Boerner et al., J. Immunol. 147(1): 86-95 (1991). Similarly, human anti-C1q, anti-C1r, and/or anti-C1s antibodies can be made by introducing human immunoglobulin loci into transgenic animals, *e.g*., mice in which the endogenous immunoglobulin genes have been partially or completely inactivated. Upon challenge, human antibody production is observed, which closely resembles that seen in humans in all respects, including gene rearrangement, assembly and antibody repertoire. This approach is described, for example, in U.S. Patent Nos. 5,545,807; 5,545,806, 5,569,825, 5,625,126, 5,633,425, 5,661,016 and in the following scientific publications: Marks et al., Bio/Technology 10: 779-783 (1992); Lonberg et al., Nature 368: 856-859 (1994); Morrison, Nature 368: 812-13 (1994), Fishwild et al., Nature Biotechnology 14: 845-51 (1996), Neuberger, Nature Biotechnology 14: 826 (1996) and Lonberg and Huszar, Intern. Rev. Immunol. 13: 65-93 (1995).

Finally, human anti-C1q, anti-C1r, and/or anti-C1s antibodies may also be generated in vitro by activated B-cells (see U.S. Patent Nos 5,567,610 and 5,229,275).

### (5) Antibody fragments

In certain embodiments there are advantages to using anti-C1q, anti-C1r, and/or anti-C1s antibody fragments, rather than whole anti-C1q, anti-C1r, and/or anti-C1s antibodies. Smaller fragment sizes allow for rapid clearance.

Various techniques have been developed for the production of antibody fragments. Traditionally, these fragments were derived via proteolytic digestion of intact antibodies *(see, e.g.,* Morimoto et al., J. Biochem. Biophys. Method. 24:107-117 (1992); and Brennan et al., Science 229:81 (1985)). However, these fragments can now be produced directly by recombinant host-cells, for example, using nucleic acids encoding anti-C1q, anti-C1r, or anti-C1s antibodies of the present disclosure. Fab, Fv and scFv antibody fragments can all be expressed in and secreted from *E. coli,* thus allowing the straightforward production of large amounts of these fragments. A anti-C1q, anti-C1r, or anti-C1s antibody fragments can also be isolated from the antibody phage libraries as discussed above. Alternatively, Fab'-SH fragments can be directly recovered from *E. coli* and chemically coupled to form F(ab')₂ fragments (Carter et al., Bio/Technology 10:163-167 (1992)). According to another approach, F(ab')₂ fragments can be isolated directly from recombinant host-cell culture. Production of Fab and F(ab')₂ antibody fragments with increased *in vivo* half-lives are described in U.S. Patent No. 5,869,046. In other embodiments, the antibody of choice is a single chain Fv fragment (scFv). See WO 93/16185; U.S. Patent No. 5,571,894 and U.S. Patent No. 5,587,458. The anti-C1q, anti-C1r, or anti-C1s antibody fragment may also be a "linear antibody," *e.g*., as described in U.S. Patent 5,641,870. Such linear antibody fragments may be monospecific or bispecific.

### (6) Bispecific and polyspecific antibodies

Bispecific antibodies (BsAbs) are antibodies that have binding specificities for at least two different epitopes, including those on the same or another protein (e.g., one or more C1q, C1r, or C1s proteins of the present disclosure). Alternatively, one part of a BsAb can be armed to bind to the target C1q, C1r, or C1s antigen, and another can be combined with an arm that binds to a second protein. Such antibodies can be derived from full length antibodies or antibody fragments (*e.g*., F(ab')₂ bispecific antibodies).

Methods for making bispecific antibodies are known in the art. Traditional production of full length bispecific antibodies is based on the coexpression of two immunoglobulin heavy-chain/light chain pairs, where the two chains have different specificities. Millstein et al., Nature, 305:537-539 (1983). Because of the random assortment of immunoglobulin heavy and light chains, these hybridomas (quadromas) produce a potential mixture of 10 different antibody molecules, of which only one has the correct bispecific structure. Purification of the correct molecule, which is usually done by affinity chromatography steps, is rather cumbersome, and the product yields are low. Similar procedures are disclosed in WO 93/08829 and in Traunecker et al., EMBO J., 10:3655-3659 (1991).

According to a different approach, antibody variable domains with the desired binding specificities (antibody-antigen combining sites) are fused to immunoglobulin constant domain sequences. The fusion preferably is with an immunoglobulin heavy chain constant domain, comprising at least part of the hinge, C_{H}2, and C_{H}3 regions. It is preferred to have the first heavy-chain constant region (C_{H}1) containing the site necessary for light chain binding, present in at least one of the fusions. DNAs encoding the immunoglobulin heavy chain fusions and, if desired, the immunoglobulin light chain, are inserted into separate expression vectors, and are co-transfected into a suitable host organism. This provides for great flexibility in adjusting the mutual proportions of the three polypeptide fragments in embodiments when unequal ratios of the three polypeptide chains used in the construction provide the optimum yields. It is, however, possible to insert the coding sequences for two or all three polypeptide chains in one expression vector when the expression of at least two polypeptide chains in equal ratios results in high yields or when the ratios are of no particular significance.

In a preferred embodiment of this approach, the bispecific antibodies are composed of a hybrid immunoglobulin heavy chain with a first binding specificity in one arm, and a hybrid immunoglobulin heavy chain-light chain pair (providing a second binding specificity) in the other arm. It was found that this asymmetric structure facilitates the separation of the desired bispecific compound from unwanted immunoglobulin chain combinations, as the presence of an immunoglobulin light chain in only half of the bispecific molecules provides for an easy way of separation. This approach is disclosed in WO 94/04690. For further details of generating bispecific antibodies, see, for example, Suresh et al., Methods in Enzymology 121: 210 (1986).

According to another approach described in WO 96/27011 or U.S. Patent No. 5,731,168, the interface between a pair of antibody molecules can be engineered to maximize the percentage of heterodimers which are recovered from recombinant-cell culture. The preferred interface comprises at least a part of the C_{H}3 region of an antibody constant domain. In this method, one or more small amino acid side chains from the interface of the first antibody molecule are replaced with larger side chains (e.g., tyrosine or tryptophan). Compensatory "cavities" of identical or similar size to the large side chains(s) are created on the interface of the second antibody molecule by replacing large amino acid side chains with smaller ones (*e.g*., alanine or threonine). This provides a mechanism for increasing the yield of the heterodimer over other unwanted end-products such as homodimers.

Techniques for generating bispecific antibodies from antibody fragments have been described in the literature. For example, bispecific antibodies can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175: 217-225 (1992) describes the production of fully humanized bispecific antibody F(ab')₂ molecules. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T-cells, as well as trigger the lytic activity of human cytotoxic lymphocytes against human breast tumor targets.

Various techniques for making and isolating bivalent antibody fragments directly from recombinant-cell culture have also been described. For example, bivalent heterodimers have been produced using leucine zippers. Kostelny et al., J. Immunol., 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. The "diabody" technology described by Hollinger et al., Proc. Nat'l Acad. Sci. USA, 90: 6444-6448 (1993) has provided an alternative mechanism for making bispecific/bivalent antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific/bivalent antibody fragments by the use of single-chain Fv (sFv) dimers has also been reported. *See* Gruber et al., J. Immunol., 152:5368 (1994).

Antibodies with more than two valencies are also contemplated. For example, trispecific antibodies can be prepared. Tutt et al., *J*. *Immunol.* 147:60 (1991).

Exemplary bispecific antibodies may bind to two different antigens. In some embodiments a bispecific antibody binds to a first antigen C1q, C1r, or C1s and a second antigen facilitating transport across the blood-brain barrier. Numerous antigens are known in the art that facilitate transport across the blood-brain barrier *(see, e.g.,* Gabathuler R., Approaches to transport therapeutic drugs across the blood-brain barrier to treat brain diseases, Neurobiol. Dis. 37 (2010) 48-57). Such second antigens include, without limitation, transferrin receptor (TR), insulin receptor (HIR), Insulin-like growth factor receptor (IGFR), low-density lipoprotein receptor related proteins 1 and 2 (LPR-1 and 2), diphtheria toxin receptor, including CRM197 (a non-toxic mutant of diphtheria toxin), llama single domain antibodies such as TMEM 30(A) (Flippase), protein transduction domains such as TAT, Syn-B, or penetratin, poly-arginine or generally positively charged peptides, and Angiopep peptides such as ANG1005 (*see, e.g.,* Gabathuler, 2010).

### (7) Multivalent antibodies

A multivalent antibody may be internalized (and/or catabolized) faster than a bivalent antibody by a cell expressing an antigen to which the antibodies bind. The anti-C1q, anti-C1r, and/or anti-C1s antibodies of the present disclosure or antibody fragments thereof can be multivalent antibodies (which are other than of the IgM class) with three or more antigen binding sites (e.g., tetravalent antibodies), which can be readily produced by recombinant expression of nucleic acid encoding the polypeptide chains of the antibody. The multivalent antibody can comprise a dimerization domain and three or more antigen binding sites. The preferred dimerization domain comprises an Fc region or a hinge region. In this scenario, the antibody will comprise an Fc region and three or more antigen binding sites amino-terminal to the Fc region. The preferred multivalent antibody herein contains three to about eight, but preferably four, antigen binding sites. The multivalent antibody contains at least one polypeptide chain (and preferably two polypeptide chains), wherein the polypeptide chain or chains comprise two or more variable domains. For instance, the polypeptide chain or chains may comprise VD1-(X1)n-VD2-(X2)n-Fc, wherein VD1 is a first variable domain, VD2 is a second variable domain, Fc is one polypeptide chain of an Fc region, X1 and X2 represent an amino acid or polypeptide, and n is 0 or 1. Similarly, the polypeptide chain or chains may comprise V_{H}-C_{H}1-flexible linker-V_{H}-C_{H}1-Fc region chain; or V_{H}-C_{H}1-V_{H}-C_{H}1-Fc region chain. The multivalent antibody herein preferably further comprises at least two (and preferably four) light chain variable domain polypeptides. The multivalent antibody herein may, for instance, comprise from about two to about eight light chain variable domain polypeptides. The light chain variable domain polypeptides contemplated here comprise a light chain variable domain and, optionally, further comprise a CL domain.

### (8) Heteroconjugate antibodies

Heteroconjugate antibodies are also within the scope of the present disclosure. Heteroconjugate antibodies are composed of two covalently joined antibodies (*e.g*., anti-Clq, anti-C1r, and/or anti-C1s antibodies of the present disclosure or antibody fragments thereof). For example, one of the antibodies in the heteroconjugate can be coupled to avidin, the other to biotin. Such antibodies have, for example, been proposed to target immune system cells to unwanted cells, U.S. Patent No. 4,676,980, and have been used to treat HIV infection. International Publication Nos. WO 91/00360, WO 92/200373 and EP 0308936. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980. Heteroconjugate antibodies may be made using any convenient cross-linking methods. Suitable cross-linking agents are well known in the art, and are disclosed in U.S. Patent No. 4,676,980, along with a number of cross-linking techniques.

### (9) Effector function engineering

It may also be desirable to modify an anti-Clq, anti-Clr, and/or anti-C1s antibody of the present disclosure to modify effector function and/or to increase serum half-life of the antibody. For example, the Fc receptor binding site on the constant region may be modified or mutated to remove or reduce binding affinity to certain Fc receptors, such as FcyRI, FcyRII, and/or FcyRIII. In some embodiments, the effector function is impaired by removing N-glycosylation of the Fc region *(e.g.,* in the CH 2 domain of IgG) of the antibody. In some embodiments, the effector function is impaired by modifying regions such as 233-236, 297, and/or 327-331 of human IgG as described in PCT WO 99/58572 and Armour et al., Molecular Immunology 40: 585-593 (2003); Reddy et al., J. Immunology 164:1925-1933 (2000).

The constant region of the anti-complement antibodies described herein may also be modified to impair complement activation. For example, complement activation of IgG antibodies following binding of the C1 component of complement may be reduced by mutating amino acid residues in the constant region in a C1 binding motif (*e.g*., C1q binding motif). It has been reported that Ala mutation for each of D270, K322, P329, P331 of human IgG1 significantly reduced the ability of the antibody to bind to C1q and activating complement. For murine IgG2b, C1q binding motif constitutes residues E318, K320, and K322. Idusogie et al. (2000) J. Immunology 164:4178-4184; Duncan et al. (1988) Nature 322: 738-740. As the C1q binding motif E318, K320, and K322 identified for murine IgG2b is believed to be common for other antibody isotypes (Duncan et al. (1988) Nature 322:738-740), C1q binding activity for IgG2b can be abolished by replacing any one of the three specified residues with a residue having an inappropriate functionality on its side chain. It is not necessary to replace the ionic residues only with Ala to abolish C1q binding. It is also possible to use other alkyl-substituted non-ionic residues, such as Gly, Ile, Leu, or Val, or such aromatic non-polar residues as Phe, Tyr, Trp and Pro in place of any one of the three residues in order to abolish C1q binding. In addition, it is also possible to use such polar non-ionic residues as Ser, Thr, Cys, and Met in place of residues 320 and 322, but not 318, in order to abolish C1q binding activity. In addition, removal of carbohydrate modifications of the Fc region necessary for complement binding can prevent complement activation Glycosylation of a conserved asparagine (Asn-297) on the CH2 domain of IgG heavy chains is essential for antibody effector functions (Jefferis et al. (1998) Immunol Rev 163:59-76). Modification of the Fc glycan alters IgG conformation and reduces the Fc affinity for binding of complement protein C1q and effector cell receptor FcR (Alhorn et al. (2008) PLos ONE 2008;3:e1413). Complete removal of the Fc glycan abolishes CDC and ADCC. Deglycosylation can be performed using glycosidase enzymes for example Endoglycosidase S (EndoS), a 108kDa enzyme encoded by the gene endoS of Streptococcus pyogenes that selectively digests asparagine-linked glycans on the heavy chain of all IgG subclasses, without action on other immunoglobulin classes or other glycoproteins (Collin et al. (2001) EMBO J 2001;20:3046-3055).

To increase the serum half-life of the antibody, one may incorporate a salvage receptor binding epitope into the antibody (especially an antibody fragment) as described in U.S. Patent 5,739,277, for example. As used herein, the term *"salvage receptor binding epitope"* refers to an epitope of the Fc region of an IgG molecule (*e.g*., IgG₁, IgG₂, IgG₃, or IgG₄) that is responsible for increasing the *in vivo* serum half-life of the IgG molecule.

### (10) Other amino acid sequence modifications

Amino acid sequence modifications of anti-C1q, anti-C1r, and/or anti-C1s antibodies of the present disclosure, or antibody fragments thereof, are also contemplated. For example, it may be desirable to improve the binding affinity and/or other biological properties of the antibodies or antibody fragments. Amino acid sequence variants of the antibodies or antibody fragments are prepared by introducing appropriate nucleotide changes into the nucleic acid encoding the antibodies or antibody fragments, or by peptide synthesis. Such modifications include, for example, deletions from, and/or insertions into and/or substitutions of, residues within the amino acid sequences of the antibody. Any combination of deletion, insertion, and substitution is made to arrive at the final construct, provided that the final construct possesses the desired characteristics (*i.e*., the ability to bind or physically interact with a C1q, C1r, or C1s protein of the present disclosure). The amino acid changes also may alter post-translational processes of the antibody, such as changing the number or position of glycosylation sites.

A useful method for identification of certain residues or regions of the anti-C1q, anti-C1r, and/or anti-C1s antibody that are preferred locations for mutagenesis is called "alanine scanning mutagenesis" as described by Cunningham and Wells in Science, 244:1081-1085 (1989). Here, a residue or group of target residues are identified (*e.g*., charged residues such as arg, asp, his, lys, and glu) and replaced by a neutral or negatively charged amino acid (most preferably alanine or polyalanine) to affect the interaction of the amino acids with the target antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, alanine scanning or random mutagenesis is conducted at the target codon or region and the expressed antibody variants are screened for the desired activity.

Amino acid sequence insertions include amino- ("N") and/or carboxy- ("C") terminal fusions ranging in length from one residue to polypeptides containing a hundred or more residues, as well as intrasequence insertions of single or multiple amino acid residues. Examples of terminal insertions include an antibody with an N-terminal methionyl residue or the antibody fused to a cytotoxic polypeptide. Other insertional variants of the antibody molecule include the fusion to the N- or C-terminus of the antibody to an enzyme or a polypeptide which increases the serum half-life of the antibody.

Another type of variant is an amino acid substitution variant. These variants have at least one amino acid residue in the antibody molecule replaced by a different residue. The sites of greatest interest for substitutional mutagenesis include the hypervariable regions, but FR alterations are also contemplated. Conservative substitutions are shown in the Table A below under the heading of "preferred substitutions". If such substitutions result in a change in biological activity, then more substantial changes, denominated "exemplary substitutions" in Table A, or as further described below in reference to amino acid classes, may be introduced and the products screened.

**TABLE A: Amino Acid Substitutions**

| **Original Residue** | **Exemplary Substitutions** | **Preferred Substitutions** |
|---|---|---|
| Ala (A) | val; leu; ile | val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | Ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions entail exchanging a member of one of these classes for another class.

Any cysteine residue not involved in maintaining the proper conformation of the antibody also may be substituted, generally with serine, to improve the oxidative stability of the molecule and prevent aberrant crosslinking. Conversely, cysteine bond(s) may be added to the antibody to improve its stability (particularly where the antibody is an antibody fragment, such as an Fv fragment).

A particularly preferred type of substitutional variant involves substituting one or more hypervariable region residues of a parent antibody (*e.g*. a humanized or human anti-C1q, anti-C1r, and/or anti-C1s antibody). Generally, the resulting variant(s) selected for further development will have improved biological properties relative to the parent antibody from which they are generated. A convenient way for generating such substitutional variants involves affinity maturation using phage display. Briefly, several hypervariable region sites (*e.g*., 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g*., binding affinity) as herein disclosed. In order to identify candidate hypervariable region sites for modification, alanine scanning mutagenesis can be performed to identify hypervariable region residues contributing significantly to antigen binding. Alternatively, or additionally, it may be beneficial to analyze a crystal structure of the antigen-antibody complex to identify contact points between the antibody and the antigen (*e.g*., a C1q, C1r, or C1s protein of the present disclosure). Such contact residues and neighboring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antibody alters the original glycosylation pattern of the antibody. By altering is meant deleting one or more carbohydrate moieties found in the antibody, and/or adding one or more glycosylation sites that are not present in the antibody.

Glycosylation of antibodies is typically either N-linked or O-linked. N-linked refers to the attachment of the carbohydrate moiety to the side chain of an asparagine residue. The tripeptide sequences asparagine-X-serine and asparagine-X-threonine, where X is any amino acid except proline, are the recognition sequences for enzymatic attachment of the carbohydrate moiety to the asparagine side chain. Thus, the presence of either of these tripeptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antibody is conveniently accomplished by altering the amino acid sequence such that it contains one or more of the above-described tripeptide sequences (for N-linked glycosylation sites). The alteration may also be made by the addition of, or substitution by, one or more serine or threonine residues to the sequence of the original antibody (for O-linked glycosylation sites).

Nucleic acid molecules encoding amino acid sequence variants of the anti-IgE antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibodies (*e.g*., anti-Clq, anti-Clr, and/or anti-C1s antibody of the present disclosure) or antibody fragments.

### (11) Other antibody modifications

Anti-Clq, anti-Clr, and/or anti-C1s antibodies of the present disclosure, or antibody fragments thereof, can be further modified to contain additional non-proteinaceous moieties that are known in the art and readily available. Preferably, the moieties suitable for derivatization of the antibody are water-soluble polymers. Non-limiting examples of water-soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, polypropylene glycol homopolymers, polypropylene oxide/ethylene oxide co-polymers, polyoxyethylated polyols (*e.g*., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, *etc.* Such techniques and other suitable formulations are disclosed in Remington: The Science and Practice of Pharmacy, 20th Ed., Alfonso Gennaro, Ed., Philadelphia College of Pharmacy and Science (2000).

### Nucleic acids, vectors, and host cells

Anti-C1q, anti-C1r, and/or anti-C1s antibodies of the present disclosure may be produced using recombinant methods and compositions, *e.g*., as described in U.S. Patent No. 4,816,567. In some aspects, isolated nucleic acids having a nucleotide sequence encoding any of the anti-C1q, anti-C1r, or anti-C1s antibodies of the present disclosure are described. Such nucleic acids may encode an amino acid sequence containing the VL and/or an amino acid sequence containing the VH of the anti-C1q, anti-C1r, or anti-C1s antibody (*e.g*., the light and/or heavy chains of the antibody). In some aspects, one or more vectors (*e.g*., expression vectors) containing such nucleic acids are described. In some aspects, a host cell containing such nucleic acid is also described. In some aspects, the host cell contains (*e.g*., has been transduced with): (1) a vector containing a nucleic acid that encodes an amino acid sequence containing the VL of the antibody and an amino acid sequence containing the VH of the antibody, or (2) a first vector containing a nucleic acid that encodes an amino acid sequence containing the VL of the antibody and a second vector containing a nucleic acid that encodes an amino acid sequence containing the VH of the antibody. In some aspects, the host cell is eukaryotic, *e.g*., a Chinese Hamster Ovary (CHO) cell or lymphoid cell (*e.g*., Y0, NS0, Sp20 cell).

Methods of making an anti-Clq, anti-C1r, and/or anti-C1s antibody of the present disclosure are also described. In some aspects, the method includes culturing a host cell of the present disclosure containing a nucleic acid encoding the anti-C1q, anti-C1r, and/or anti-C1s antibody, under conditions suitable for expression of the antibody. In some aspects, the antibody is subsequently recovered from the host cell (or host cell culture medium).

For recombinant production of an anti-C1q, anti-C1r, and/or anti-C1s antibody of the present disclosure, a nucleic acid encoding the anti-C1q, anti-C1r, and/or anti-C1s antibody is isolated and inserted into one or more vectors for further cloning and/or expression in a host cell. Such nucleic acid may be readily isolated and sequenced using conventional procedures (*e.g*., by using oligonucleotide probes that are capable of binding specifically to genes encoding the heavy and light chains of the antibody).

Suitable vectors containing a nucleic acid sequence encoding any of the anti-Clq, anti-C1r, and/or anti-C1s antibodies of the present disclosure, or fragments thereof polypeptides (including antibodies) described herein include, without limitation, cloning vectors and expression vectors. Suitable cloning vectors can be constructed according to standard techniques, or may be selected from a large number of cloning vectors available in the art. While the cloning vector selected may vary according to the host cell intended to be used, useful cloning vectors generally have the ability to self-replicate, may possess a single target for a particular restriction endonuclease, and/or may carry genes for a marker that can be used in selecting clones containing the vector. Suitable examples include plasmids and bacterial viruses, *e.g*., pUC18, pUC19, Bluescript (*e.g*., pBS SK+) and its derivatives, mpl8, mpl9, pBR322, pMB9, ColE1, pCR1, RP4, phage DNAs, and shuttle vectors such as pSA3 and pAT28. These and many other cloning vectors are available from commercial vendors such as BioRad, Strategene, and Invitrogen.

Expression vectors generally are replicable polynucleotide constructs that contain a nucleic acid of the present disclosure. The expression vector may replicable in the host cells either as episomes or as an integral part of the chromosomal DNA. Suitable expression vectors include but are not limited to plasmids, viral vectors, including adenoviruses, adeno-associated viruses, retroviruses, cosmids, and expression vector(s) disclosed in PCT Publication No. WO 87/04462. Vector components may generally include, but are not limited to, one or more of the following: a signal sequence; an origin of replication; one or more marker genes; suitable transcriptional controlling elements (such as promoters, enhancers and terminator). For expression (*i.e*., translation), one or more translational controlling elements are also usually required, such as ribosome binding sites, translation initiation sites, and stop codons.

The vectors containing the nucleic acids of interest can be introduced into the host cell by any of a number of appropriate means, including electroporation, transfection employing calcium chloride, rubidium chloride, calcium phosphate, DEAE-dextran, or other substances; microprojectile bombardment; lipofection; and infection (*e.g*., where the vector is an infectious agent such as vaccinia virus). The choice of introducing vectors or polynucleotides will often depend on features of the host cell. In some aspects, the vector contains a nucleic acid containing one or more amino acid sequences encoding an anti-C1q, anti-C1r, and/or anti-C1s antibody of the present disclosure.

Suitable host cells for cloning or expression of antibody-encoding vectors include prokaryotic or eukaryotic cells. For example, anti-Clq, anti-Clr, and/or anti-C1s antibodies of the present disclosure may be produced in bacteria, in particular when glycosylation and Fc effector function are not needed. For expression of antibody fragments and polypeptides in bacteria (*e.g*., U.S. Patent Nos. 5,648,237, 5,789,199, and 5,840,523; and Charlton, Methods in Molecular Biology, Vol. 248 (B.K.C. Lo, ed., Humana Press, Totowa, NJ, 2003), pp. 245-254, describing expression of antibody fragments in *E. coli.*)*.* After expression, the antibody may be isolated from the bacterial cell paste in a soluble fraction and can be further purified.

In addition to prokaryotes, eukaryotic microorganisms, such as filamentous fungi or yeast, are also suitable cloning or expression hosts for antibody-encoding vectors, including fungi and yeast strains whose glycosylation pathways have been "humanized," resulting in the production of an antibody with a partially or fully human glycosylation pattern (*e.g*., Gerngross, Nat. Biotech. 22:1409-1414 (2004); and Li et al., Nat. Biotech. 24:210-215 (2006)).

Suitable host cells for the expression of glycosylated antibody can also be derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells. Plant cell cultures can also be utilized as hosts (*e.g.,* U.S. Patent Nos. 5,959,177, 6,040,498, 6,420,548, 7,125,978, and 6,417,429, describing PLANTIBODIES™ technology for producing antibodies in transgenic plants.).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, *e.g.,* in Graham et al., J. Gen Virol. 36:59 (1977)); baby hamster kidney cells (BHK); mouse sertoli cells (TM4 cells as described, *e.g.,* in Mather, Biol. Reprod. 23:243-251 (1980)); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, *e.g.,* in Mather et al., Annals N. Y. Acad. Sci. 383:44-68 (1982); MRC 5 cells; and FS4 cells. Other useful mammalian host cell lines include Chinese hamster ovary (CHO) cells, including DHFR- CHO cells (Urlaub et al., Proc. Natl. Acad. Sci. USA 77:4216 (1980)); and myeloma cell lines such as Y0, NS0 and Sp2/0. For a review of certain mammalian host cell lines suitable for antibody production, see, *e.g.,* Yazaki and Wu, Methods in Molecular Biology, Vol. 248 (B.K.C. Lo, ed., Humana Press, Totowa, NJ), pp. 255-268 (2003).

### Pharmaceutical compositions

Anti-C1q, anti-C1r, and/or anti-C1s antibodies of the present disclosure can be incorporated into a variety of formulations for therapeutic use (*e.g.,* by administration) or in the manufacture of a medicament (*e.g*., for treating NMO) by combining the antibodies with appropriate pharmaceutically acceptable carriers or diluents, and may be formulated into preparations in solid, semi-solid, liquid or gaseous forms. Examples of such formulations include, without limitation, tablets, capsules, powders, granules, ointments, solutions, suppositories, injections, inhalants, gels, microspheres, and aerosols. Pharmaceutical compositions can include, depending on the formulation desired, pharmaceutically-acceptable, non-toxic carriers of diluents, which are vehicles commonly used to formulate pharmaceutical compositions for animal or human administration. The diluent is selected so as not to affect the biological activity of the combination. Examples of such diluents include, without limitation, distilled water, buffered water, physiological saline, PBS, Ringer's solution, dextrose solution, and Hank's solution. A pharmaceutical composition or formulation of the present disclosure can further include other carriers, adjuvants, or non-toxic, nontherapeutic, nonimmunogenic stabilizers, excipients and the like. The compositions can also include additional substances to approximate physiological conditions, such as pH adjusting and buffering agents, toxicity adjusting agents, wetting agents and detergents.

A pharmaceutical composition of the present disclosure can also include any of a variety of stabilizing agents, such as an antioxidant for example. When the pharmaceutical composition includes a polypeptide, the polypeptide can be complexed with various well-known compounds that enhance the *in vivo* stability of the polypeptide, or otherwise enhance its pharmacological properties (*e.g*., increase the half-life of the polypeptide, reduce its toxicity, and enhance solubility or uptake). Examples of such modifications or complexing agents include, without limitation, sulfate, gluconate, citrate and phosphate. The polypeptides of a composition can also be complexed with molecules that enhance their *in vivo* attributes. Such molecules include, without limitation, carbohydrates, polyamines, amino acids, other peptides, ions (*e.g*., sodium, potassium, calcium, magnesium, manganese), and lipids.

Further examples of formulations that are suitable for various types of administration can be found in Remington's Pharmaceutical Sciences, Mace Publishing Company, Philadelphia, PA, 17th ed. (1985). For a brief review of methods for drug delivery, see, Langer, Science 249:1527-1533 (1990).

For oral administration, the active ingredient can be administered in solid dosage forms, such as capsules, tablets, and powders, or in liquid dosage forms, such as elixirs, syrups, and suspensions. The active component(s) can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate. Examples of additional inactive ingredients that may be added to provide desirable color, taste, stability, buffering capacity, dispersion or other known desirable features are red iron oxide, silica gel, sodium lauryl sulfate, titanium dioxide, and edible white ink. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain coloring and flavoring to increase patient acceptance.

Formulations suitable for parenteral administration include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives.

The components used to formulate the pharmaceutical compositions are preferably of high purity and are substantially free of potentially harmful contaminants (*e.g*., at least National Food (NF) grade, generally at least analytical grade, and more typically at least pharmaceutical grade). Moreover, compositions intended for *in vivo* use are usually sterile. To the extent that a given compound must be synthesized prior to use, the resulting product is typically substantially free of any potentially toxic agents, particularly any endotoxins, which may be present during the synthesis or purification process. Compositions for parental administration are also sterile, substantially isotonic and made under GMP conditions.

Formulations may be optimized for retention and stabilization in the brain or central nervous system. When the agent is administered into the cranial compartment, it is desirable for the agent to be retained in the compartment, and not to diffuse or otherwise cross the blood brain barrier. Stabilization techniques include cross-linking, multimerizing, or linking to groups such as polyethylene glycol, polyacrylamide, neutral protein carriers, *etc.* in order to achieve an increase in molecular weight.

Other strategies for increasing retention include the entrapment of the antibody, such as an anti-Clq, anti-Clr, and/or anti-C1s antibody of the present disclosure, in a biodegradable or bioerodible implant. The rate of release of the therapeutically active agent is controlled by the rate of transport through the polymeric matrix, and the biodegradation of the implant. The transport of drug through the polymer barrier will also be affected by compound solubility, polymer hydrophilicity, extent of polymer cross-linking, expansion of the polymer upon water absorption so as to make the polymer barrier more permeable to the drug, geometry of the implant, and the like. The implants are of dimensions commensurate with the size and shape of the region selected as the site of implantation. Implants may be particles, sheets, patches, plaques, fibers, microcapsules and the like and may be of any size or shape compatible with the selected site of insertion.

The implants may be monolithic, *i.e.* having the active agent homogenously distributed through the polymeric matrix, or encapsulated, where a reservoir of active agent is encapsulated by the polymeric matrix. The selection of the polymeric composition to be employed will vary with the site of administration, the desired period of treatment, patient tolerance, the nature of the disease to be treated and the like. Characteristics of the polymers will include biodegradability at the site of implantation, compatibility with the agent of interest, ease of encapsulation, a half-life in the physiological environment.

Biodegradable polymeric compositions which may be employed may be organic esters or ethers, which when degraded result in physiologically acceptable degradation products, including the monomers. Anhydrides, amides, orthoesters or the like, by themselves or in combination with other monomers, may find use. The polymers will be condensation polymers. The polymers may be cross-linked or non-cross-linked. Of particular interest are polymers of hydroxyaliphatic carboxylic acids, either homo- or copolymers, and polysaccharides. Included among the polyesters of interest are polymers of D-lactic acid, L-lactic acid, racemic lactic acid, glycolic acid, polycaprolactone, and combinations thereof. By employing the L-lactate or D-lactate, a slowly biodegrading polymer is achieved, while degradation is substantially enhanced with the racemate. Copolymers of glycolic and lactic acid are of particular interest, where the rate of biodegradation is controlled by the ratio of glycolic to lactic acid. The most rapidly degraded copolymer has roughly equal amounts of glycolic and lactic acid, where either homopolymer is more resistant to degradation. The ratio of glycolic acid to lactic acid will also affect the brittleness of in the implant, where a more flexible implant is desirable for larger geometries. Among the polysaccharides of interest are calcium alginate, and functionalized celluloses, particularly carboxymethylcellulose esters characterized by being water insoluble, a molecular weight of about 5 kD to 500 kD, *etc.* Biodegradable hydrogels may also be employed in the implants of the subject invention. Hydrogels are typically a copolymer material, characterized by the ability to imbibe a liquid. Exemplary biodegradable hydrogels which may be employed are described in Heller in: Hydrogels in Medicine and Pharmacy, N. A. Peppes ed., Vol. III, CRC Press, Boca Raton, Fla., 1987, pp 137-149.

### Pharmaceutical dosages

Pharmaceutical compositions of the present disclosure containing an anti-Clq, anti-C1r, and/or anti-C1s antibody of the present disclosure may be used (e.g., administered to an individual in need of treatment with an anti-Clq, anti-Clr, and/or anti-C1s antibody, preferably a human) in accordance with known methods, such as intravenous administration as a bolus or by continuous infusion over a period of time, by intramuscular, intraperitoneal, intracerobrospinal, intracranial, intraspinal, subcutaneous, intra-articular, intrasynovial, intrathecal, oral, topical, or inhalation routes.

Dosages and desired drug concentration of pharmaceutical compositions of the present disclosure may vary depending on the particular use envisioned. The determination of the appropriate dosage or route of administration is well within the skill of an ordinary artisan. Animal experiments provide reliable guidance for the determination of effective doses for human therapy. Interspecies scaling of effective doses can be performed following the principles described in Mordenti, J. and Chappell, W. "The Use of Interspecies Scaling in Toxicokinetics," In Toxicokinetics and New Drug Development, Yacobi et al., Eds, Pergamon Press, New York 1989, pp.42-46.

For *in vivo* administration of any of the anti-C1q, anti-C1r, and/or anti-C1s antibodies of the present disclosure, normal dosage amounts may vary from about 10 ng/kg up to about 100 mg/kg of an individual's body weight or more per day, preferably about 1 mg/kg/day to 10 mg/kg/day, depending upon the route of administration. For repeated administrations over several days or longer, depending on the severity of the disease, disorder, or condition to be treated, the treatment is sustained until a desired suppression of symptoms is achieved.

An exemplary dosing regimen may include administering an initial dose of an anti-C1q, anti-C1r, and/or anti-C1s antibody, of about 2 mg/kg, followed by a weekly maintenance dose of about 1 mg/kg every other week. Other dosage regimens may be useful, depending on the pattern of pharmacokinetic decay that the physician wishes to achieve. For example, dosing an individual from one to twenty-one times a week is contemplated herein. In certain embodiments, dosing ranging from about 3 µg/kg to about 2 mg/kg (such as about 3 µg/kg, about 10 µg/kg, about 30 µg/kg, about 100 µg/kg, about 300 µg/kg, about 1 mg/kg, or about 2 mg/kg) may be used. In certain embodiments, dosing frequency is three times per day, twice per day, once per day, once every other day, once weekly, once every two weeks, once every four weeks, once every five weeks, once every six weeks, once every seven weeks, once every eight weeks, once every nine weeks, once every ten weeks, or once monthly, once every two months, once every three months, or longer. Progress of the therapy is easily monitored by conventional techniques and assays. The dosing regimen, including the anti-C1q, anti-C1r, and/or anti-C1s antibody administered, can vary over time independently of the dose used.

Dosages for a particular anti-C1q, anti-C1r, and/or anti-C1s antibody may be determined empirically in individuals who have been given one or more administrations of the anti-C1q, anti-C1r, and/or anti-C1s antibody. Individuals are given incremental doses of an anti-C1q, anti-Clr, and/or anti-C1s antibody. To assess efficacy of an anti-Clq, anti-Clr, and/or anti-C1s antibody, any clinical symptom of NMO can be monitored.

Administration of an anti-C1q, anti-C1r, and/or anti-C1s antibody of the present disclosure can be continuous or intermittent, depending, for example, on the recipient's physiological condition, whether the purpose of the administration is therapeutic or prophylactic, and other factors known to skilled practitioners. The administration of an anti-C1q, anti-C1r, and/or anti-C1s antibody may be essentially continuous over a preselected period of time or may be in a series of spaced doses.

Guidance regarding particular dosages and methods of delivery is provided in the literature; see, for example, U.S. Patent Nos. 4,657,760; 5,206,344; or 5,225,212. It is within the scope of the invention that different formulations will be effective for different treatments and different disorders, and that administration intended to treat a specific organ or tissue may necessitate delivery in a manner different from that to another organ or tissue. Moreover, dosages may be administered by one or more separate administrations, or by continuous infusion. For repeated administrations over several days or longer, depending on the condition, the treatment is sustained until a desired suppression of disease symptoms occurs. However, other dosage regimens may be useful. The progress of this therapy is easily monitored by conventional techniques and assays.

### Therapeutic uses

Described herein are methods of inhibiting the interaction between C1q and an autoantibody, such as an AQP4-targeted autoantibody, and/or the interaction between C1q and C1r, and/or the interaction between C1q and C1s by administering to an individual a therapeutically effective amount of an anti-C1q, anti-C1r, and/or anti-C1s antibody of the present disclosure.

The present disclosure also provides anti-C1q antibodies, anti-C1r antibodies, and anti-C1s antibodies for therapeutic uses for preventing, reducing risk of developing, or treating neuromyelitis optica (NMO), by for example, inhibiting the interaction between C1q and an autoantibody, such as an AQP4-targeted autoantibody, the interaction between C1q and C1r, the interaction between C1q and C1s, the interaction between C1r and C1q, the interaction between C1r and C1s, the interaction between C1s and C1q, the interaction between C1s and C1r, and/or the interaction between C1s and C2 and/or C4, or of inhibiting the catalytic activity of C1r, the processing of pro-C1r to an active protease, the catalytic activity of C1s, and/or the processing of pro-C1s to an active protease.

As disclosed herein, anti-Clq, anti-Clr, and/or anti-C1s antibodies of the present disclosure may be used for preventing, reducing risk of developing, or treating NMO.

As disclosed herein, "neuromyelitis optica," "NMO," "Devic's disease," and "Devic's syndrome" may be used interchangeably and refer to an autoimmune 'aquaporinopathy' of the central nervous system that causes simultaneous inflammation and demyelination primarily in the spinal cord and optic nerve. Such simultaneous inflammation and demyelination may cause inflammatory demyelating lesions that can lead to paralysis, blindness, and even death. NMO patients may frequently raise autoantibodies (NMO-IgG) that are generally directed at astrocyte water channel aquaporin-4 (AQP4), and are known to activate the complement system, resulting in widespread tissue damage. NMO may affect children as young as 3 years and adults as old as 90 years. NMO can be more common in women than men, with women comprising over two-thirds of patients and more than 80% of those with the relapsing form of the disease. NMO appears to be present across the world. Africans and Asians, especially in the Far East, may have a higher risk of NMO, although the exact incidence is unknown. The majority of NMO patients appear to have no affected relatives, and as such NMO may be generally regarded as a nonfamilial condition. NMO may be monophasic (*i.e*., a single episode with permanent remission), or relapsing. In patients with the monophasic form, the transverse myelitis and optic neuritis may occur simultaneously or within days of each other. Alternatively, patients with the relapsing form may be more likely to have weeks or months between the initial attacks, and to have better motor recovery after the initial transverse myelitis event. Relapses may usually occur early, with relapses occurring in the first year to first five years after the first acute attack. Disabilities due to NMO may arise from the acute attacks.

NMO symptoms may include, without limitation, inflammatory demyelating lesions in the spinal cord and /or optic nerve, decrease in visual acuity, visual field defects, loss of color vision, loss of vision, loss of spinal cord function, muscle weakness, reduced sensation, loss of bladder and bowel control, acute and severe spastic weakness of the legs (paraparesis), acute and severe spastic weakness of all four limbs (quadriparesis), loss of motor control, paralysis in one or both legs, respiratory failure, and death.

Accordingly, the antibodies of the present disclosure may also be used to treat or improve one of more symptoms of NMO. In some aspects, the present disclosure provides methods of preventing, treating, or improving one or more symptoms in individuals having NMO, by administering an anti-Clq, anti-Clr, and/or anti-C1s antibody of the present disclosure to, for example, inhibit the interaction between C1q and an autoantibody, such as an AQP4-targeted autoantibody, the interaction of C1q and C1r, and/or the interaction of C1q and C1s. The present disclosure also describes methods of preventing lesion formation in individuals having NMO by administering an anti-Clq, anti-Clr, and/or anti-C1s antibody of the present disclosure to inhibit the interaction between C1q and an autoantibody, such as an AQP4-targeted autoantibody, the interaction of C1q and C1r, or the interaction of C1q and C1s. The anti-Clq, anti-C1r, and/or anti-C1s antibody may be administered to cells *in vitro* to prevent complement dependent cytotoxicity or complement-dependent cell-mediated cytotoxicity. The anti-Clq, anti-C1r, and/or anti-C1s antibody may also be administered *ex vivo* in spinal cord slice models of NMO. Alternatively, the anti-C1q, anti-C1r, and/or anti-C1s antibody may be administered *in vivo* (*e.g*., by administering the antibody to an individual, such as a murine or human individual) to prevent lesion formation (as indicated, *e.g*., by loss of AQP4, GFAP, or myelin.

### Combination Treatments

The antibodies for use in the methods of the present disclosure may be used, without limitation, in combination with any additional treatments of NMO, including immunosuppressive therapies, and/or with any additional treatments of other diseases.

In some embodiments, the methods of this disclosure include the administration of therapeutically effective amounts of a first antibody selected from an anti-Clq, anti-Clr, and/or anti-C1s antibody and a second antibody selected from an anti-Clq, anti-Clr, and/or anti-C1s antibody. In certain embodiments, the methods further include the administration of a therapeutically effective amount of a third antibody selected from an anti-Clq, anti-Clr, and/or anti-C1s antibody.

In some embodiments, the methods of this disclosure further include administration of an inhibitor of antibody-dependent cellular cytotoxicity (ADCC). ADCC inhibitors may include, without limitation, soluble NK cell inhibitory receptors such as the killer cell Ig-like receptors (KIRs), which recognize HLA-A, HLA-B, or HLA-C and C-type lectin CD94/NKG2A heterodimers, which recognize HLA-E (*see, e.g*., López-Botet M., T. Bellón, M. Llano, F. Navarro, P. Garcia & M. de Miguel. (2000), Paired inhibitory and triggering NK cell receptors for HLA class I molecules. Hum. Immunol. 61: 7-17; Lanier L.L. (1998) Follow the leader: NK cell receptors for classical and nonclassical MHC class I. Cell 92: 705-707.), and cadmium (*see, e.g*., Immunopharmacology 1990; Volume 20, Pages 73-8).

In some embodiments, the methods of this disclosure further include administration of an inhibitor of the alternative pathway of complement activation. Such inhibitors may include, without limitation, factor B blocking antibodies, factor D blocking antibodies, soluble, membrane-bound, tagged or fusion-protein forms of CD59, DAF, CR1, CR2, Crry or Comstatin-like peptides that block the cleavage of C3, non-peptide C3aR antagonists such as SB 290157, Cobra venom factor or non-specific complement inhibitors such as nafamostat mesilate (FUTHAN; FUT-175), aprotinin, K-76 monocarboxylic acid (MX-1) and heparin (*see, e.g.,* T.E. Mollnes & M. Kirschfink, Molecular Immunology 43 (2006) 107-121). In some embodiments, the methods of this disclosure further include administration of an inhibitor of the interaction between the autoantibody and its autoantigen. In some embodiments, the autoantibody is an NMO-Igg. In some embodiments, the autoantibody binds Aquaporin 4 (AQP4). Such inhibitors may include purified soluble forms of the autoantigen, or antigen mimetics such as peptide or RNA-derived mimotopes, including mimotopes of the AQP4 antigen. Alternatively, such inhibitors may include blocking agents that recognize the autoantigen and prevent binding of the autoantibody without triggering the classical complement pathway. Such blocking agents may include, *e.g*., autoantigen-binding RNA aptamers or antibodies lacking functional C1q binding sites in their Fc domains (*e.g*., Fab fragments or antibody otherwise engineered not to bind C1q).

### Kits

Also described herein are kits for use in the methods as described herein. Kits of the disclosure include one or more containers comprising a purified anti-C1q, anti-C1r, and/or anti-C1s antibody and instructions for use in accordance with any of the methods described herein. Generally, these instructions comprise a description of administration of the inhibitor to prevent, reduce the risk of developing, and/or treat NMO, according to any of the methods described herein. The kit may further comprise a description of selecting an individual suitable for treatment based on identifying whether that individual has or is at risk of developing the disease and/or the stage of the disease.

The instructions may generally include information as to dosage, dosing schedule, and route of administration for the intended treatment. The containers may be unit doses, bulk packages (e.g., multi-dose packages) or sub-unit doses. Instructions supplied in the kits of the invention are typically written instructions on a label or package insert (*e.g*., a paper sheet included in the kit), but machine-readable instructions (*e.g*., instructions carried on a magnetic or optical storage disk) are also acceptable.

The label or package insert indicates that the composition is used for treating NMO. Instructions may be provided for practicing any of the methods described herein.

The kits described herein are in suitable packaging. Suitable packaging includes, but is not limited to, vials, bottles, jars, flexible packaging (*e.g*., sealed Mylar or plastic bags), and the like. Also contemplated are packages for use in combination with a specific device, such as an inhaler, nasal administration device (*e.g*., an atomizer) or an infusion device such as a minipump. A kit may have a sterile access port (for example the container may be an intravenous solution bag or a vial having a stopper pierceable by a hypodermic injection needle). The container may also have a sterile access port (*e.g.,* the container may be an intravenous solution bag or a vial having a stopper pierceable by a hypodermic injection needle). At least one active agent in the composition is an inhibitor of classical complement pathway. The container may further comprise a second pharmaceutically active agent.

Kits may optionally provide additional components such as buffers and interpretive information. Normally, the kit comprises a container and a label or package insert(s) on or associated with the container.

The invention will be more fully understood by reference to the following Examples.

### EXAMPLES

### Example 1: Materials and Methods

### Cell culture and antibodies

Human M23-AQP4 expressing Chinese hamster ovary (CHO) cells were generated by stable transfection as described (Phuan et al. (2012) J Biol Chem. 287(17):13829-13839). CHO cells were cultured in F-12 Ham's Nutrient mix medium supplemented with 10% fetal bovine serum, 100 units/ml penicillin, and 100 µg/ml streptomycin. Geneticin (200 µg/ml) was used as selection marker. Cells were grown at 37°C in 5% CO₂ / 95% air. Recombinant monoclonal NMO antibodies (rAb-53) were generated from clonally-expanded plasma blasts from cerebrospinal fluid (CSF) of NMO patients and purified as described (Bennett et al. (2009) Ann Neurol. 66(5):617-629). Isotype-matched control antibodies included a non-NMO rAb (rAb-2B4) against measles virus nucleocapsid protein, and mouse IgG1 kappa monoclonal (ab 18447) from Abcam (Cambridge, MA). NMO serum was obtained from NMO-IgG seropositive individuals who met the revised diagnostic criteria for clinical disease (Wingerchuk et al. (2006) Lancet Neurol. 6(9):805-815). Non-NMO human serum was used as control. Purified C1q protein and C1q-depleted serum were purchased from Complement Technology Inc. (Tyler, TX). Mouse monoclonal antibodies against C1q (C1q^{mAb}) and C1s (two antibodies: C1s^{mAb1}, C1s^{mAb2}) were generated and purified as described in Example 2.

### Complement-dependent cytotoxicity (CDC)

M23-AQP4-expressing CHO cells were plated onto 96-well microplates (Costar, Corning Inc., Corning, NY) at 20,000 cells/well and grown at 37°C / 5% CO₂ for 18-24 h. Cells were washed with phosphate-buffered saline (PBS) and incubated at room temperature for 30 min with 50 µL of rAb-53 or heat-inactivated NMO serum in live cell buffer (PBS containing 6 mM glucose and 1 mM sodium pyruvate). A solution containing pooled normal human complement serum (Innovative Research, Novi, MI) and anti-C1 antibody was incubated on ice for 30 min before added to the CHO cells. Some experiments were done using purified C1q and C1q-depleted serum in which specified quantities of C1q were added to C1q-depleted serum. For studies of the alternative complement pathway, C1q-depleted or factor-B depleted serum (Quidel, Inc., San Diego, CA) was used instead of normal serum. In some experiments, 100 µg/ml neutralizing anti-factor B antibody (R&D Systems, Inc., Mineapolis, MN) was incubated with normal serum 30 min prior to CDC assay. After addition of human complement, rAb-53 or heat-inactivated NMO serum and anti-C1 antibody, cells were incubated for 60 min at 27°C, washed once with PBS, and incubated with 50 µl of a 20% AlamarBlue solution for 45 min at 27°C. Cytotoxicity was measured from resorufin fluorescence using a TECAN Infinite M1000 plate reader (TECAN Groups Ltd, Mannedorf, Switzerland) (excitation/emission 560/590 nm). For live/dead cells staining, cells were washed with PBS and then incubated with 1 µM calcein-AM (live cells, green fluorescence) and 2 µM ethidium homodimer-1 (dead cells, red fluorescence) (Invitrogen) in PBS for 15 min prior to imaging.

### Antibody-dependent cell-mediated cytotoxicity (ADCC)

M23-AQP4-expressing CHO cells on 96-well microplates (as for CDC) were washed with PBS and incubated for 1 h at 37°C with rAb-53 (1 and 10 µg/ml) or control-rAb, and NK cells overexpressing CD16 (Conkwest, San Diego, CA) as effector cells at an effector:target cell ratio of 20:1, with or without C1q^{mAb}. Wells were washed gently five times with PBS to remove NK cells before addition of 50 µl of 20% AlamarBlue solution. Cells were incubated for 45 min at 27°C and cytotoxicity was determined from resorufin fluorescence.

### Complement-dependent cell-mediated cytotoxicity (CDCC)

M23-AQP4-expressing CHO cells on 96-well microplates were washed with PBS and incubated for 1 h at 37°C with rAb-53 (0.25 µg/ml), 1% human complement and NK cells (effector:target cell ratio 20:1), with or without 2.5 µg/ml C1q^{mAb}. Wells were washed to remove NK cells and cytotoxicity was measured as above.

### Surface plasmon resonance

Surface plasmon resonance (SPR) measurements were done on a Biacore T100 instrument (GE Healthcare, Piscataway, NJ). Purified C1q protein was diluted with 10 mM MES buffer (pH 6.5) to 10 µg/ml and immobilized onto the surface of a carboxymethylate-functionalized CM5 sensor chip (GE Healthcare) using standard carbodiimide coupling. Unreacted surface was blocked by injection of 1 M ethanolamine-HCl. The reference control flow cell was coupled with 1 M ethanolamine-HCl only. Binding was measured at 25°C using pH 7.4 Hepes-buffered saline as running buffer at a flow rate of 20 µl/min. C1q^{mAb} and a control mouse IgG1 antibody in identical buffer were injected for 180 s over sensor chip followed by a 300 s washout period at the same flow rate. Sensorgrams were corrected by subtraction of responses of the unloaded reference flow cell. Equilibrium constants for antibody-antigen binding were determined using a 1:1 Langmuir binding-affinity model.

### NMO-IgG binding

M23-AQP4-expressing CHO cells were plated onto 96-well microplate and grown for 18-24 h to confluence. Cells were washed twice with PBS and blocked with 1% bovine serum albumin (BSA) in PBS for 30 min. After removal of blocking solution, a premixed solution (10 µl) of rAb-53 (1.5 µg/ml) and horseradish peroxidase (HRP)-labeled goat anti-human IgG secondary antibody (1:500 dilution; Invitrogen) was added. Following 1 h incubation at room temperature, cells were washed 3 times with PBS containing 0.05% Tween-20, and HRP activity was assayed by addition of 50 µl Amplex Red substrate (100 µM, Sigma) and 2 mM H₂O₂ as described (Tradtrantip et al. (2012) FASEB J. 26:2197-2208). Fluorescence was measured after 10 min (excitation/emission 540/590 nm).

### Immunofluorescence

Cells were grown on glass coverslips for 24 h and then washed with PBS and incubated for 30 min in live cell blocking buffer (PBS containing 6 mM glucose, 1 mM sodium pyruvate, 1% BSA). Cells were incubated for 60 min with rAb-53 in blocking buffer at a final concentration of 1 µg/ml. Cells were then rinsed in PBS, fixed in 4% paraformaldehyde for 15 min, and permeabilized with 0.1% Triton X-100. Cells were incubated for 60 min with 0.4 µg/ml polyclonal, C-terminal specific rabbit anti-AQP4 antibody (H-80, Santa Cruz Biotechnology, Santa Cruz, CA) and then for 30 min with 4 µg/ml goat anti-human IgG-conjugated Alexa Fluor 488 and 4 µg/ml goat anti-rabbit IgG-conjugated Alexa Fluor 555 (Invitrogen) in blocking buffer, as described (Crane et al. (2011) J Biol Chem. 286(18):16516-16524). Red and green fluorescence was imaged on a Nikon Eclipse TE2000S inverted epifluorescence microscope (Nikon, Melville, NY).

### Ex vivo spinal cord slice model of NMO

Transverse slices of cervical spinal cord from 7-day old CD1 mice were cut 300 µm thickness using a vibratome and placed in ice-cold Hank's balanced salt solution (HBSS, pH 7.2), as described (Zhang et al. (2011) Ann Neurol. 70(6):943-954). All animal studies were approved by the UCSF Committee on Animal Research. Slices were placed on transparent membrane inserts (Millipore, Millicell-CM 0.4 µm pores, 30 mm diameter) in 6-well plates containing 1 ml culture medium, with a thin film of culture medium covering the slices. Slices were cultured in 5% CO₂ at 37°C for 7 days in 50% MEM, 25% HBSS, 25% horse serum, 1% penicillin-streptomycin, 0.65% glucose and 25 mM HEPES. On day 7, slices were incubated for 1 h with rAb-53 (10 µg/mL) and human complement (5 %). Slices were cultured for an additional 24 h, and immunostained for AQP4 and glial fibrillary acid protein (GFAP). Sections were scored as follows: 0, intact slice with normal GFAP and AQP4 staining; 1, mild astrocyte swelling and/or reduced AQP4 staining; 2, at least one lesion with loss of GFAP and AQP4 staining; 3, multiple lesions affecting > 30 % of slice area; 4, lesions affecting > 80 % of slice area.

### In vivo intracerebral injection model of NMO

Adult CD1 mice (30-35 g) were anesthetized with 2,2,2-tribromoethanol (125 mg/kg intraperitoneally) and mounted in a stereotactic frame. Following a midline scalp incision, a burr hole of diameter 1 mm was made 2 mm to the right of the bregma. A 30-gauge needle attached to 50 µl gas-tight glass syringe was inserted 3 mm deep, as described (Saadoun et al. (2010) *Brain* 133(Pt 2):349-361) to infuse 0.9 µg of rAb-53, 3 µl of human complement (with or without) 1.3 µg C1q^{mAb} in a total volume of 8 µl (at 2 µl/min). 3 µl of human complement and 1.3 µg C1q^{mAb} (total volume of 8 µl) was injected as a control. After 3 days mice were anesthetized and perfused through the left cardiac ventricle with 5 ml PBS and then 20 ml PBS containing 4% PFA. Brains were post-fixed for 2 h in 4% PFA. 5-mm thick paraffin sections were immunostained for AQP4, GFAP and MBP, as described (Ratelade et al. (2012) Acta Neuropathol. 123(6):861-872). Data are presented as percentage of immunonegative area, as defined by hand and quantified using ImageJ, normalized to total area of hemibrain slice.

### Example 2: Production and Characterization of anti-C1q and anti-C1s Antibodies

The anti-C1q antibody 4A4B11, also referred to as C1q^{mAb}, is described in U.S. Patent No. 4,595,654 and the hybridoma for this line is available from ATCC (ATCC HB-8327TM).

The C1s antibodies 5A1 and 5C12, also referred to as C1s^{mAb1} and C1s^{mAb2}, were generated by immunizing mice with human C1s enzyme purified from human plasma (Complement Technology Inc. Tyler Texas, Cat # A103) using standard mouse immunization and hybridoma screening technologies (Milstein, C (1999). Bioessays 21: 966-73; Mark Page, Robin Thorpe, The Protein Protocols Handbook 2002, Editors: John M. Walker, pp 1111-1113). In brief, female BALB/c mice were injected intraperitoneal with 25 µg of protein in complete Freund's adjuvant (CFA) on Day 0 and boosts were done with 25 µg of C1s enzyme in incomplete Freund's adjuvant (IFA) on days 21, 42, 52, and a final intravenous boost on Day 63. Four days following the final boost the mice were euthanized, spleens removed and splenocytes were fused with the myeloma cell line SP2/0. Fused cells were grown on hypoxanthine-aminopterin-thymidine (HAT) selective semi-solid media for 10-12 days and the resulting hybridomas clones were transferred to 96-well tissue culture plates and grown in HAT medium until the antibody titre is high. The antibody rich supernatants of the clones were isolated and tested in an ELISA assay for reactivity with C1s. Positive clones are isotyped and cultured for 32 days (post HAT selection) to identify stable expressing clones.

The C1s antibodies were deposited with ATCC on 5/15/2013 (ATCC Accession Number PTA-120351 and PTA-120352).

ELISA assays were used to determine the specificity of the antibodies generated against the C1s enzyme. The day before the assay was performed plates were coated at 0.2 µg/well of C1s-Enzyme antigen in 100 µL/well carbonate coating buffer pH9.6 overnight at 4°C. Blocked with 3% Milk powder in PBS for 1 hour at room temperature Hybridoma TC Sup on plates at 100 µL/well for 1 hour at 37°C with shaking Secondary antibody: 1:10,000 Goat antiMouse IgG/IgM(H+L)-HRP at 100 µL/well for 1.5 hours at room temperature with shaking TMB Substrate added at 50 µL/well for 5 minutes at room temperature in the dark Reaction stopped with 50 µL/well 1M HCl and read at 450nm.

C1q and C1s antibodies were tested for inhibitory activity in complement hemolytic assays. The C1q antibody 4A4B11 was tested for inhibitory activity in a CH50 hemolytic assay essentially as described in Current Protocols in Immunology (1994) Supplement 9 Unit 13.1. The C1s antibodies were tested for inhibitory activity in a C1H50 assay. This assay was performed at the Complement Laboratory at the National Jewish Hospital (Denver CO) and is a modified CH50 assay that provides limiting quantities of the C1 complex from human serum to provide greater sensitivity for assessing C1 activity and potential C1 inhibitors. In brief, the assay is as follows. First, 3x10⁷ sheep red blood cells (RBC) are incubated with anti-sheep RBC IgM antibody to generate activated erythrocytes (EA cells). The EA cells are incubated with purified C4b protein to create EAC4b cells. These cells are incubated with diluted (1:1000-1:10000) normal human serum (NHS) that is pre-incubated with or without anti-C1s and control mouse IgG antibodies, to provide a limiting quantity of human C1. The resulting EAC14 cells are incubated with purified human C2 protein to generate EAC14b2a cells and then guinea pig serum in an EDTA buffer at 37°C for 30 minutes is added. Cell lysis is measured in a spectrophotometer at 450 nm.

The C1s antibodies 5A1 and 5C12 were tested for the ability to inhibit C1s cleavage of the C4 protein in vitro. Human C1s enzyme (2 ng; Complement Technology Inc., Catalog #A103) was incubated with an approximately 10-fold molar excess (25 ng) of C1s antibodies for 30 minutes at 4°C. Protein dilutions were made in PBS containing 0.1 mg gelatin/mL. The antibody/C1s mixture was incubated with 3 mg of human C4 protein (Complement Technology Inc. Cat # A105) for 5 minutes at 37°C, SDS-DTT Sample buffer was added to each sample, mixed and immediately placed in a 37°C water bath for 15 minutes. Samples loaded immediately onto aNuPage 10% BisTris SDS gel (Invitrogen Life Technologies) gel and ran for 1 hour at 150 V. The gel was fixed for 1 hour, stained with Coomassie Blue for 24 hr and destained overnight.

Surface plasmon resonance (SPR) was used to measure C1q^{mAb} binding affinity to C1q. Surface plasmon resonance (SPR) measurements were done on a Biacore T100 instrument (GE Healthcare, Piscataway, NJ). Purified C1q protein was diluted with 10 mM MES buffer (pH 6.5) to 10 µg/ml and immobilized onto the surface of a carboxymethylate-functionalized CM5 sensor chip (GE Healthcare) using standard carbodiimide coupling. Unreacted surface was blocked by injection of 1 M ethanolamine-HCl. The reference control flow cell was coupled with 1 M ethanolamine-HCl only. Binding was measured at 25°C using pH 7.4 Hepes-buffered saline as running buffer at a flow rate of 20 µl/min. C1q^{mAb} and a control mouse IgG1 antibody in identical buffer were injected for 180 s over sensor chip followed by a 300 s washout period at the same flow rate. Sensorgrams were corrected by subtraction of responses of the unloaded reference flow cell. Equilibrium constants for antibody-antigen binding were determined using a 1:1 Langmuir binding-affinity model.

### Example 3: Anti-C1q and anti-C1s Antibodies Inhibit Complement-Dependent Lysis of AQP4 Expressing Cells Incubated with anti-AQP4 Antibodies

Complement dependent lysis (CDC) was determined essentially as described in Tradtrantip et al. (2013) Annals of Neurology 73:77-85. Briefly, CDC caused by NMO-IgG binding to AQP4 was measured in AQP4-expressing cell cultures, in which human complement was incubated for 30 min with monoclonal antibodies against C1q (C1q^{mAb}) or C1s (C1s^{mAb1}, C1s^{mAb2}) prior to addition to cells. Cytotoxicity was assayed using the AlamarBlue assay.

**FIG.** 3A shows that C1q^{mAb}, C1s^{mAb1} and C1s^{mAb2} prevented CDC in a concentration-dependent manner in cells exposed to the monoclonal NMO antibody rAb-53 (1.5 µg/ml) and human complement (2% human serum). EC₅₀ for the C1 antibodies were -750 ng/ml. In control studies, a non-specific mouse IgG1 antibody did not prevent CDC. Antibody efficacy was also demonstrated in a live/dead cell staining assay **(****Fig. 3B****).** The C1q antibody, which was further studied, was also effective in preventing CDC caused by human NMO sera.

**FIG. 3C** shows C1q^{mAb} prevention of CDC in cells incubated with 2.5% heat-inactivated sera from five different NMO patients, together with 2% human complement. **FIG. 3D** shows that C1q^{mAb} reduced CDC in primary cultures of murine astrocytes. To produce robust CDC in astrocytes, a mutated, CDC-enhanced recombinant NMO-IgG was used because astrocytes express complement inhibitor proteins such as CD59.

**FIG. 3E** shows C1q^{mAb} prevention of CDC as a function of rAb-53 concentration at fixed 2% complement. EC₅₀ was approximately independent of rAb-53 concentration. **FIG. 3F** shows CDC as a function of complement concentration at fixed rAb-53 concentration of 1.5 µg/ml. The increased EC₅₀ with increasing complement is due to the greater amount of C1q^{mAb} needed to neutralize the greater amount of C1q.

### Example 4: Characterization of C1q^{mAb}

Surface plasmon resonance (SPR) was used to measure C1q^{mAb} binding affinity to C1q. Purified C1q protein was covalently immobilized by primary amine coupling to the carboxymethylated dextran matrix of a CM5 sensor chip. **FIG. 4A** shows C1q binding curves for different concentrations of C1q^{mAb}. C1q^{mAb} produced a concentration-dependent increase in SPR signal, showing fast binding and very slow dissociation, which is characteristic of a high-affinity antibody-antigen binding interaction. C1q binding was not seen for a control mouse IgG1 antibody. Using a 1:1 binding model, the dissociation constant (*K_{d}*) for C1q^{mAb} binding to C1q was 11 nM.

The stoichiometry of C1q^{mAb} binding to C1q was measured, which was not predictable *a priori* because of the multi-valency of both the antibody and antigen. C1q^{mAb} is an immunoglobulin G antibody with bivalent binding sites, and C1q is a hexavalent glycoprotein containing six globular heads that are connected to a central subunit by a collagen-like strand (**FIG. 2****;** Gadjeva et al., 2008, Kishore and Reid, 2000). The C1q^{mAb} : C1q binding stoichiometry could theoretically range from 1:1 to 6:1. **FIG. 4B** shows the C1q^{mAb} concentration-dependence of CDC for different concentrations of purified C1q protein added to C1q-depleted human serum. Linear regression analysis of EC₅₀ values gave a stoichiometry of ∼1.5:1. Whether C1q^{mAb} binds to C1q in a bivalent or monovalent manner is not determined at this point.

The kinetics of C1q^{mAb} binding to C1q was measured by incubation of C1q^{mAb} and complement for different times before addition to cells with rAb-53 already bound to AQP4. **FIG. 4C** shows the time course of cytoprotection, with t_{1/2} ∼ 15 min under the conditions of the experiment.

To investigate additive effects of different antibodies used in combination, CDC was measured at different concentrations of C1q^{mAb} and C1s^{mAb1}, each with 2% human complement and 1.5 µg/ml rAb-53. **Fig. 4D** illustrates additive effects on CDC that were observed for combinations of C1q^{mAb} and C1s^{mAb1} antibodies.

Control studies were performed to demonstrate that rAb-53 binding to M23-AQP4 is not affected by C1q^{mAb}. At a high C1q^{mAb} concentration of 2.5 µg/ml, no effect on rAb-53 binding to AQP4 was seen as measured by immunofluorescence (**FIG. 4E****,** left) or using a HRP-based AmplexRed assay (**FIG. 4E****,** right).

### Example 5: C1q Antibody Inhibits Complement-Dependent Cell-Mediated Cytotoxicity but not Antibody-Dependent Cellular Cytotoxicity

Antibody-dependent cellular cytotoxicity (ADCC) involves binding of effector cell FcRy receptors to an IgG1 Fc region (**FIG. 5A****,** top). Using NK cells as effector cells, incubation of NK cells and rAb-53 in M23-AQP4-expressing CHO cells produced ADCC (**FIG. 5A****,** bottom). Inclusion of a high concentration (2.5 µg/ml) of C1q^{mAb} did not reduce cytotoxicity caused by an ADCC mechanism. As controls, incubation of NK-cells with C1q^{mAb} or control antibody did not produce cytotoxicity.

Complement-dependent cell-mediated cytotoxicity (CDCC) involves complement-dependent enhancement in effector cell recruitment, function and AQP4 binding (**FIG. 5B****,** top). To assay CDCC, sub-maximal rAb-53 (0.25 µg/ml) and human complement (1%) was used, which produced little cytotoxicity in the absence of effector cells (**FIG. 5B****,** bottom). Addition of sub-maximal concentration of NK cells produced significant cytotoxicity when added together with rAb-53 and complement, whereas the same concentration of NK cells added only with rAb-53 did not cause cytotoxicity. Inclusion of 2.5 µg/ml C1q^{mAb} prevented CDCC produced by the combination of rAb-53, complement and NK cells.

The contribution of the alternative complement pathway to NMO-IgG-dependent CDC was investigated. Exposure of AQP4-expressing cells to rAb-53 and factor B-depleted serum, or to serum preincubated anti-factor-B antibody, showed ∼2-fold reduced CDC compared to normal human complement **(****FIG. 5C****).** The alternative complement pathway thus amplifies CDC initiated by C1q binding.

### Example 6: C1q^{mAb} Prevents Lesion Formation in an Ex Vivo Spinal Cord Slice Model of NMO

C1q^{mAb} efficacy was tested in an *ex vivo* spinal cord slice culture model of NMO in which NMO-IgG and complement produces lesions with loss of GFAP, AQP4, and myelin, deposition of activated complement, and activation of microglia (Zhang and Verkman, 2011).

As diagrammed in **FIG. 6A****,** 300 µm-thick spinal cord slices were cultured for 7 days, then incubated for 24 h with rAb-53, and complement, with or without C1q^{mAb}, and then immunostained for AQP4 and GFAP as markers of NMO pathology. **FIG. 6B** shows marked loss of AQP4 and GFAP immunoreactivity in spinal cord slices incubated for 24 h with 10 µg/ml rAb-53 and 5 % human complement. **FIG. 6C** summarizes lesion scores (0, no pathology; 4, extensive pathology). Inclusion of C1q^{mAb} during the 24 h incubation with rAb-53 and complement greatly reduced lesion severity. In control studies, rAb-53, complement or C1q^{mAb}, each alone, did not produce pathology.

### Example 7: C1q^{mAb} Prevents Lesion Formation in an In Vivo Mouse Model of NMO

The efficacy of C1q^{mAb} was tested in an *in vivo* mouse model of NMO in which intracerebral injection of rAb-53 and human complement produces marked loss of AQP4, GFAP and myelin **(****FIG. 7A****).** **FIG. 7B** shows a higher magnification of the lesion, showing loss of AQP4, GFAP, and myelin. The lesions are surrounded by reactive astrocytes that overexpress GFAP. Intracerebral injection of rAb-53 and human complement together with C1q^{mAb} produced little loss of AQP4, GFAP or myelin. As control, intracerebral injection of human complement and C1q^{mAb}, without rAb-53, did not cause pathology. Quantification of lesion size showed greatly reduced lesion size when C1q^{mAb} was present **(****FIG. 7C****).**

### Example 8: Characterization of Anti-C1r and Anti-C1s Antibodies

### C1r and C1s antibodies inhibit complement-dependent cell-mediated cytotoxicity but not antibody-dependent cellular cytotoxicity

Utilizing the materials and methods described in Examples 1 and 5, anti-C1r and anti-C1s antibodies are tested for their ability to inhibit antibody-dependent cellular cytotoxicity (ADCC) and complement-dependent cell-mediated cytotoxicity (CDCC).

Using NK cells as effector cells, incubation of NK cells and rAb-53 in M23-AQP4-expressing CHO cells produce ADCC. A high concentration (2.5 µg/ml) of anti-C1r or anti-C1s antibodies is included and ADCC is measured to determine whether cytotoxicity is reduced caused by an ADCC mechanism.

To assay CDCC, sub-maximal rAb-53 (0.25 µg/ml) and human complement (1%) are used, which produces little cytotoxicity in the absence of effector cells. Addition of sub-maximal concentration of NK cells produces significant cytotoxicity when added together with rAb-53 and complement, whereas the same concentration of NK cells added only with rAb-53 does not cause cytotoxicity. A high concentration (2.5 µg/ml) of anti-C1r or anti-C1s antibodies is included and CDCC is measured to determine whether the anti-C1r or anti-C1s antibodies prevent CDCC produced by the combination of rAb-53, complement and NK cells.

### C1r and C1s antibodies prevent lesion formation in an ex vivo spinal cord slice model ofNMO

Utilizing the materials and methods described in Examples 1 and 6, the efficacy of anti-C1r and anti-C1s antibodies is tested in an *ex vivo* spinal cord slice culture model of NMO in which NMO-IgG and complement produces lesions with loss of GFAP, AQP4, and myelin, deposition of activated complement, and activation of microglia (Zhang and Verkman, 2011).

300 µm-thick spinal cord slices are cultured for 7 days, and then incubated for 24 h with rAb-53, and complement, with or without anti-C1r or anti-C1s antibodies, and then immunostained for AQP4 and GFAP as markers of NMO pathology. Lesion severity scores (0, no pathology; 4, extensive pathology) are measured as described in Example 6 with and without inclusion of the anti-C1r or anti-C1s antibodies.

### C1r and C1s antibodies prevent lesion formation in an in vivo mouse model of NMO

Utilizing the materials and methods described in Examples 1 and 7, the efficacy of anti-C1r and anti-C1s antibodies is tested in an *in vivo* mouse model of NMO in which intracerebral injection of rAb-53 and human complement produces marked loss of AQP4, GFAP and myelin. In this model, the lesions are surrounded by reactive astrocytes that overexpress GFAP. Intracerebral injection of rAb-53 and human complement together with anti-C1r and anti-C1s antibodies is tested for the induction of a loss of AQP4, GFAP or myelin. Lesion size is then quantified to determine whether anti-C1r and/or anti-C1s antibodies reduce lesion size when in the mice.

### DEPOSIT OF MATERIAL

The following materials have been deposited according to the Budapest Treaty in the American Type Culture Collection, ATCC Patent Depository, 10801 University Blvd., Manassas, Va. 20110-2209, USA (ATCC):

| **Sample ID** | **Isotype** | **Deposit Date** | **ATCC Accession No.** |
|---|---|---|---|
| Mouse anti-C1s-RP mAb cell line 5A1 IgG1 producing antibody 5A1 | IgG1, kappa | 5/15/2013 | PTA-120351 |
| Mouse anti-Cls-RP mAb cell line 5C12 IgG1 producing antibody 5C12 | IgG1, kappa | 5/15/2013 | PTA-120352 |

The hybridoma cell lines producing the 5A1 and 5C12 antibodies (mouse anti-C1s-RP mAb cell line 5A1 IgG1 and mouse anti-C1s-RP mAb cell line 5C12 IgG1) have each been deposited with ATCC under conditions that assure that access to the culture will be available during pendency of the patent application and for a period of 30 years, or 5 years after the most recent request, or for the effective life of the patent, whichever is longer. A deposit will be replaced if the deposit becomes nonviable during that period. Each of the deposits is available as required by foreign patent laws in countries wherein counterparts of the subject application, or its progeny are filed. However, it should be understood that the availability of the deposits does not constitute a license to practice the subject invention in derogation of patent rights granted by governmental action.

### REFERENCES

Aberer, W. Hereditary angioedema treatment options: the availability of new therapies. Ann Med. 2012, 44(6), 523-9.
Ayzenberg, I, Kleiter, I, Schröde,r A, Hellwig, K, Chan, A, Yamamura, T, Gold, R. Interleukin 6 Receptor Blockade in Patients With Neuromyelitis Optica Nonresponsive to Anti-CD20 Therapy. JAMA Neurol. 2013, 1-4.
Bennett, JL, Lam, C, Kalluri, SR, Saikali, P, Bautista, K, Dupree, C, Glogowska, M, Case, D, Antel, JP, Owens, GP, Gilden, D, Nessler, S, Stadelmann, C, Hemmer, B. Intrathecal pathogenic anti-aquaporin-4 antibodies in early neuromyelitis optica. Ann Neurol. 2009, 66(5), 617-629.
Botto, M, Agnola, CD, Bygrave, AE, Thompson, EM, Cook, HT, Petry, F, Loos, M, Pandolfi PP, and Walport, MJ. Homozygous C1q deficiency causes glomerulonephritis associated with multiple apoptotic bodies. Nature Genetics, 1998, 19, 56-59.
Botto, M, Walport, MJ. C1q, autoimmunity and apoptosis. Immunobiology. 2002, 205, 395-406. Crane, JM, Lam, C, Rossi, A, Gupta, T, Bennett, JL, and Verkman, AS. Binding affinity and specificity of neuromyelitis optica autoantibodies to aquaporin-4 M1/M23 isoforms and orthogonal arrays. J Biol Chem. 2011, 286(18), 16516-16524.
Gadjeva, MG, Rouseva, MM, Zlatarova, AS, Reid, KB, Kishore, U, and Kojouharova MS. Interaction of human C1q with IgG and IgM: revisited. Biochemistry 2008, 47(49), 13093-102. Georgy MS, Pongracic JA. Chapter 22: Hereditary and acquired angioedema. Allergy Asthma Proc. 2012, 33, Suppl 1:S73-6.
Ghai, R, Waters, P, Roumenina, LT, Gadjeva, M, Kojouharova, MS, Reid, KB, Sim, RB, and Kishore, U. C1q and its growing family. Immunobiology 2007, 212(4-5), 253-66.
Gomez, AM, Van Den Broeck, J, Vrolix, K, Janssen, SP, Lemmens, MA, Van Der Esch, E, Duimel, H, Frederik, P, Molenaar, PC, Martinez-Martinez, P, De Baets, MH, and Losen, M Antibody effector mechanisms in myasthenia gravis pathogenesis at the neuromuscular junction. Autoimmunity 2010, 43, 353-370.
Gomez-Almaguer, D. Monoclonal antibodies in the treatment of immune thrombocytopenic purpura (ITP). Hematology. 2012, Suppl 1:S25-7.
Greenberg, BM, Graves, D, Remington, G, Hardeman, P, Mann, M, Karandikar, N, Stuve, O, Monson, N, Frohman, E. Rituximab dosing and monitoring strategies in neuromyelitis optica patients: creating strategies for therapeutic success. Mult Scler. 2012, 18(7), 1022-6.
Hengstman, GJ, Wesseling, P, Frenken, CW, and Jongen, PJ. Neuromyelitis optica with clinical and histopathological involvement of the brain. Mult Scler. 2007, 13(5), 679-682.
Hinson, SR, Pittock, SJ, Lucchinetti, CF, Roemer, SF, Fryer, JP, Kryzer, TJ, Lennon, VA. Pathogenic potential of IgG binding to water channel extracellular domain in neuromyelitis optica. Neurology. 2007, 69(24), 2221-31.
Hinson, SR, McKeon, A, Fryer, JP, Apiwattanakul, M, Lennon, VA, Pittock, SJ. Prediction of neuromyelitis optica attack severity by quantitation of complement-mediated injury to aquaporin-4-expressing cells. Arch. Neurol. 2009, 66, 1164-1167.
Jacob, A, McKeon, A, Nakashima, I, Sato, DK, Elsone, L, Fujihara, K, de Seze, J. Current concept of neuromyelitis optica (NMO) and NMO spectrum disorders. J Neurol Neurosurg Psychiatry. 2012, *epub.*
Jarius, S, Paul, F, Franciotta, D, Waters, P, Zipp, F, Hohlfeld, R, Vincent, A, and Wildemann, B. Mechanisms of disease: aquaporin-4 antibodies in neuromyelitis optica. Nat Clin Pract Neurol. 2008, 4(4), 202-214.
Jarius, S, and Wildemann, B. AQP4 antibodies in neuromyelitis optica: diagnostic and pathogenetic relevance. Nat Rev Neurol. 2010, 6(7), 383-392.
Kieseier, BC, Stüve, O, Dehmel, T, Goebels, N, Leussink, VI, Mausberg, AK, Ringelstein, M, Turowski, B, Aktas, O, Antoch, G, and Hartung, HP. Disease amelioration with Tocilizumab in a treatment-resistant patient with neuromyelitis optica: implication for cellular immune responses. Arch Neurol. 2012, 24, 1-4.
Kim, SH, Kim, W, Huh, SY, Lee, KY, Jun,g IJ, Kim, HJ. Clinical efficacy of plasmapheresis in patients with neuromyelitis optica spectrum disorder and effects on circulating anti-aquaporin-4 antibody levels. J Clin Neurol. 2013, 9(1), 36-42.
Kira, J. Autoimmunity in neuromyelitis optica and opticospinal multiple sclerosis: astrocytopathy as a common denominator in demyelinating disorders. J Neurol Sci. 2011, 311(1-2), 69-77.
Kishore, U, and Reid, KBM. C1q: structure, function, and receptors. Immunopharmacology 2000, 49, 159.170.
Klos, A, Tenner, AJ, Johswich, KO, Ager, RR, Reis, ES, Köhl, J. The role of the anaphylatoxins in health and disease. Mol Immunol. 2009, 46(14), 2753-66.
Lennon, VA, Kryzer, TJ, Pittock, SJ, Verkman, AS, and Hinson, SR. IgG marker of optic-spinal multiple sclerosis binds to the aquaporin-4 water channel. J Exp Med. 2005, 202(4), 473-477.
Lucchinetti, CF, Mandler, RN, McGavern, D, Bruck, W, Gleich, G, Ransohoff, RM, Trebst, C, Weinshenker, B, Wingerchuk, D, Parisi, JE, and Lassmann H. A role for humoral mechanisms in the pathogenesis of Devic's neuromyelitis optica. Brain. 2002, 125(Pt 7), 1450-1461.
Manderson, AP, Botto, M, Walport, MJ. The role of complement in the development of systemic lupus erythematosus. Annu. Rev. Immunol. 2004, 22, 431-456.
Misu, T, Fujihara, K, Kakita, A, Konno, H, Nakamura, M, Watanabe, S, Takahashi, T, Nakashima, I, Takahashi, H, and Itoyama, Y. Loss of aquaporin 4 in lesions of neuromyelitis optica: distinction from multiple sclerosis. Brain. 2007, 130(Pt 5), 1224-1234.
Nayak, A, Pednekar, L, Reid, KB, and Kishore U. Complement and non-complement activating functions of C1q: a prototypical innate immune molecule. Innate Immun. 2012, 18(2), 350-63.
Papadopoulos, MC, and Verkman, AS. Aquaporin 4 and neuromyelitis optica. Lancet Neurol. 2012, 11(6), 535-544.
Papadopoulos, M.C. and A.S. Verkman. Aquaporin water channels in neurobiology. Nature Rev. Neurosci. 2013, in press*.*
Parker, C. Eculizumab for paroxysmal nocturnal haemoglobinuria. Lancet. 2009;373(9665), 759-767.
Phuan, PW, Ratelade, J, Rossi, A, Tradtrantip, L, and Verkman, AS. Complement-dependent cytotoxicity in neuromyelitis optica requires aquaporin-4 protein assembly in orthogonal arrays. J Biol Chem. 2012, 287(17), 13829-13839.
Ratelade, J, Zhang, H, Saadoun, S, Bennett, JL, Papadopoulos, MC, and Verkman, AS. Neuromyelitis optica IgG and natural killer cells produce NMO lesions in mice without myelin loss. Acta Neuropathol. 2012, 123(6), 861-872.
Ricklin, D, Lambris, JD. Progress and trends in complement therapeutics. Adv Exp Med Biol. 2013, 735, 1-22.
Ricklin, D, Hajishengallis, G, and Lambris, JD. Complement: a key system for immune surveillance and homeostatis. Nat Immunology. 2010, 11(9), 785-797.
Risitano, AM. Paroxysmal nocturnal hemoglobinuria and the complement system: recent insights and novel anticomplement strategies. Adv Exp Med Biol. 2013, 735, 155-72.
Roemer, SF, Parisi JE, Lennon, VA, Benarroch, EE, Lassmann H, Bruck, W, Mandler, R, Weinshenker BG, Pittsock SJ, Wingerchuk, DM, and Lucchinetti CF. Pattern-specific loss of aquaporin-4 immunoreactivity distinguishes neuromyelitis optica from multiple sclerosis. Brain. 2007, 130 (Pt 5), 1194-1205.
Rossi A, Ratelade, J, Papadopoulos MC, Bennett JL, Verkman AS. Neuromyelitis optica IgG does not alter aquaporin-4 water permeability, plasma membrane M1/M23 isoform content, or supramolecular assembly. Glia. 2012, 60(12), 2027-2039.
Saadoun, S, Waters, P, Bell, BA, Vincent, A, Verkman, AS, and Papadopoulos, MC. Intracerebral injection of neuromyelitis optica immunoglobulin G and human complement produces neuromyelitis optica lesions in mice. Brain. 2010, 133(Pt 2), 349-361.
Saadoun, S, Waters, P, MacDonald, C, Bell, BA, Vincent, A, Verkman, AS, and Papadopoulos, MC. Neutrophil protease inhibition reduces neuromyelitis optica-immunoglobulin G-induced damage in mouse brain. Ann Neurol. 2012, 71(3), 323-333.
Sabater, L, Giralt, A, Boronat, A, Hankiewicz, K, Blanco, Y, Llufriu, S, Alberch, J, Graus, F, Saiz, A. Cytotoxic effect of neuromyelitis optica antibody (NMO-IgG) to astrocytes: an in vitro study. J. Neuroimmunol. 2009, 215, 31-35.
Sarzi-Puttini, P, Atzeni, F, Capsoni, F, Lubrano, E, Doria, A. Drug-induced lupus erythematosus. Autoimmunity. 2005, 38(7), 507-18.
Sato, D, Callegaro, D, Lana-Peixoto, MA, Fujihara, K. Treatment of neuromyelitis optica: an evidence based review. Arq Neuropsiquiatr. 2012, 70(1), 59-66.
Tradtrantip, L, Zhang, H, Anderson, MO, Saadoun, S, Phuan, PW, Papadopoulos, MC, Bennett, JL, and Verkman, AS. Small molecule inhibitors of NMO-IgG binding to aquaporin-4 reduce astrocyte cytotoxicity in neuromyelitis optica. FASEB J. 2012a, 26, 2197-2208.
Tradtrantip, L, Zhang, H, Saadoun, S, Phuan, PW, Lam, C, Papadopoulos, MC, Bennett, JL, Verkman, AS. Anti-aquaporin-4 monoclonal antibody blocker therapy for neuromyelitis optica. Ann. Neurol. 2012b, 71, 314-322.
Tradtrantip, L, Ratelade, J, Zhang, H, and Verkman, AS. Enzymatic deglycosylation converts pathogenic neuromyelitis optica anti-aquaporin-4 immunoglobulin G into therapeutic antibody. Ann Neurol. 2013, 73(1), 77-85.
Tüzün, E, Li, J, Saini, SS, Yang, H, Christadoss, P. Targeting classical complement pathway to treat complement mediated autoimmune diseases. Adv Exp Med Biol. 2008, 632, 265-72.
Van Herle, K, Behne, JM, Van Herle, A, Blaschke, TF, Smith, TJ, Yeaman, MR Integrative continuum: accelerating therapeutic advances in rare autoimmune diseases. Annu Rev Pharmacol Toxicol. 2012, 52, 523-47.
Vedove, CD, Del Giglio, M, Schena, D, Girolomoni, G. Drug-induced lupus erythematosus. Arch Dermatol Res. 2009, 301(1), 99-105.
Wallis, R, Mitchell, DA, Schmid, R, Schwaeble, WJ, Keeble, AH. Paths reunited: Initiation of the classical and lectin pathways of complement activation. Immunobiol. 2010, 215, 1-11.
Wilde, B, van Paassen, P, Witzke, O, and Tervaert, JW. New pathophysiological insights and treatment of ANCA-associated vasculitis. Kidney Int. 2011, 79, 599-612.
Wingerchuk, DM. Neuromyelitis optica: potential roles for intravenous immunoglobulin. J Clin Immunol. 2013, 33, Suppl 1:S33-7.
Wingerchuk, DM, Lennon, VA, Lucchinetti, CF, Pittock, SJ, and Weinshenker, BG. The spectrum of neuromyelitis optica. Lancet Neurol. 2007, 6(9), 805-815.
Zhang, H, Bennett, JL, and Verkman, AS. Ex vivo spinal cord slice model of neuromyelitis optica reveals novel immunopathogenic mechanisms. Ann Neurol. 2011, 70(6), 943-954.
Zhang, H, and Verkman AS. Eosinophil pathogenicity mechanisms and therapeutics in neuromyelitis optica. J. Clin. Invest. 2013, in press
Zipfel, PF and Skerka, C. Complement: The alternative pathway. 2008, eLS*.*
Zipfel, PF and Skerka, C. Complement regulators and inhibitory proteins. Nature Rev. Immunol. 2009, 9,729-740.

## Claims

1. An antibody, wherein the antibody is:
i) an anti-C1q antibody, wherein the anti-C1q antibody inhibits the interaction between C1q and an autoantibody or between C1q and C1r, or between C1q and C1s;
ii) an anti-C1r antibody, wherein the anti-C1r antibody inhibits the interaction between C1r and C1q or between C1r and C1s, or wherein the anti-C1r antibody inhibits the catalytic activity of C1r or inhibits the processing of pro-C1r to an active protease; or
iii) an anti-C1s antibody, wherein the anti-C1s antibody inhibits the interaction between C1s and C1q or between C1s and C1r or between C1s and C2 or C4, or wherein the anti-C1s antibody inhibits the catalytic activity of C1s or inhibits the processing of pro-C1s to an active protease,
for use in preventing, reducing risk of developing, or treating neuromyelitis optica (NMO).

2. The antibody for use according to claim 1, wherein the antibody is a monoclonal antibody, preferably an antibody that binds mammalian C1q, C1r, C1s or C1 complex.

3. The antibody for use according to claim 2, wherein the antibody binds human C1q, C1r, C1s, or C1 complex.

4. The antibody for use according to any one of claims 1 to 3, wherein the antibody is a mouse antibody, a human antibody, a humanized antibody, or a chimeric antibody.

5. The antibody for use according to one of claims 1 to 4, wherein the antibody is an antibody fragment selected from the group consisting of Fab, Fab'-SH, Fv, scFv, and (Fab')₂ fragments.

6. The antibody for use according to one of claims 1 to 5, wherein the antibody is a bispecific antibody that recognizes a first antigen and a second antigen.

7. The antibody for use according to claim 6, wherein the first antigen is selected from the group consisting of C1q, C1r, and C1s and the second antigen is an antigen that facilitates transport across the blood-brain-barrier.

8. The antibody for use according to claim 6 or 7, wherein the first antigen is selected from C1q, C1r, and C1s and the second antigen is selected from the group consisting of transferrin receptor (TR), insulin receptor (HIR), insulin-like growth factor receptor (IGFR), low-density lipoprotein receptor related proteins 1 and 2 (LPR-1 and 2), diphtheria toxin receptor, CRM197, a llama single domain antibody, TMEM 30(A), a protein transduction domain, TAT, Syn-B, penetratin, a poly-arginine peptide, an angiopep peptide, and ANG1005.

9. The antibody for use according to one of claims 1 to 8, wherein the antibody inhibits the classical complement activation pathway by an amount from at least 30% to at least 99.9%.

10. The antibody for use according to one of claims 1 to 9, wherein the antibody inhibits the alternative complement activation pathway by an amount from at least 30% to at least 99.9%.

11. The antibody for use according to one of claims 1 to 10, wherein the antibody inhibits complement-dependent cell-mediated cytotoxicity (CDCC) activation pathway by an amount from at least 30% to at least 99.9%.

12. The antibody for use according to one of claims 1 to 11, wherein the antibody inhibits autoantibody-dependent and complement-dependent cytotoxicity (CDC).

13. The antibody for use according to one of claims 1 to 12, wherein the antibody inhibits complement-dependent cell-mediated cytotoxicity (CDCC).

14. The antibody for use according to one of claims 1 to 13, wherein the antibody inhibits amplification of the alternative complement activation pathway initiated by C1q binding.

15. The antibody for use according to one of claims 1 to 14, wherein a therapeutically effective amount of two antibodies is to be administered, and wherein the two antibodies are selected from the group consisting of an anti-C1q antibody, anti-C1r antibody, and an anti-C1s antibody.

## Patentansprüche

1. Antikörper, wobei der Antikörper:
i) ein Anti-C1q-Antikörper ist, wobei der Anti-C1q-Antikörper die Wechselwirkung zwischen C1q und einem Autoantikörper oder zwischen C1q und C1r, oder zwischen C1q und C1s inhibiert;
ii) ein Anti-C1r-Antikörper ist, wobei der Anti-C1r-Antikörper die Wechselwirkung zwischen C1r und C1q oder zwischen C1r und C1s inhibiert, oder wobei der Anti-C1r-Antiköper die katalytische Aktivität von C1r inhibiert oder das Prozessieren von Pro-C1r zu einer aktiven Protease inhibiert; oder
iii) ein Anti-C1s-Antikörper ist, wobei der Anti-C1s-Antikörper die Wechselwirkung zwischen C1s und C1q oder zwischen C1s und C1r oder zwischen C1s und C2 oder C4 inhibiert, oder wobei der Anti-C1s-Antikörper die katalytische Aktivität von C1s inhibiert oder das Prozessieren von Pro-C1s zu einer aktiven Protease inhibiert,
für die Verwendung zum Vorbeugen von Neuromyelitis Optica (NMO), zum Reduzieren des Risikos NMO zu entwickeln, oder zum Behandeln von NMO.

2. Antikörper für die Verwendung nach Anspruch 1, wobei der Antikörper ein monoklonaler Antikörper ist, vorzugsweise ein Antikörper der an einen C1q-, C1r-, C1s- oder C1-Komplex aus Säugern bindet.

3. Antikörper für die Verwendung nach Anspruch 2, wobei der Antikörper humanen C1q-, C1r-, C1s- oder C1-Komplex bindet.

4. Antikörper für die Verwendung nach einem der Ansprüche 1 bis 3, wobei der Antikörper ein Maus-Antikörper, ein menschlicher Antikörper, ein humanisierter Antikörper oder ein chimärer Antikörper ist.

5. Antikörper für die Verwendung nach einem der Ansprüche 1 bis 4, wobei der Antikörper ein Antikörperfragment ist, ausgewählt aus Fab-, Fab'-SH-, Fv-, scFv-, und (Fab')₂-Fragmenten.

6. Antikörper für die Verwendung nach einem der Ansprüche 1 bis 5, wobei der Antikörper ein bispezifischer Antikörper ist, der ein erstes und ein zweites Antigen erkennt.

7. Antikörper für die Verwendung nach Anspruch 6, wobei das erste Antigen ausgewählt ist aus C1q, C1r, und C1s und das zweite Antigen ein Antigen ist, das den Transport über die Blut-Hirn-Schranke ermöglicht.

8. Antikörper für die Verwendung nach Anspruch 6 oder 7, wobei das erste Antigen aus C1q, C1r, und C1s ausgewählt ist und das zweite Antigen ausgewählt ist aus Transferrin-Rezeptor (TR), Insulin-Rezeptor (HIR), Rezeptor des insulin-ähnlichen Wachstumsfaktorrezeptors (IGFR), Lipoprotein niedriger Dichte-Rezeptor verwandten Proteinen 1 und 2 (LPR-1 und -2), Diphtherietoxin-Rezeptor, CRM197, einem Lama-Einzel-Domäne-Antikörper, TMEM 30(A), einer Proteintransduktionsdomäne, TAT, Syn-B, Penetratin, einem PolyArginin-Peptid, einem Angiopep-Peptid, und ANG1005.

9. Antikörper für die Verwendung nach einem der Ansprüche 1 bis 8, wobei der Antikörper den klassischen Komplementaktivierungspfad in einem Ausmaß von mindestens 30% bis mindestens 99,9% inhibiert.

10. Antikörper für die Verwendung nach einem der Ansprüche 1 bis 9, wobei der Antikörper den alternativen Komplementaktivierungspfad in einem Ausmaß von mindestens 30% bis mindestens 99,9% inhibiert.

11. Antikörper für die Verwendung nach einem der Ansprüche 1 bis 10, wobei der Antikörper den Komplement-abhängigen Zell-vermittelten Zytotoxizität (CDCC)-Aktivierungspfad in einem Ausmaß von mindestens 30% bis mindestens 99,9% inhibiert.

12. Antikörper für die Verwendung nach einem der Ansprüche 1 bis 11, wobei der Antikörper Autoantikörper-abhängige und Komplement-abhängige Zytotoxizität (CDC) inhibiert.

13. Antikörper für die Verwendung nach einem der Ansprüche 1 bis 12, wobei der Antikörper Komplement-abhängige Zell-vermittelte Zytotoxizität (CDCC) inhibiert.

14. Antikörper für die Verwendung nach einem der Ansprüche 1 bis 13, wobei der Antikörper die durch C1q-Bindung initiierte Amplifizierung des alternativen Komplementaktivierungspfades inhibiert.

15. Antikörper für die Verwendung nach einem der Ansprüche 1 bis 14, wobei eine therapeutisch wirksame Menge von zwei Antikörpern zu verabreichen ist, und wobei die beiden Antikörper aus einem Anti-C1q-Antikörper, einem Anti-C1r-Antikörper und einem Anti-C1s-Antikörper ausgewählt werden.

## Revendications

1. Anticorps, lequel anticorps est :
i) un anticorps anti-Clq, lequel anticorps anti-C1q inhibe l'interaction entre C1q et un auto-anticorps ou entre C1q et C1r, ou entre C1q et C1s ;
ii) un anticorps anti-Clr, lequel anticorps anti-C1r inhibe l'interaction entre C1r et C1q ou entre C1r et C1s, ou bien lequel anticorps anti-C1r inhibe l'activité catalytique de C1r ou inhibe la transformation de pro-C1r en une protéase active ; ou
iii) un anticorps anti-C1s, lequel anticorps anti-C1s inhibe l'interaction entre C1s et C1q ou entre C1s et C1r ou entre C1s et C2 ou C4, ou bien lequel anticorps anti-C1s inhibe l'activité catalytique de C1s ou inhibe la transformation de pro-C1s en une protéase active,
pour une utilisation dans la prévention, la réduction du risque de développement, ou le traitement, d'une neuromyélite optique (NMO).

2. Anticorps pour une utilisation selon la revendication 1, lequel anticorps est un anticorps monoclonal, de préférence un anticorps qui se lie au complexe C1q, C1r, C1s ou C1 de mammifère.

3. Anticorps pour une utilisation selon la revendication 2, lequel anticorps se lie au complexe C1q, C1r, C1s ou C1 humain.

4. Anticorps pour une utilisation selon l'une des revendications 1 à 3, lequel anticorps est un anticorps de souris, un anticorps humain, un anticorps humanisé, ou un anticorps chimérique.

5. Anticorps pour une utilisation selon l'une des revendications 1 à 4, lequel anticorps est un fragment d'anticorps choisi dans le groupe constitué par les fragments Fab, Fab'-SH, Fv, scFv, et (Fab')₂.

6. Anticorps pour une utilisation selon l'une des revendications 1 à 5, lequel anticorps est un anticorps bispécifique qui reconnaît un premier antigène et un deuxième antigène.

7. Anticorps pour une utilisation selon la revendication 6, dans lequel le premier antigène est choisi dans le groupe constitué par C1q, C1r et C1s, et le deuxième antigène est un antigène qui facilite le transport à travers la barrière hémato-encéphalique.

8. Anticorps pour une utilisation selon la revendication 6 ou 7, dans lequel le premier antigène est choisi parmi C1q, C1r et C1s, et le deuxième antigène est choisi dans
le groupe constitué par le récepteur de transferrine (TR), le récepteur à insuline (HIR), le récepteur au facteur de croissance insulin-like (IGFR), les protéines 1 et 2 apparentées au récepteur aux lipoprotéines basse densité (LPR-1 et -2), le récepteur de toxine diphtérique, CRM197, un anticorps à domaine unique de lama, TMEM 30(A), un domaine de transduction de protéine, TAT, Syn-B, la pénétratine, un peptide polyarginine, un peptide angiopep, et ANG1005.

9. Anticorps pour une utilisation selon l'une des revendications 1 à 8, lequel anticorps inhibe la voie d'activation du complément classique en une quantité d'au moins 30 % à au moins 99,9 %.

10. Anticorps pour une utilisation selon l'une des revendications 1 à 9, lequel anticorps inhibe la voie d'activation du complément alternative en une quantité d'au moins 30 % à au moins 99,9 %.

11. Anticorps pour une utilisation selon l'une des revendications 1 à 10, lequel anticorps inhibe la voie d'activation de la cytotoxicité à médiation cellulaire dépendante du complément (CDCC) en une quantité d'au moins 30 % à au moins 99,9 %.

12. Anticorps pour une utilisation selon l'une des revendications 1 à 11, lequel anticorps inhibe la cytotoxicité dépendante du complément et dépendante d'un auto-anticorps (CDC).

13. Anticorps pour une utilisation selon l'une des revendications 1 à 12, lequel anticorps inhibe la cytotoxicité à médiation cellulaire dépendante du complément (CDCC).

14. Anticorps pour une utilisation selon l'une des revendications 1 à 13, lequel anticorps inhibe l'amplification de la voie d'activation du complément alternative initiée par la liaison de C1q.

15. Anticorps pour une utilisation selon l'une des revendications 1 à 14, dans lequel une quantité thérapeutique efficace de deux anticorps est à administrer, et dans lequel les deux anticorps sont choisis dans le groupe constitué par un anticorps anti-C1q, un anticorps anti-C1r, et un anticorps anti-C1s.
